# EUROPEAN PATENT APPLICATION

(11) **EP 4 782 447 A1**
(43) Date of publication of application: **29.07.2026**
(21) Application number: 24878875.4
(22) Date of filing: 09.10.2024
(51) Int. Cl.: C07D 417/14, C07D 417/04, C07D 413/14, C07D 401/14, C07D 471/04, A61P 35/00, A61P 35/02, A61K 31/444, A61K 31/4439, A61K 31/53, A61K 31/506, A61K 31/496

(54) **HETEROAROMATIC SULFONAMIDE STRUCTURAL COMPOUND, PHARMACEUTICAL COMPOSITION THEREOF, AND USE THEREOF**

(30) Priority: 20.10.2023 CN 202311362643; 10.11.2023 CN 202311497375; 06.12.2023 CN 202311657771; 10.01.2024 CN 202410038300
(71) Applicant: Shanghai Yingli Pharmaceutical Co., Ltd, Shanghai 201203 (CN)
(72) Inventor: XU, Zusheng, Shanghai 201203 (CN); LOU, Yangtong, Shanghai 201203 (CN)
(74) Representative: Vogelbruch, Keang
(86) International application number: PCT/CN2024/123657
(87) International publication number: WO 2025/082231

(57) **Abstract**

Disclosed in the present invention are a heteroaromatic sulfonamide structural compound, a pharmaceutical composition thereof, and a use thereof. The present invention provides a heteroaromatic sulfonamide structural compound as shown in formula I, and a pharmaceutically acceptable salt, stereoisomer, tautomer, or isotope compound thereof. The heteroaromatic sulfonamide structural compound is expected to be used for treating and/or preventing various diseases related to Poly(ADP-ribose)glycohydrolase (PARG).

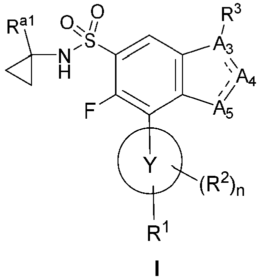

## Description

### CROSS-REFERENCE TO RELATED APPLICATIONS

This application claims the priority of Chinese Patent Application No. 2023113626436 filed October 20, 2023, Chinese Patent Application No. 2023114973759 filed November 10, 2023, Chinese Patent Application No. 2023116577713 filed December 06, 2023, and Chinese Patent Application No. 2024100383002 filed January 10, 2024. The entire contents of the aforementioned Chinese patent applications are incorporated by reference into this application.

### TECHNICAL FIELD

The present disclosure relates to a heteroaromatic sulfonamide structural compound, pharmaceutical composition thereof and use thereof.

### BACKGROUND OF THE INVENTION

The division and proliferation of cancer cells are often uncontrolled, leading to a higher incidence of DNA damage and defects in DNA repair. Compared with normal cells, cancer cells are more dependent on the DNA damage repair machinery.

Poly(ADP-ribose) polymerase (PARP), poly(ADP-ribose) glycohydrolase (PARG) and several other proteins play crucial roles in DNA repair, thus becoming important targets for the research and development of anticancer drugs. PARP can bind to DNA single-strand break sites and promote the synthesis of poly(ADP-ribose) (PAR) chains, thereby initiating the repair process. PARG functions to degrade PAR attached to PARP, facilitating the completion of the entire repair cycle. Inhibition of either PARP or PARG will disrupt the overall repair process.

To date, the research and development of PARP inhibitors have achieved remarkable success, with multiple drugs approved for marketing, which has also verified the feasibility of DNA repair proteins as therapeutic targets. Meanwhile, PARP inhibitors are still associated with limitations such as limited efficacy across all patient populations and the emergence of drug resistance. In contrast, research on PARG inhibitors remains in the exploratory stage, and this target category boasts substantial research potential, which is expected to address unmet clinical needs.

### SUMMARY OF THE INVENTION

The technical problem to be solved by the present disclosure is the lack of effective drugs acting as PARG inhibitors for clinical therapy in the prior art. To this end, the present disclosure provides a heteroaromatic sulfonamide structural compound, pharmaceutical composition thereof and use thereof, wherein the heteroaromatic sulfonamide structural compound is expected to be used for the treatment and/or prevention of various diseases associated with PARG.

The present disclosure solves the aforementioned technical problem through the following technical solutions.

The present disclosure provides a heteroaromatic sulfonamide structural compound represented by formula I, a pharmaceutically acceptable salt thereof, a stereoisomer thereof, a tautomer thereof or an isotopically labeled compound thereof: 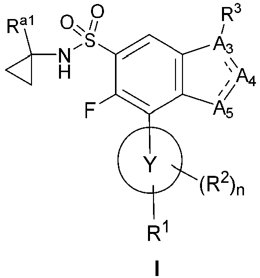
"-̅ -̅ -̅" represents a single bond or a double bond;
A₃ is C or N;
A₄ is N or CR⁴;
A₅ is NR⁵ or CR⁵; 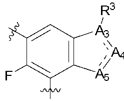
is 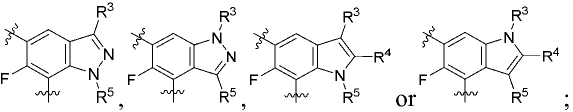
R^{a1} is cyano or C₁₋₆ alkyl;
Ring Y is a "4- to 12-membered heterocycloalkyl containing 1 to 3 heteroatoms each independently selected from O, S and N", or a "4- to 8-membered heterocycloalkenyl containing 1 to 3 heteroatoms each independently selected from O, S and N";
R¹ is 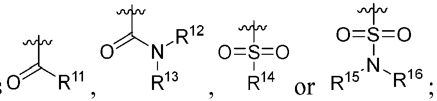
R¹¹, R¹², R¹³, R¹⁴, R¹⁵ and R¹⁶ are each independently C₁₋₆ alkyl, C₁₋₆ alkyl substituted with one or more R¹⁻¹, C₃₋₁₀ cycloalkyl, C₃₋₁₀ cycloalkyl substituted with one or more R¹⁻², a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R¹⁻³, C₆₋₂₀ aryl, C₆₋₂₀ aryl substituted with one or more R¹⁻⁴, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R¹⁻⁵, C₄₋₈ cycloalkenyl, C₄₋₈ cycloalkenyl substituted with one or more R¹⁻⁶, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R¹⁻⁷;
R¹⁻¹, R¹⁻², R¹⁻³, R¹⁻⁴, R¹⁻⁵, R¹⁻⁶ and R¹⁻⁷ are each independently halogen, hydroxyl, cyano, C₁₋₆ alkyl, C₂₋₆ alkenyl, C₂₋₆ alkynyl, -OC₁₋₆ alkyl, -SC₁₋₆ alkyl, -C(=O)R^{1a}, -NR^{1b1}R^{1b2}, -C(=O)OR^{1c}, -C(=O)NR^{1d1}R^{1d2}, -S(O)₂NR^{1c1}R^{1c2}, -S(O)₂R^{1f}, C₃₋₁₀ cycloalkyl, a "4- to 12-membered heterocycloalkyl containing 1 to 3 heteroatoms each independently selected from O, S and N", C₆₋₂₀ aryl, a "5- to 12-membered heteroaryl containing 1 to 4 heteroatoms each independently selected from O, S and N", C₄₋₈ cycloalkenyl, or a "4- to 8-membered heterocycloalkenyl containing 1 to 3 heteroatoms each independently selected from O, S and N"; when there are multiple substituents, they may be the same or different;
R^{1a}, R^{1b1}, R^{1b2}, R^{1c}, R^{1d1}, R^{1d2}, R^{1c1}, R^{1c2} and R^{1f} are each independently hydrogen, C₁₋₆ alkyl, or C₁₋₆ alkyl substituted with one or more halogen atoms;
n is 0, 1, 2, 3, 4, 5 or 6;
R² is halogen, hydroxyl, cyano, oxo, C₁₋₆ alkyl, C₁₋₆ alkyl substituted with one or more R²⁻¹, C₃₋₈ cycloalkyl, C₃₋₈ cycloalkyl substituted with one or more R²⁻², C C₂₋₆ alkenyl, C₂₋₆ alkynyl, - OC₁₋₆ alkyl, -SC₁₋₆ alkyl, -C(=O)R^{2a}, -NR^{2b1}R^{2b2}, -C(=O)OR^{2c}, or -C(=O)NR^{2d1}R^{2d2}; alternatively, when n is 2, 3, 4, 5 or 6, any two R² are linked to each other, independently form, together with the atoms on the ring to which they are attached, a 3- to 8-membered carbocyclic ring, or a "3- to 8-membered heterocyclic ring containing 1 to 3 heteroatoms each independently selected from O, S and N";
R²⁻¹ and R²⁻² are each independently halogen, cyano, C₁₋₆ alkyl-O-, hydroxyl, -C(=O)R^{22a}, - NR^{22b1}R^{22b2}, -C(=O)OR^{22c}, or -C(=O)NR^{22d1}R^{22d2};
R^{2a}, R^{2b1}, R^{2b2}, R^{2c}, R^{2d1}, R^{2d2}, R^{22a}, R^{22b1}, R^{22b2}, R^{22c}, R^{22d1} and R^{22d2} are each independently hydrogen, C₁₋₆ alkyl, or C₁₋₆ alkyl substituted with one or more halogen atoms;
R³ is a "5- to 6-membered heteroaryl containing 1 to 4 heteroatoms each independently selected from O, S and N", or a "5- to 6-membered heteroaryl containing 1 to 4 heteroatoms each independently selected from O, S and N" and substituted with one or more R³⁻¹;
R³⁻¹ is halogen, hydroxyl, amino, cyano, C₁₋₆ alkyl, C₁₋₆ alkyl substituted with one or more halogen atoms, C₁₋₆ alkyl substituted with one or more -OC₁₋₆ alkyl, -OC₁₋₆ alkyl, -SC₁₋₆ alkyl, -OC₁₋₆ alkyl substituted with one or more halogen atoms, pentafluorosulfanyl, or -SC₁₋₆ alkyl substituted with one or more halogen atoms; when there are multiple substituents, they may be the same or different;
R⁴ is hydrogen, halogen, C₁₋₆ alkyl, or C₁₋₆ alkyl substituted with one or more halogen atoms; R⁵ is hydrogen, C₁₋₆ alkyl, or C₁₋₆ alkyl substituted with one or more R⁵⁻¹;
R⁵⁻¹ is deuterium, halogen, cyano, C₂₋₆ alkenyl, C₂₋₆ alkynyl, hydroxyl, -OC₁₋₆ alkyl, -C(=O)R^{5a}, -NR^{5b1}R^{5b2}, -C(=O)OR^{5c}, -C(=O)NR^{5d1}R^{5d2}, C₆₋₂₀ aryl, C₆₋₂₀ aryl substituted with one or more R⁵⁻¹⁻¹, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R⁵⁻¹⁻², C₃₋₁₂ cycloalkyl, C₃₋₁₂ cycloalkyl substituted with one or more R⁵⁻¹⁻³, a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R⁵⁻¹⁻⁴, C₄₋₈ cycloalkenyl, C₄₋₈ cycloalkenyl substituted with one or more R⁵⁻¹⁻⁵, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R⁵⁻¹⁻⁶; whem there are multiple substituents, they may be the same or different;
R⁵⁻¹⁻¹, R⁵⁻¹⁻², R⁵⁻¹⁻³, R⁵⁻¹⁻⁴, R⁵⁻¹⁻⁵ and R⁵⁻¹⁻⁶ are each independently halogen, C₁₋₆ alkyl, or C₁₋₆ alkyl substituted with one or more halogen atoms;
R^{5a}, R^{5b1}, R^{5b2}, R^{5c}, R^{5d1} and R^{5d2} are each independently hydrogen, C₁₋₆ alkyl, or C₁₋₆ alkyl substituted with one or more halogen atoms;
The heteroaromatic sulfonamide structural compound represented by formula I is not any one of the following compounds:
   N-(1-methylcyclopropyl)-3-(5-(difluoromethyl)-1,3,4-thiadiazol-2-yl)-1-ethyl-6-fluoro-7-(4-isobutyrylpiperazin-1-yl)-1H-indazole-5-sulfonamide;
   N-(1-cyanocyclopropyl)-6-fluoro-7-(4-(isopropylsulfonyl)piperazin-1-yl)-1-methyl-3-(5-(trifluoromethyl)-1,3,4-thiadiazol-2-yl)-1H-indazole-5-sulfonamide.

In one embodiment, the structure of the aforementioned heteroaromatic sulfonamide structural compound represented by formula I is as shown in formula II:

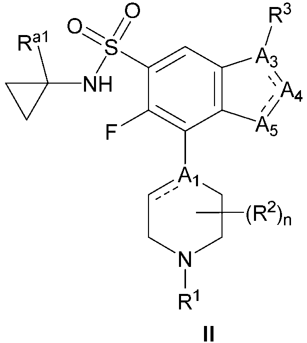

Wherein, "-̅ -̅ -̅" represents a single bond or a double bond;
A₁ is C, CH or N;
A₃ is C or N;
A₄ is N or CR⁴;
A₅ is NR⁵ or CR⁵; 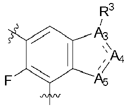
is 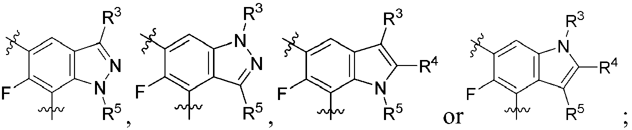
R^{a1} is cyano or C₁₋₆ alkyl;
R¹ is 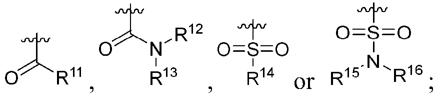
R¹¹, R¹², R¹³, R¹⁴, R¹⁵ and R¹⁶ are each independently C₁₋₆ alkyl, C₁₋₆ alkyl substituted with one or more R¹⁻¹, C₃₋₁₀ cycloalkyl, C₃₋₁₀ cycloalkyl substituted with one or more R¹⁻², a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R¹⁻³, C₆₋₂₀ aryl, C₆₋₂₀ aryl substituted with one or more R¹⁻⁴, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R¹⁻⁵, C₄₋₈ cycloalkenyl, C₄₋₈ cycloalkenyl substituted with one or more R¹⁻⁶, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R¹⁻⁷;
R¹⁻¹, R¹⁻², R¹⁻³, R¹⁻⁴, R¹⁻⁵, R¹⁻⁶ and R¹⁻⁷ are each independently halogen, hydroxyl, cyano, C₁₋₆ alkyl, C₂₋₆ alkenyl, C₂₋₆ alkynyl, -OC₁₋₆ alkyl, -SC₁₋₆ alkyl, -C(=O)R^{1a}, -NR^{1b1}R^{1b2}, -C(=O)OR^{1c}, -C(=O)NR^{1d1}R^{1d2}, -S(O)₂NR^{1c1}R^{1c2}, -S(O)₂R^{1f}, C₃₋₁₀ cycloalkyl, a "4- to 12-membered heterocycloalkyl containing 1 to 3 heteroatoms each independently selected from O, S and N", C₆₋₂₀ aryl, a "5- to 12-membered heteroaryl containing 1 to 4 heteroatoms each independently selected from O, S and N", C₄₋₈ cycloalkenyl, or a "4- to 8-membered heterocycloalkenyl containing 1 to 3 heteroatoms each independently selected from O, S and N"; when there are multiple substituents, they may be the same or different;
R^{1a}, R^{1b1}, R^{1b2}, R^{1c}, R^{1d1}, R^{1d2}, R^{1c1}, R^{1c2} and R^{1f} are each independently hydrogen, C₁₋₆ alkyl, or C₁₋₆ alkyl substituted with one or more halogen atoms;
n is 0, 1, 2, 3, 4, 5 or 6;
R² is halogen, hydroxyl, cyano, oxo, C₁₋₆ alkyl, C₁₋₆ alkyl substituted with one or more R²⁻¹, C₃₋₈ cycloalkyl, C₃₋₈ cycloalkyl substituted with one or more R²⁻² C₂₋₆ alkenyl,
C₂₋₆ alkynyl, -OC₁₋₆ alkyl, -SC₁₋₆ alkyl, -C(=O)R^{2a}, -NR^{2b1}R^{2b2}, -C(=O)OR^{2c}, or - C(=O)NR^{2d1}R^{2d2}; alternatively, when n is 2, 3, 4, 5 or 6, any two R² are linked to each other, independently form, together with the atoms on the ring to which they are attached, a 3- to 8-membered carbocyclic ring, or a "3- to 8-membered heterocyclic ring containing 1 to 3 heteroatoms each independently selected from O, S and N";
R²⁻¹ and R²⁻² are each independently halogen, cyano, C₁₋₆ alkyl-O-, hydroxyl, -C(=O)R^{22a}, - NR^{22b1}R^{22b2}, -C(=O)OR^{22c}, or -C(=O)NR^{22d1}R^{22d2};
R^{2a}, R^{2b1}, R^{2b2}, R^{2c}, R^{2d1}, R^{2d2}, R^{22a}, R^{22b1}, R^{22b2}, R^{22c}, R^{22d1} and R^{22d2} are each independently hydrogen, C₁₋₆ alkyl, or C₁₋₆ alkyl substituted with one or more halogen atoms;
R³ is a "5- to 6-membered heteroaryl containing 1 to 4 heteroatoms each independently selected from O, S and N", or a "5- to 6-membered heteroaryl containing 1 to 4 heteroatoms each independently selected from O, S and N" and substituted with one or more R³⁻¹;
R³⁻¹ is halogen, hydroxyl, amino, cyano, C₁₋₆ alkyl, C₁₋₆ alkyl substituted with one or more halogen atoms, C₁₋₆ alkyl substituted with one or more -OC₁₋₆ alkyl, -OC₁₋₆ alkyl, -SC₁₋₆ alkyl, -OC₁₋₆ alkyl substituted with one or more halogen atoms, pentafluorosulfanyl, or -SC₁₋₆ alkyl substituted with one or more halogen atoms; when there are multiple substituents, they may be the same or different;
R⁴ is hydrogen, halogen, C₁₋₆ alkyl, or C₁₋₆ alkyl substituted with one or more halogen atoms; R⁵ is hydrogen, C₁₋₆ alkyl, or C₁₋₆ alkyl substituted with one or more R⁵⁻¹
R⁵⁻¹ is deuterium, halogen, cyano, C₂₋₆ alkenyl, C₂₋₆ alkynyl, hydroxyl, -OC₁₋₆ alkyl, -C(=O)R^{5a}, -NR^{5b1}R^{5b2}, -C(=O)OR^{5c}, -C(=O)NR^{5d1}R^{5d2}, C₆₋₂₀ aryl, C₆₋₂₀ aryl substituted with one or more R⁵⁻¹⁻¹, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R⁵⁻¹⁻², C₃₋₁₂ cycloalkyl, C₃₋₁₂ cycloalkyl substituted with one or more R⁵⁻¹⁻³, a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R⁵⁻¹⁻⁴, C₄₋₈ cycloalkenyl, C₄₋₈ cycloalkenyl substituted with one or more R⁵⁻¹⁻⁵, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R⁵⁻¹⁻⁶; when there are multiple substituents, they may be the same or different;
R⁵⁻¹⁻¹, R⁵⁻¹⁻², R⁵⁻¹⁻³, R⁵⁻¹⁻⁴, R⁵⁻¹⁻⁵ and R⁵⁻¹⁻⁶ are each independently halogen, C₁₋₆ alkyl, or C₁₋₆ alkyl substituted with one or more halogen atoms;
R^{5a}, R^{5b1}, R^{5b2}, R^{5c}, R^{5d1} and R^{5d2} are each independently hydrogen, C₁₋₆ alkyl, or C₁₋₆ alkyl substituted with one or more halogen atoms;
The heteroaromatic sulfonamide structural compound represented by formula I is not any one of the following compounds:
   N-(1-methylcyclopropyl)-3-(5-(difluoromethyl)-1,3,4-thiadiazol-2-yl)-1-ethyl-6-fluoro-7-(4-isobutyrylpiperazin-1-yl)-1H-indazole-5-sulfonamide;
   N-(1-cyanocyclopropyl)-6-fluoro-7-(4-(isopropylsulfonyl)piperazin-1-yl)-1-methyl-3-(5-(trifluoromethyl)-1,3,4-thiadiazol-2-yl)-1H-indazole-5-sulfonamide.

In one embodiment, in the structure represented by formula I,
A₃ is C;
A₄ is N;
A₅ is NR⁵; 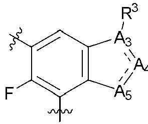
is 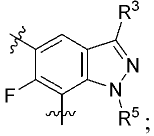
R^{a1} is cyano or C₁₋₆ alkyl;
Ring Y is a "4- to 12-membered heterocycloalkyl containing 1 to 3 heteroatoms each independently selected from O, S and N", or a "4- to 8-membered heterocycloalkenyl containing 1 to 3 heteroatoms each independently selected from O, S and N";
R¹ is 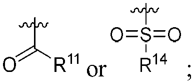
R¹¹ and R¹⁴ are each independently C₁₋₆ alkyl;
n is 0, 1 or 2;
R² is cyano, C₃₋₈ cycloalkyl or C₁₋₆ alkyl;
R³ is a "5- to 6-membered heteroaryl containing 1 to 4 heteroatoms each independently selected from O, S and N" and substituted with one or more R³⁻¹;
R³⁻¹ is C₁₋₆ alkyl substituted with one or more halogen atoms;
R⁵ is C₁₋₆ alkyl;
The heteroaromatic sulfonamide structural compound represented by formula I is not any one of the following compounds:
   N-(1-methylcyclopropyl)-3-(5-(difluoromethyl)-1,3,4-thiadiazol-2-yl)-1-ethyl-6-fluoro-7-(4-isobutyrylpiperazin-1-yl)-1H-indazole-5-sulfonamide;
   N-(1-cyanocyclopropyl)-6-fluoro-7-(4-(isopropylsulfonyl)piperazin-1-yl)-1-methyl-3-(5-(trifluoromethyl)-1,3,4-thiadiazol-2-yl)-1H-indazole-5-sulfonamide.

In one embodiment, the heteroaromatic sulfonamide structural compound represented by formula I, a pharmaceutically acceptable salt thereof, a stereoisomer thereof, a tautomer thereof or an isotopically labeled compound thereof, is as defined in Embodiment 1, Embodiment 2 or Embodiment 3:
Embodiment 1: in the heteroaromatic sulfonamide structural compound represented by formula I,
   "-̅ -̅ -̅" represents a single bond or a double bond;
   A₃ is C or N;
   A₄ is N or CR⁴;
   A₅ is NR⁵ or CR⁵; 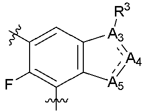
   is 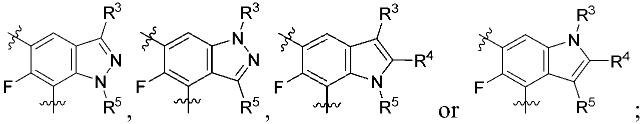
   R^{a1} is cyano or C₁₋₆ alkyl;
   Ring Y is a "4- to 12-membered heterocycloalkyl containing 1 to 3 heteroatoms each independently selected from O, S and N", or a "4- to 8-membered heterocycloalkenyl containing 1 to 3 heteroatoms each independently selected from O, S and N";
   R¹ is 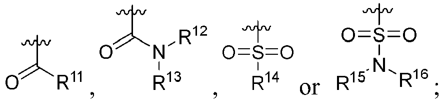
   R¹¹, R¹², R¹³, R¹⁴, R¹⁵ and R¹⁶ are each independently C₁₋₆ alkyl, C₁₋₆ alkyl substituted with one or more R¹⁻¹, C₃₋₁₀ cycloalkyl, C₃₋₁₀ cycloalkyl substituted with one or more R¹⁻², a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R¹⁻³, C₆₋₂₀ aryl, C₆₋₂₀ aryl substituted with one or more R¹⁻⁴, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R¹⁻⁵, C₄₋₈ cycloalkenyl, C₄₋₈ cycloalkenyl substituted with one or more R¹⁻⁶, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R¹⁻⁷;
   R¹⁻¹, R¹⁻², R¹⁻³, R¹⁻⁴, R¹⁻⁵, R¹⁻⁶ and R¹⁻⁷ are each halogen, hydroxyl, cyano, C₁₋₆ alkyl, C₂₋₆ alkenyl, C₂₋₆ alkynyl, -OC₁₋₆ alkyl, -SC₁₋₆ alkyl, -C(=O)R^{1a}, -NR^{1b1}R^{1b2}, -C(=O)OR^{1c}, - C(=O)NR^{1d1}R^{1d2}, -S(O)₂NR^{1c1}R^{1c2}, -S(O)₂R^{1f}, C₃₋₁₀ cycloalkyl, a "4- to 12-membered heterocycloalkyl containing 1 to 3 heteroatoms each independently selected from O, S and N", C₆₋₂₀ aryl, a "5- to 12-membered heteroaryl containing 1 to 4 heteroatoms each independently selected from O, S and N", C₄₋₈ cycloalkenyl, or a "4- to 8-membered heterocycloalkenyl containing 1 to 3 heteroatoms each independently selected from O, S and N"; when there are multiple substituents, they may be the same or different;
   R^{1a}, R^{1b1}, R^{1b2}, R^{1c}, R^{1d1}, R^{1d2}, R^{1c1}, R^{1c2} and R^{1f} are each independently hydrogen, C₁₋₆ alkyl, or C₁₋₆ alkyl substituted with one or more halogen atoms;
   n is 0, 1, 2, 3, 4, 5 or 6;
   R² is halogen, hydroxyl, cyano, oxo, C₁₋₆ alkyl, C₁₋₆ alkyl substituted with one or more R²⁻¹, C₂₋₆ alkenyl, C₂₋₆ alkynyl, -OC₁₋₆ alkyl, -SC₁₋₆ alkyl, -C(=O)R^{2a}, -NR^{2b1}R^{2b2}, -C(=O)OR^{2c} or - C(=O)NR^{2d1}R^{2d2}; alternatively, when n is 2, 3, 4, 5 or 6, any two R² are linked to each other, independently form, together with the atoms on the ring to which they are attached, a 3- to 8-membered carbocyclic ring, or a "3- to 8-membered heterocyclic ring containing 1 to 3 heteroatoms each independently selected from O, S and N";
   R²⁻¹ is halogen, cyano, C₁₋₆ alkyl-O-, hydroxyl, -C(=O)R^{22a}, -NR^{22b1}R^{22b2}, -C(=O)OR^{22c} or - C(=O)NR^{22d1}R^{22d2};
   R^{2a}, R^{2b1}, R^{2b2}, R^{2c}, R^{2d1}, R^{2d2}, R^{22a}, R^{22b1}, R^{22b2}, R^{22c}, R^{22d1} and R^{22d2} are each independently hydrogen, C₁₋₆ alkyl, or C₁₋₆ alkyl substituted with one or more halogen atoms;
   R³ is a "5- to 6-membered heteroaryl containing 1 to 4 heteroatoms each independently selected from O, S and N", or a "5- to 6-membered heteroaryl containing 1 to 4 heteroatoms each independently selected from O, S and N" and substituted with one or more R³⁻¹;
   R³⁻¹ is halogen, hydroxyl, amino, cyano, C₁₋₆ alkyl, C₁₋₆ alkyl substituted with one or more halogen atoms, C₁₋₆ alkyl substituted with one or more -OC₁₋₆ alkyl, -OC₁₋₆ alkyl, -SC₁₋₆ alkyl, -OC₁₋₆ alkyl substituted with one or more halogen atoms, pentafluorosulfanyl, or -SC₁₋₆ alkyl substituted with one or more halogen atoms; when there are multiple substituents, they may be the same or different;
   R⁴ is hydrogen, halogen, C₁₋₆ alkyl, or C₁₋₆ alkyl substituted with one or more halogen atoms; R⁵ is hydrogen, C₁₋₆ alkyl, or C₁₋₆ alkyl substituted with one or more R⁵⁻¹;
   R⁵⁻¹ is deuterium, halogen, cyano, C₂₋₆ alkenyl, C₂₋₆ alkynyl, hydroxyl, -OC₁₋₆ alkyl, -C(=O)R^{5a}, -NR^{5b1}R^{5b2}, -C(=O)OR^{5c}, -C(=O)NR^{5d1}R^{5d2}, C₆₋₂₀ aryl, C₆₋₂₀ aryl substituted with one or more R⁵⁻¹⁻¹, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R⁵⁻¹⁻², C₃₋₁₂ cycloalkyl, C₃₋₁₂ cycloalkyl substituted with one or more R⁵⁻¹⁻³ , a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R⁵⁻¹⁻⁴, C₄₋₈ cycloalkenyl, C₄₋₈ cycloalkenyl substituted with one or more R⁵⁻¹⁻⁵, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R⁵⁻¹⁻⁶; when there are multiple substituents, they may be the same or different;
   R⁵⁻¹⁻¹, R⁵⁻¹⁻², R⁵⁻¹⁻³, R⁵⁻¹⁻⁴, R⁵⁻¹⁻⁵ and R⁵⁻¹⁻⁶ are each independently halogen, C₁₋₆ alkyl, or C₁₋₆ alkyl substituted with one or more halogen atoms;
   R^{5a}, R^{5b1}, R^{5b2}, R^{5c}, R^{5d1} and R^{5d2} are each independently hydrogen, C₁₋₆ alkyl, or C₁₋₆ alkyl substituted with one or more halogen atoms;
   The heteroaromatic sulfonamide structural compound represented by formula I is not any one of the following compounds:
      N-(1-methylcyclopropyl)-3-(5-(difluoromethyl)-1,3,4-thiadiazol-2-yl)-1-ethyl-6-fluoro-7-(4-isobutyrylpiperazin-1-yl)-1H-indazole-5-sulfonamide;
      N-(1-cyanocyclopropyl)-6-fluoro-7-(4-(isopropylsulfonyl)piperazin-1-yl)-1-methyl-3-(5-(trifluoromethyl)-1 ,3,4-thiadiazol-2-yl)-1H-indazole-5-sulfonamide.
Embodiment 2: the structure of the said heteroaromatic sulfonamide structural compound represented by formula I is as shown in formula II: 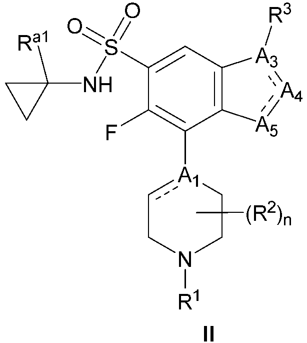
   wherein, "-̅ -̅ -̅" represents a single bond or a double bond;
   A₁ is C, CH or N;
   A₃ is C or N;
   A₄ is N or CR⁴;
   A₅ is NR⁵ or CR⁵; 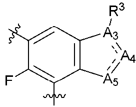
   is 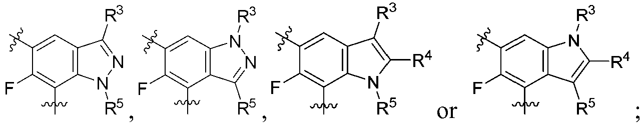
   R^{a1} is cyano or C₁₋₆ alkyl;
   R¹ is 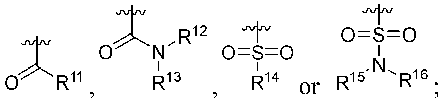
   R¹¹, R¹² R¹³, R¹⁴, R¹⁵ and R¹⁶ are each independently C₁₋₆ alkyl, C₁₋₆ alkyl substituted with one or more R¹⁻¹, C₃₋₁₀ cycloalkyl, C₃₋₁₀ cycloalkyl substituted with one or more R¹⁻², a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R¹⁻³, C₆₋₂₀ aryl, C₆₋₂₀ aryl substituted with one or more R¹⁻⁴, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R¹⁻⁵, C₄₋₈ cycloalkenyl, C₄₋₈ cycloalkenyl substituted with one or more R¹⁻⁶, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R¹⁻⁷;
   R¹⁻¹, R¹⁻², R¹⁻³, R¹⁻⁴ R¹⁻⁵, R¹⁻⁶ and R¹⁻⁷ are each halogen, hydroxyl, cyano, C₁₋₆ alkyl, C₂₋₆ alkenyl, C₂₋₆ alkynyl, -OC₁₋₆ alkyl, -SC₁₋₆ alkyl, -C(=O)R^{1a}, -NR^{1b1}R^{1b2}, -C(=O)OR^{1c}, -C(=O)NR^{1d1}R^{1d2}, -S(O)₂NR^{1c1}R^{1c2}, -S(O)₂R^{1f}, C₃₋₁₀ cycloalkyl, a "4- to 12-membered heterocycloalkyl containing 1 to 3 heteroatoms each independently selected from O, S and N", C₆₋₂₀ aryl, a "5-to 12-membered heteroaryl containing 1 to 4 heteroatoms each independently selected from O, S and N", C₄₋₈ cycloalkenyl, or a "4- to 8-membered heterocycloalkenyl containing 1 to 3 heteroatoms each independently selected from O, S and N"; when there are multiple substituents, they may be the same or different;
   R^{1a}, R^{1b1}, R^{1b2}, R^{1c}, R^{1d1}, R^{1d2}, R^{1c1}, R^{1c2} and R^{1f} are independently hydrogen, C₁₋₆ alkyl,
   or C₁₋₆ alkyl substituted with one or more halogen atoms;
   n is 0, 1, 2, 3, 4, 5 or 6;
   R² is halogen, hydroxyl, cyano, oxo, C₁₋₆ alkyl, C₁₋₆ alkyl substituted with one or more R²⁻¹, C₂₋₆ alkenyl, C₂₋₆ alkynyl, -OC₁₋₆ alkyl, -SC₁₋₆
   alkyl, -C(=O)R^{2a}, -NR^{2b1}R^{2b2}, -C(=O)OR^{2c} or -C(=O)NR^{2d1}R^{2d2}; alternatively, when n is 2, 3, 4, 5 or 6, any two R² are linked to each other, independently form, together with the atoms on the ring to which they are attached, a 3- to 8-membered carbocyclic ring, or a "3- to 8-membered heterocyclic ring containing 1 to 3 heteroatoms each independently selected from O, S and N"; R²⁻¹ is halogen, cyano, C₁₋₆ alkyl-O-,
   hydroxyl, -C(=O)R^{22a}, -NR^{22b1}R^{22b2}, -C(=O)OR^{22c} or -C(=O)NR^{22d1}R^{22d2};
   R^{2a}, R^{2b1}, R^{2b2}, R^{2c}, R^{2d1}, R^{2d2}, R^{22a}, R^{22b1}, R^{22b2}, R^{22c}, R^{22d1} and R^{22d2} are
   independently hydrogen, C₁₋₆ alkyl, or C₁₋₆ alkyl substituted with one or more halogen atoms; R³ is a "5- to 6-membered heteroaryl containing 1 to 4 heteroatoms each independently selected from O, S and N", or a "5- to 6-membered heteroaryl containing 1 to 4 heteroatoms each independently selected from O, S and N" and substituted with one or more R³⁻¹;
   R³⁻¹ is halogen, hydroxyl, amino, cyano, C₁₋₆ alkyl, C₁₋₆ alkyl substituted with one or more halogen atoms, C₁₋₆ alkyl substituted with one or more -OC₁₋₆ alkyl, -OC₁₋₆ alkyl, -SC₁₋₆ alkyl, -OC₁₋₆ alkyl substituted with one or more halogen atoms, pentafluorosulfanyl, or -SC₁₋₆ alkyl substituted with one or more halogen atoms; when there are multiple substituents, they may be the same or different;
   R⁴ is hydrogen, halogen, C₁₋₆ alkyl, or C₁₋₆ alkyl substituted with one or more halogen atoms; R⁵ is hydrogen, C₁₋₆ alkyl, or C₁₋₆ alkyl substituted with one or more R⁵⁻¹;
   R⁵⁻¹ is deuterium, halogen, cyano, C₂₋₆ alkenyl, C₂₋₆ alkynyl, hydroxyl, -OC₁₋₆ alkyl, -C(=O)R^{5a}, -NR^{5b1}R^{5b2}, -C(=O)OR^{5c,} -C(=O)NR^{5d1}R^{5d2}, C₆₋₂₀ aryl, C₆₋₂₀ aryl
   substituted with one or more R⁵⁻¹⁻¹, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R⁵⁻¹⁻², C₃₋₁₂ cycloalkyl, C₃₋₁₂ cycloalkyl substituted with one or more R⁵⁻¹⁻³, a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R⁵⁻¹⁻⁴, C₄₋₈ cycloalkenyl, C₄₋₈ cycloalkenyl substituted with one or more R⁵⁻¹⁻⁵, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R⁵⁻¹⁻⁶; when there are multiple substituents, they may be the same or different;
   R⁵⁻¹⁻¹, R⁵⁻¹⁻², R⁵⁻¹⁻³, R⁵⁻¹⁻⁴ , R⁵⁻¹⁻⁵ and R⁵⁻¹⁻⁶ are independently halogen, C₁₋₆ alkyl, or C₁₋₆ alkyl substituted with one or more halogen atoms;
   R^{5a}, R^{5b1}, R^{5b2}, R^{5c}, R^{5d1} and R^{5d2} are independently hydrogen, C₁₋₆ alkyl, or C₁₋₆ alkyl substituted with one or more halogen atoms;
   The heteroaromatic sulfonamide structural compound represented by formula I is not any one of the following compounds:
      N-(1-methylcyclopropyl)-3-(5-(difluoromethyl)-1,3,4-thiadiazol-2-yl)-1-ethyl-6-fluoro-7-(4-isobutyrylpiperazin-1-yl)-1H-indazole-5-sulfonamide;
      N-(1-cyanocyclopropyl)-6-fluoro-7-(4-(isopropylsulfonyl)piperazin-1-yl)-1-methyl-3-(5-(trifluoromethyl)-1,3,4-thiadiazol-2-yl)-1H-indazole-5-sulfonamide.
Embodiment 3: in the structure represented by formula I,
   A₃ is C;
   A₄ is N;
   A₅ is NR⁵; 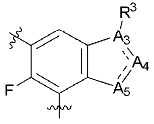
   is 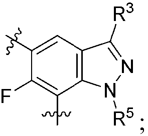
   R^{a1} is cyano or C₁₋₆ alkyl;
   Ring Y is a "4- to 12-membered heterocycloalkyl containing 1 to 3 heteroatoms each independently selected from O, S and N", or a "4- to 8-membered heterocycloalkenyl containing 1 to 3 heteroatoms each independently selected from O, S and N";
   R¹ is 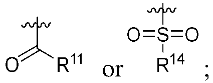
   R¹¹ and R¹⁴ are independently C₁₋₆ alkyl;
   n is 0, 1 or 2;
   R² is cyano or C₁₋₆ alkyl;
   R³ is a "5- to 6-membered heteroaryl containing 1 to 4 heteroatoms each independently selected from O, S and N" and substituted with one or more R³⁻¹;
   R³⁻¹ is C₁₋₆ alkyl substituted with one or more halogen atoms;
   R⁵ is C₁₋₆ alkyl;
   The heteroaromatic sulfonamide structural compound represented by formula I is not any one of the following compounds:
      N-(1-methylcyclopropyl)-3-(5-(difluoromethyl)-1,3,4-thiadiazol-2-yl)-1-ethyl-6-fluoro-7-(4-isobutyrylpiperazin-1-yl)-1H-indazole-5-sulfonamide;
      N-(1-cyanocyclopropyl)-6-fluoro-7-(4-(isopropylsulfonyl)piperazin-1-yl)-1-methyl-3-(5-(trifluoromethyl)-1,3,4-thiadiazol-2-yl)-1H-indazole-5-sulfonamide.

In one embodiment, the structure of the heteroaromatic sulfonamide structural compound represented by formula I or formula II is as shown in formula III: 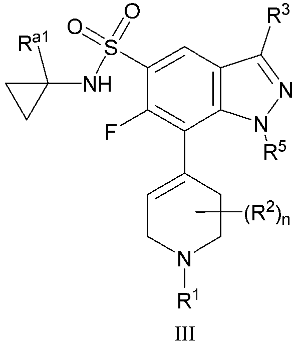
R^{a1} is cyano or C₁₋₆ alkyl;
R¹ is 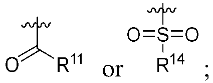
R¹¹ and R¹⁴ are independently C₁₋₆ alkyl;
n is 0 or 1;
R² is C₁₋₆ alkyl;
R³ is a "5- to 6-membered heteroaryl containing 1 to 4 heteroatoms each independently selected from O, S and N" and substituted with one or more R³⁻¹;
R³⁻¹ is C₁₋₆ alkyl substituted with one or more halogen atoms;
R⁵ is C₁₋₆ alkyl.

In one embodiment, when n is 1, 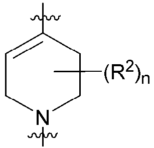 is

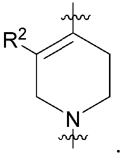

In one embodiment, R¹ is

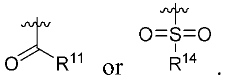

In one embodiment, A₃ is C.

In one embodiment, A₄ is N.

In one embodiment, A₅ is NR⁵.

In one embodiment, 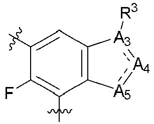 is

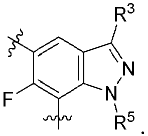

In one embodiment, R^{a1} is cyano or C₁₋₆ alkyl.

In one embodiment, when the definitions of R^{a1}, R¹¹, R¹², R¹³, R¹⁴, R¹⁵, R¹⁶, R¹⁻¹, R¹⁻², R¹⁻³, R¹⁻⁴, R¹⁻⁵, R¹⁻⁶, R¹⁻⁷, R^{1a}, R^{1b1}, R^{1b2}, R^{1c}, R^{1d1}, R^{1d2}, R^{1e1}, R^{1e2}, R^{1f}, R², R²⁻¹, R²⁻², R^{2a}, R^{2b1}, R^{2b2}, R^{2c}, R^{2d1}, R^{2d2}, R^{22a}, R^{22b1}, R^{22b2}, R^{22c}, R^{22d1}, R^{22d2}, R³⁻¹, R⁴, R⁵, R⁵⁻¹, R⁵⁻¹⁻¹, R⁵⁻¹⁻², R⁵⁻¹⁻³, R⁵⁻¹⁻⁴, R⁵⁻¹⁻⁵, R⁵⁻¹⁻⁶, R^{5a}, R^{5b1}, R^{5b2}, R^{5c}, R^{5d1} and R^{5d2} refer to C₁₋₆ alkyl, the said C₁₋₆ alkyl is C₁₋₄ alkyl, for example, methyl, ethyl, n-propyl, isopropyl, n-butyl, sec-butyl, isobutyl or tert-butyl.

In one embodiment, R^{a1} is cyano or methyl.

In one embodiment, R¹ is

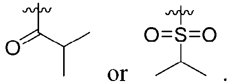

In one embodiment, n is 0, 1, or 2.

In one embodiment, R² is cyano, C₃₋₈ cycloalkyl or C₁₋₆ alkyl.

In one embodiment, R² is cyano, cyclopropyl, ethyl, n-propyl or methyl.

In one embodiment, when R³ is a "5- to 6-membered heteroaryl containing 1 to 4 heteroatoms each independently selected from O, S and N", or a "5- to 6-membered heteroaryl containing 1 to 4 heteroatoms each independently selected from O, S and N" and substituted with one or more R³⁻¹, the said R³ is a "5- to 6-membered heteroaryl containing 1 to 3 heteroatoms each independently selected from S and N".

In one embodiment, when R³⁻¹ is a C₁₋₆ alkyl substituted with one or more halogen atoms, the said R³⁻¹ is a C₁₋₂ alkyl substituted with two or three halogen atoms, for example, a methyl substituted with two or three fluorine atoms.

In one embodiment, R³ is

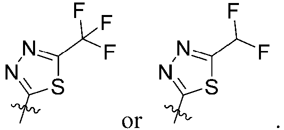

In one embodiment, R⁵ is methyl.

In one embodiment, when ring Y is a "4- to 12-membered heterocycloalkyl containing 1 to 3 heteroatoms each independently selected from O, S and N", the said ring Y is a "4- to 8-membered heterocycloalkyl containing 1 to 2 heteroatoms each independently selected from O, S and N", for example, a 4- to 6-membered heterocycloalkyl containing 2 nitrogen heteroatoms. In one embodiment, when ring Y is a "4- to 8-membered heterocycloalkenyl containing 1 to 3 heteroatoms each independently selected from O, S and N", the said ring Y is a "4- to 6-membered heterocycloalkenyl containing 1 to 3 heteroatoms each independently selected from O, S and N", for example, a 4- to 6-membered heterocycloalkenyl containing one nitrogen heteroatom.

In one embodiment, ring Y is

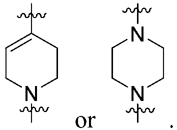

In one embodiment, 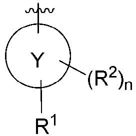 is

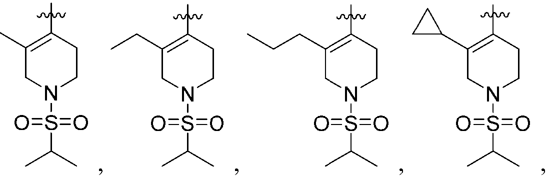

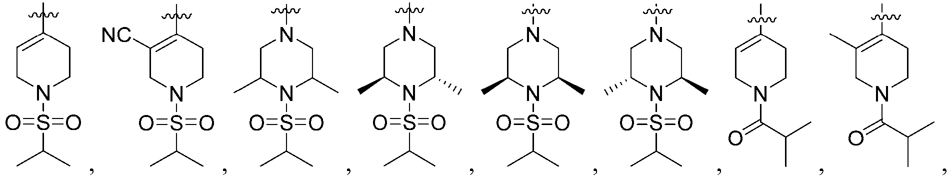

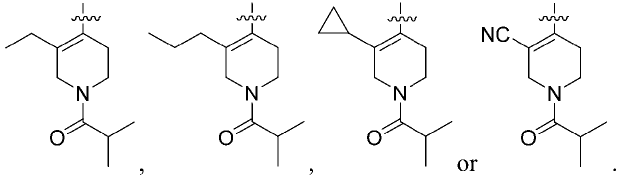

In one embodiment, among the heteroaromatic sulfonamide structural compound represented by formula I, a pharmaceutically acceptable salt thereof, a stereoisomer thereof, a tautomer thereof or an isotopically labeled compound thereof, the compound represented by formula I is any one of the following structures:

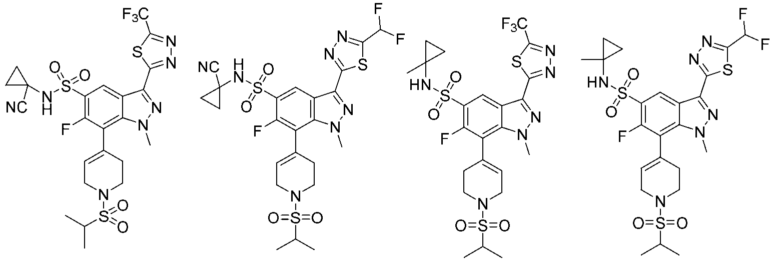

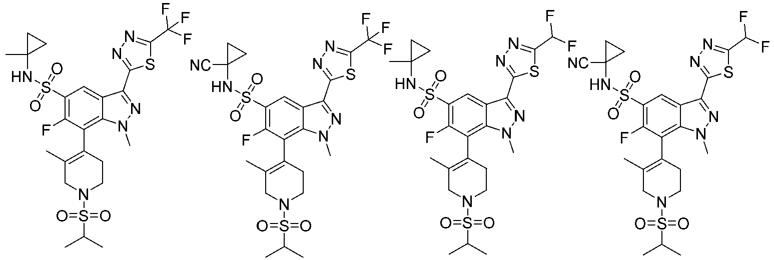

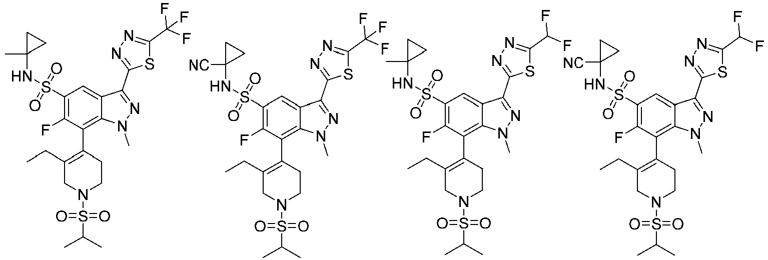

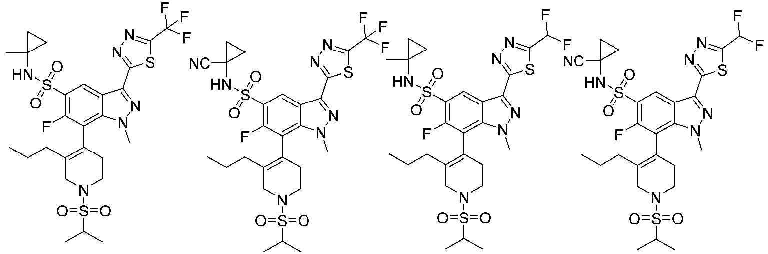

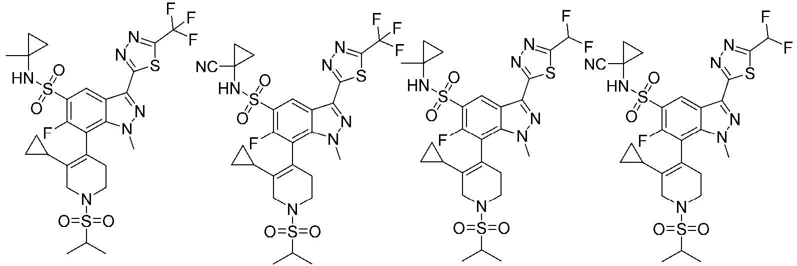

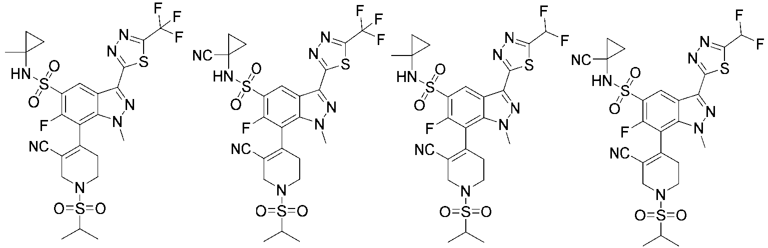

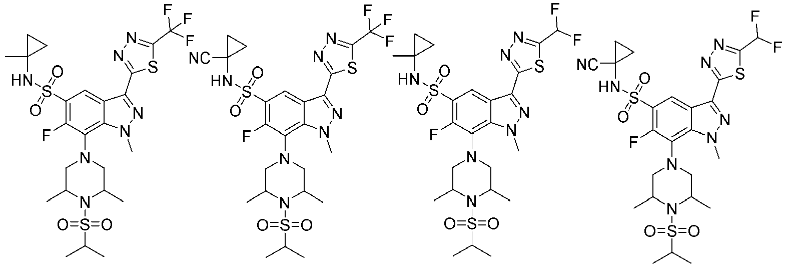

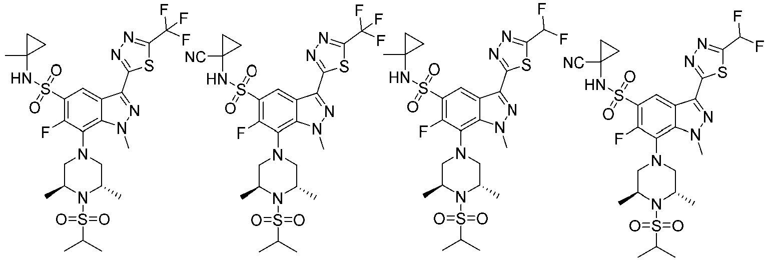

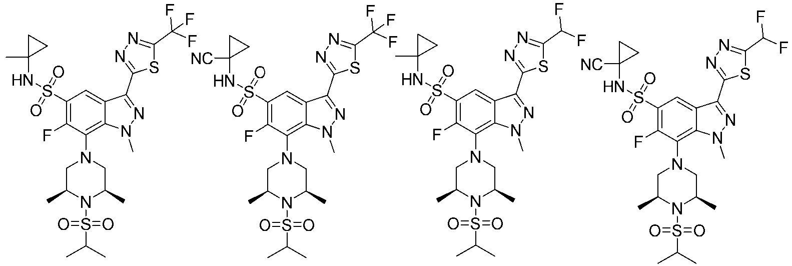

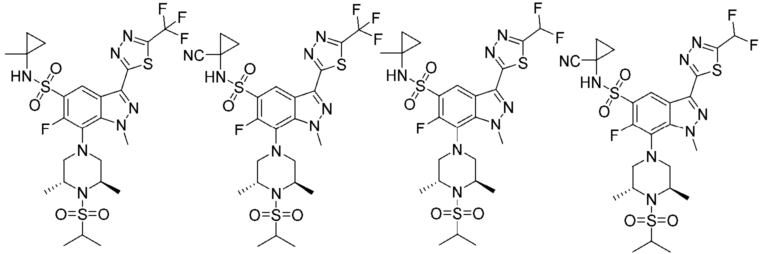

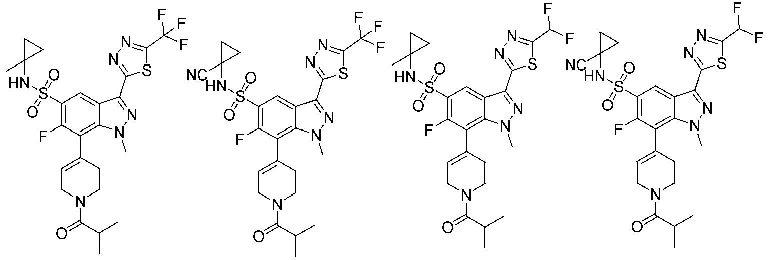

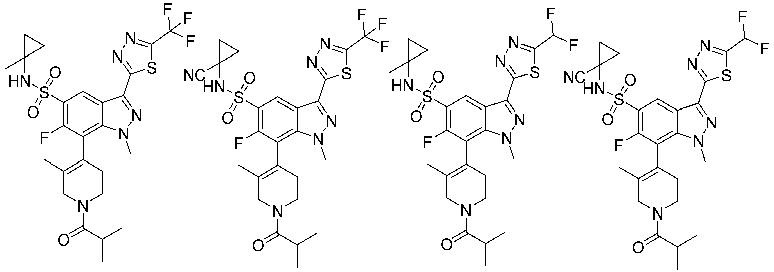

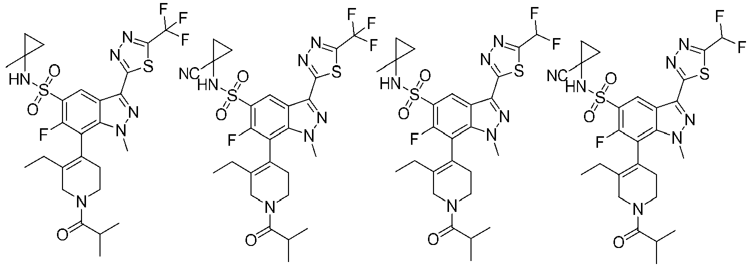

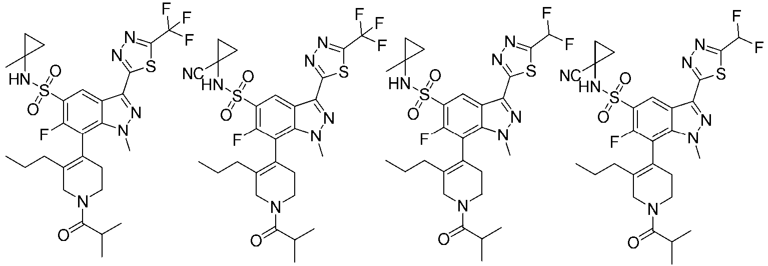

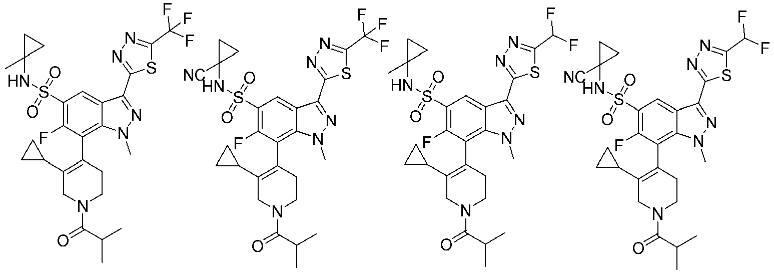

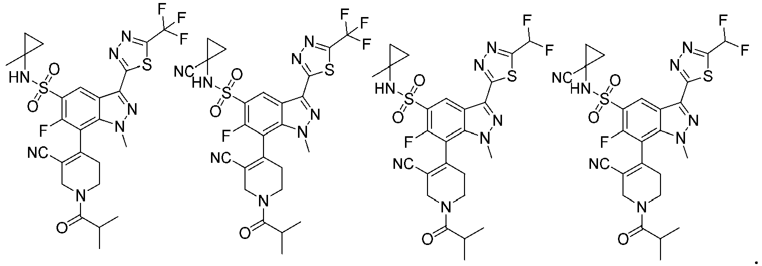

The present disclosure further provides a pharmaceutical composition, which comprises substance A and pharmaceutically acceptable excipients; wherein the said substance A is a therapeutically effective amount of the aforementioned heteroaromatic sulfonamide structural compound represented by formula I, a pharmaceutically acceptable salt thereof, a stereoisomer thereof, a tautomer thereof or an isotopically labeled compound thereof.

The present disclosure further provides an use of substance A in the preparation of a PARG inhibitor, wherein the said substance A is the aforementioned heteroaromatic sulfonamide structural compound represented by formula I, a pharmaceutically acceptable salt thereof, a stereoisomer thereof, a tautomer thereof or an isotopically labeled compound thereof.

The present disclosure further provides a method for inhibiting PARG by using substance A, which comprises administering an effective amount of substance A to a subject. Wherein the said substance A is the aforementioned heteroaromatic sulfonamide structural compound represented by formula I, a pharmaceutically acceptable salt thereof, a stereoisomer thereof, a tautomer thereof or an isotopically labeled compound thereof.

The present disclosure further provides an use of substance A in the preparation of a medicament, wherein the said medicament is used for the treatment or prevention of PARG-mediated diseases; the said substance A is the aforementioned heteroaromatic sulfonamide structural compound represented by formula I, a pharmaceutically acceptable salt thereof, a stereoisomer thereof, a tautomer thereof or an isotopically labeled compound thereof.

The present disclosure further provides a method for treating or preventing PARG-mediated diseases by using substance A, which comprises administering an effective amount of substance A to a subject. Wherein the said substance A is the aforementioned heteroaromatic sulfonamide structural compound represented by formula I, a pharmaceutically acceptable salt thereof, a stereoisomer thereof, a tautomer thereof or an isotopically labeled compound thereof.

The present disclosure further provides an use of substance A in the preparation of a medicament for the treatment or prevention of cancer. Wherein the said substance A is the aforementioned heteroaromatic sulfonamide structural compound represented by formula I, a pharmaceutically acceptable salt thereof, a stereoisomer thereof, a tautomer thereof or an isotopically labeled compound thereof. Examples of the said cancer include one or more of colon cancer, appendiceal cancer, pancreatic cancer, MYH-associated polyposis, hematological cancer, breast cancer, endometrial cancer, gallbladder cancer, cholangiocarcinoma, prostate cancer, lung cancer, brain cancer, ovarian cancer, cervical cancer, testicular cancer, renal cancer, head and neck cancer, bone cancer, skin cancer, rectal cancer, liver cancer, esophageal cancer, gastric cancer, thyroid cancer, bladder cancer, lymphoma, leukemia and melanoma.

The present disclosure further provides a method for treating or preventing cancer by using substance A, which comprises administering an effective amount of substance A to a subject. Wherein the said substance A is the aforementioned heteroaromatic sulfonamide structural compound represented by formula I, a pharmaceutically acceptable salt thereof, a stereoisomer thereof, a tautomer thereof or an isotopically labeled compound thereof. Examples of the said cancer include one or more of colon cancer, appendiceal cancer, pancreatic cancer, MYH-associated polyposis, hematological cancer, breast cancer, endometrial cancer, gallbladder cancer, cholangiocarcinoma, prostate cancer, lung cancer, brain cancer, ovarian cancer, cervical cancer, testicular cancer, renal cancer, head and neck cancer, bone cancer, skin cancer, rectal cancer, liver cancer, esophageal cancer, gastric cancer, thyroid cancer, bladder cancer, lymphoma, leukemia and melanoma.

The term "pharmaceutically acceptable salt" refers to a salt prepared from a compound of the present disclosure and a relatively non-toxic, pharmaceutically acceptable acid or base. When the compounds of the present disclosure contain relatively acidic functional groups, base addition salts can be obtained by contacting the neutral form of such compounds with a sufficient amount of a pharmaceutically acceptable base in a pure solution or a suitable inert solvent. Pharmaceutically acceptable base addition salts include, but are not limited to, lithium salt, sodium salt, potassium salt, calcium salt, aluminum salt, magnesium salt, zinc salt, bismuth salt, ammonium salt and diethanolamine salt.When the compounds of the present disclosure contain relatively basic functional groups, acid addition salts can be obtained by contacting the neutral form of such compounds with a sufficient amount of a pharmaceutically acceptable acid in a pure solution or a suitable inert solvent. The said pharmaceutically acceptable acids include inorganic acids, which include, but are not limited to, hydrochloric acid, hydrobromic acid, hydroiodic acid, nitric acid, carbonic acid, phosphoric acid, phosphorous acid, sulfuric acid, etc. The said pharmaceutically acceptable acids also include organic acids, which include, but are not limited to, acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, acid citrate, oleic acid, tannic acid, pantothenic acid, bitartrate, ascorbic acid, gentisic acid, fumaric acid, gluconic acid, saccharic acid, formic acid, ethanesulfonic acid, pamoic acid (i.e., 4,4'-methylene-bis(3-hydroxy-2-naphthoic acid)), amino acids (e.g., glutamic acid, arginine), etc. When the compounds of the present disclosure contain both relatively acidic and relatively basic functional groups, they can be converted into either base addition salt or acid addition salt. For details, reference may be made to Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, eds., Wiley-VCH, 2002).

In the present disclosure, when there are a multiple substituents, they may be the same or different.

The term "stereoisomer" refers to isomers that arise from the identical connectivity of atoms or atomic groups in a molecule but differ in their spatial arrangement, such as cis-trans isomers, optical isomers, or atropisomers, etc. These stereoisomers can be separated, purified, and enriched by asymmetric synthesis methods or chiral separation methods (including, but not limited to, thin-layer chromatography, rotational chromatography, column chromatography, gas chromatography, high-performance liquid chromatography, etc.). They can also be obtained via chiral resolution through bonding (e.g., chemical conjugation) or salt formation (e.g., physical association) with other chiral compounds.

The term "tautomer" refers to functional group isomers formed by the rapid migration of a certain atom between two positions within a molecule. For example, acetone and 1-propen-2-ol be converted into each other through the rapid migration of a hydrogen atom between the oxygen atom and the α-carbon atom.

The term "isotopically labeled compound" refers to a compound in which one or more atoms are replaced by one or more atoms with a specific atomic mass or mass number. Examples of isotopes that can be incorporated into the compounds of the present disclosure include, but are not limited to, isotopes of hydrogen, carbon, nitrogen, oxygen, fluorine, sulfur, and chlorine (e.g., ²H, ³H, ¹³C, ¹⁴C, ¹⁵N, ¹⁸O, ¹⁷O, ¹⁸F, ³⁵S, and ³⁶Cl). The isotopically labeled compounds of the present disclosure can generally be prepared by replacing non-isotopically labeled reagents with isotopically labeled reagents in accordance with the methods described herein.

The term "halogen" refers to fluorine, chlorine, bromine or iodine.

The term "alkyl" refers to a straight-chain or branched-chain alkyl group having a specified number of carbon atoms. Examples of alkyl groups include methyl, ethyl, n-propyl, isopropyl, n-butyl, tert-butyl, isobutyl, sec-butyl, n-pentyl, n-hexyl, n-heptyl, n-octyl and similar alkyl groups thereof.

The terms "cycloalkyl" and "carbocycle" refer to saturated cyclic groups consisting solely of carbon atoms, having a specified number of carbon atoms (e.g., C₃-C₆), which may be monocyclic, bridged cyclic or spirocyclic. Cycloalkyl groups include, but are not limited to, cyclopropyl, cyclobutyl, cyclopentyl, cyclohexyl, etc.

The term "aryl" refers to an aromatic group composed solely of carbon atoms, wherein each ring exhibits aromaticity. Examples include phenyl or naphthyl.

The term "heteroaryl" refers to a cyclic group having a specified number of ring atoms (e.g., 5 ~ 12-membered), a specified number of heteroatoms (e.g., 1, 2, or 3), and a specified type of heteroatoms (one or more of N, O, and S), which may be monocyclic or polycyclic, and at least one of the rings possesses aromaticity (in compliance with Hückel's rule). A heteroaryl group is linked to other moieties in the molecule either via an aromatic ring or a non-aromatic ring. Heteroaryl groups include, but are not limited to, furyl, pyrrolyl, thienyl, pyrazolyl, imidazolyl, oxazolyl, thiazolyl, pyridyl, pyrimidinyl, indolyl, etc.

The term "hydroxyl group" refers to a -OH group.

The term "cyano group" refers to a -CN group.

For a substituent defined with a carbon number range denoted in the form of "Cₓ₁-C_{y1}" (wherein ₓ₁ and _{y1} are integers), such as "Cₓ₁-C_{y1}" alkyl, "Cₓ₁-C_{y1}" cycloalkyl, "Cₓ₁-C_{y1}" cycloalkenyl, "Cₓ₁-C_{y1}" alkoxy, "Cₓ₁-C_{y1}" alkenyl, "Cₓ₁-C_{y1}" alkynyl, "Cₓ₁-C_{y1}" aryl, "Cₓ₁-C_{y1}" heteroaryl or "Cₓ₁-C_{y1}" heterocyclyl, the specified carbon number range excludes the carbon atoms of any substituents attached thereto. For example, a C₁₋₆ alkyl refers to an alkyl group having 1 to 6 carbon atoms, excluding the carbon atoms of any substituents.

On the premise of not violating the common general knowledge in the art, the aforementioned preferred conditions can be combined arbitrarily to obtain various preferred embodiments of the present disclosure.

All the reagents and raw materials used in the present disclosure are commercially available.

The positive and progressive effects of the present disclosure are as follows: the present disclosure provides a heteroaromatic sulfonamide structural compound, a pharmaceutical composition thereof and use thereof. The said heteroaromatic sulfonamide compound exhibits favorable inhibitory activity against PARG, and is expected to be used for the treatment and/or prevention of various PARG-associated diseases.

### DETAILE DISCRIPTION OF THE EMBODIMENTS

The present disclosure is further illustrated by the following examples, but it is not intended to limit the present disclosure to the scope of the said examples. For the experimental methods without specifying the specific conditions in the following examples, they shall be selected in accordance with conventional methods and conditions or the product specifications.

In the present disclosure, room temperature refers to the ambient temperature, ranging from 10°C to 35°C. Overnight refers to a duration of 8 to 15 hours. Reflux refers to the reflux temperature of the solvent under atmospheric pressure.

All the solvents involved in the following examples are of analytical grade or chromatographic grade. Where the solvents involved in the following examples are mixed solvents, the ratios are by volume unless otherwise specified.

Following is a list of abbreviations used in the examples:
DCM dichloromethane
DMF *N, N*-dimethylformamide
EA ethyl acetate
DMSO Dimethyl sulfoxide
DMAP 4-dimethylamino-pyridine
DIPEA diisopropylethylamine
TEA Triethylamine
NCS N-Chlorosuccinimide
Pd(dtbpf)Cl₂ 1,1'-Bis(di-tert-butylphosphino)ferrocene palladium dichloride
Pd(Amphos)Cl₂ Bis(di-tert-butyl(4-dimethylaminophenyl)phosphine)dichloropalladium (II)
Pd₂(dba)₃ tris(dibenzylideneacetone)dipalladium
m-CPBA *m*-chloroperoxybenzoic acid
Xantphos 4,5-Bis(diphenylphosphino)-9,9-dimethylxanthene
RuPhos Dicyclohexyl(2',6'-diisopropoxy-[1,1'-biphenyl]-2-yl)phosphine
THF Tetrahydrofuran
THP 2-Tetrahydropyran
TFA Trifluoroacetic acid
Tf Trifluoromethanesulfonyl
DBDMH 1,3-Dibromo-5,5-Dimethylhydantoin
SEMCl (trimethyl silicon) ethoxymethyl
DHP Dihydropyran
DBH 1,3-Dibromo-5,5-Dimethylhydantoin
Boc t-Butyloxy carbonyl
RuPhos Pd G3 Methanesulfonato(2-dicyclohexylphosphino-2',6'-di-i-propoxy-1,1'-biphenyl)(2'-amino-1,1'-biphenyl-2-yl)palladium(II)
NMM 4-Methylmorpholine
[Ir(COD)OMe]₂ (1,5-Cyclooctadiene)(methoxy)iridium(I) Dimer

### Example 1 Synthetic route of compound 1

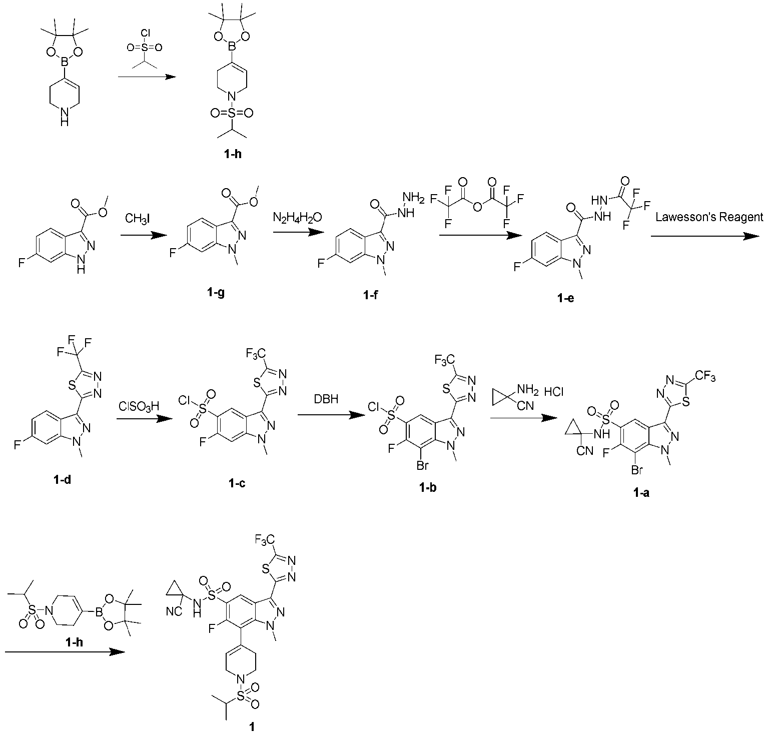

### Synthesis of compound 1-h

Compound 1,2,3,6-tetrahydropyridine-4-boronic acid pinacol ester (200 mg, 0.96 mmol) was dissolved in dichloromethane (20 mL), to which was added triethylamine (194 mg, 1.92 mmol), then isopropylsulfonyl chloride (164 mg, 1.15 mmol) dropwise under an ice bath, and the reaction mixture was stirred at room temperature for 2 hours. The reaction mixture was added water (100 mL), extracted with dichloromethane (100 mL), the organic phase was washed with brine (100 mL), dried over sodium sulfate, filtered off the desiccant, the filtrate was concentrated under reduced pressure to obtain the crude product. The crude product was purified by column chromatography (mobile phase, PE/EA 1/1) to give compound **1-h** (120mg, 40%). LC-MS (ESI): m/z 316.2 (M+H)⁺.

### Synthesis of compound 1-g

To a solution of methyl 6-fluoro-1H-indazole-3-carboxylate (2.5 g, 12.88 mmol) in DMF (10 mL) was added cesium carbonate (8.39 g, 25.75 mmol) and iodomethane (1.60 mL, 25.75 mmol) at room temperature. After addition, the reaction mixture was stirred at room temperature overnight. Upon completion, the reaction mixture was diluted by adding ethyl acetate, washed with water, washed with brine, dried over anhydrous sodium sulfate, filtered, and evaporated to dryness. The residue was purified by a flash column chromatography (mobile phase: ethyl acetate/petroleum ether 0% to 50%) to give compound **1-g** (1.4 g, 52%) as a white solid. LC-MS (ESI): m/z = 209.2 [M+H]⁺; ¹H NMR (DMSO-*d*₆, 400 MHz): δ 8.07 (1H, dd, *J* = 8.8, 5.2 Hz), 7.72 (1H, dd, *J* = 9.6, 2.0 Hz), 7.24 (1H, td, *J* = 9.2, 2.0 Hz), 4.13 (3H, s), 3.92 (3H, s).

### Synthesis of compound 1-f

To a reaction flask was added **1-g** (1.4 g, 6.72 mmol), hydrazine hydrate (5 mL) and ethanol (50 mL), the mixture was stirred at 60°C overnight. Upon completion, the reaction mixture was cooled to room temperature and concentrated to remove the organic solvent. The crude product was washed with ethanol (20 mL), filtered and the filter cake was dried to give compound **1-f** (1.2 g, 86%). LC-MS (ESI): m/z = 209.1 [M+H]⁺.

### Synthesis of compound 1-e

To a reaction flask, **1-f** (1.2 g, 5.76 mmol), triethylamine (2.4 mL, 17.29 mmol) and dichloromethane (20 mL) were added to a reaction flask under an ice-water bath and stirred for 10 min. Then trifluoroacetic anhydride (1.2 mL, 8.65 mmol) was slowly added dropwise to the reaction mixture and stirred at 0°C for 3 hours under protection of nitrogen. Upon completion, the reaction mixture was washed with saturated aqueous sodium bisulfate solution (40 mL*2). The organic phase was concentrated. The crude product was dissolved with ethyl acetate (5 mL), and the resulting mixture was slowly added dropwise to stirred petroleum ether (200 mL). Filtration and drying to give white solid compound **1-e** (1.5 g, 86%). LC-MS (ESI): m/z = 305.1 [M+H]⁺.

### Synthesis of compound 1-d

At room temperature, **1-e** (1.5 g, 4.93 mmol), Lawson's reagent (4.99 g, 12.33 mmol), and toluene (30 mL) were added to a reaction flask and the mixture was stirred at 75°C under atmosphere of nitrogen for 12 hours. Upon completion, the reaction mixture was quenched by the addition of saturated aqueous sodium carbonate solution (15 mL). The mixture was stirred at 35°C for 3 hours. The liquid was partitioned and the organic phase was concentrated. The crude product was added ethanol (18mL) and water (1.5mL) and stirred at 85°C for 3 hours. Cooled to 0°C, filtered and the filter cake was dried to give compound **1-d** (1.3 g, 87%) as a white solid. LC -MS (ESI): m/z = 303.0 [M+H]⁺.

### Synthesis of compound 1-c

To a reaction flask, chlorosulfonic acid (5.5 mL) was added under an ice bath and stirred at 0°C for 10 minutes. Then **1-d** (0.5 g, 1.65 mmol), was slowly added to the reaction mixture. After addition, the ice bath was withdrawn and the reaction mixture was heated to 65°C under protection of nitrogen and stirred for 18 hours. Upon completion, the reaction mixture was cooled to room temperature, and then was slowly added to ice-colded water (100 mL), filtered, and the filter cake was dried to give a white solid compound **1-c** (550 mg, 83%). LC-MS (ESI): m/z = 401.0 [M+H]⁺.

### Synthesis of compound 1-b

To a reaction flask was added **1-c** (550 mg, 1.37 mmol), dichloromethane (20 mL), dibromohydantoin (471 mg, 1.65 mmol), and the mixture was stirred under an ice-water bath for 10 min. Trifluoromethanesulfonic acid (3 mL) was then added slowly. After addition, the mixture was brought to room temperature and stirred for 6 hours. Upon completion, the reaction mixture was quenched by adding ice-colded water (100 mL) and extracted with dichloromethane (50 mL*2). The organic phases were combined, washed with brine, dried over anhydrous sodium sulfate, filtered, and the filtrate was concentrated under reduced pressure. The crude was pulped with (PE/EA=3/1) (30 mL) and filtered to give compound **1-b** (500 mg, 76%) as an earthy yellow solid. LC -MS (ESI): m/z = 479.0 [M+H]⁺.

### Synthesis of compound 1-a

A mixture of 1-amino-1-cyclopropanecarbonitrile hydrochloride (247 mg, 2.09 mmol) and pyridine (2 mL) was stirred for half an hour under an ice-colded water bath, then was added a solution of **1-b** (500 mg, 1.04 mmol) in dichloromethane (20 mL). After addition, the mixture was stirred for 3 hours at room temperature. Upon completion, the solvent was removed by concentration under reduced pressure and saturated sodium bisulfate was added until pH was adjusted to 4 - 5. The mixture was extracted with ethyl acetate (30 mL*3), the organic phases were combined, washed with brine, then dried over anhydrous sodium sulfate, filtered, and the filtrate was concentrated to obtain the crude product. The crude product was purified by flash column chromatography (mobile phase: ethyl acetate/petroleum ether: 0% to 100%) to give compound **1-a** (400 mg, 73%) as a yellowish solid.

### Synthesis of compound 1

To a reaction flask were added **1-a** (60 mg, 0.11 mmol), **1-h** (54 mg, 0.17 mmol), [1,1'-bis(di-tert-butylphosphino)ferrocene]palladium dichloride (15 mg, 0.023 mmol), cesium fluoride (52 mg, 0.34 mmol), 1,4-dioxane (9 mL) and H₂O (1 mL). After degassed and purged with nitrogen for 3 times, the reaction mixture was heated and stirred at 100°C for 5 hours. Upon completion, the reaction mixture was cooled to room temperature, concentrated under reduced pressure to remove the organic solvent.The residue was diluted by adding ethyl acetate, the organic phase was washed with water, brine, dried over anhydrous sodium sulfate, filtered and concentrated under reduced pressure to obtain the crude product. The crude product was purified by Prep-HPLC to give compound **1** (35 mg, 48%) as a white solid. LC-MS (ESI): m/z 634.2 [M+H]⁺; ¹H NMR (DMSO-*d₆*, 400 MHz): *δ* 9.55 (1H, s), 8.87 (1H, d, *J* = 6.8 Hz), 6.08 (1H, s), 4.21 (3H, s), 4.00-4.16 (2H, m), 3.55-3.69 (2H, m), 3.40-3.52 (1H, m), 2.49-2.59 (2H, m), 1.41-1.50 (2H, m), 1.32-1.39 (2H, m), 1.29 (6H, d, *J* = 6.8 Hz).

### Example 2 Synthetic route of compound 2

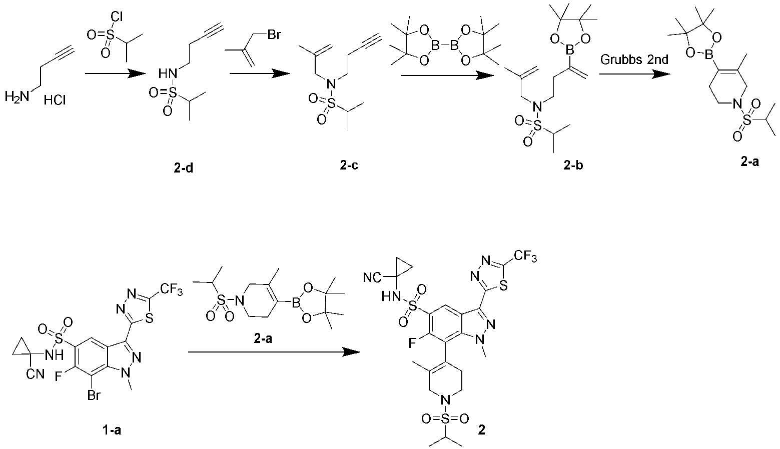

### Synthesis of compound 2-d

Tetrahydrofuran (70 mL) and water (70 mL) were added to a reaction flask. Anhydrous potassium carbonate (12.45 g, 90.09 mmol) was added under an ice bath. The mixture was stirred for 5 minutes under an ice bath and then was added 3-butyn-1-amine hydrochloride (3.17 g, 30.03 mmol). The reaction mixture was stirred under an ice bath for 10 minutes and then was added isopropylsulfonyl chloride (7.7 g, 54.00 mmol) dropwise. After addition, the reaction mixture was stirred under an ice bath for 5 minutes and then stirred at room temperature for 5 hours. The organic solvent was removed by rotary evaporation at room temperature and extracted twice with ethyl acetate. The organic phases were combined, washed with water, washed with brine, dried over anhydrous sodium sulfate, filtered and evaporated to give compound **2-d** (3.0 g, 57% yield).

### Synthesis of compound 2-c

To a reaction flask was added **2-d** (3.0 g, 17.12 mmol), acetone (20 mL) and anhydrous potassium carbonate (4.73 g, 34.24 mmol). After was added 3-Bromo-2-methylpropene (4.62 g, 34.24 mmol) dropwise at room temperature followed by tetrabutylammonium iodide (0.36 g, 0.98 mmol), the reaction mixture was heated and stirred at 60°C overnight. The solvent was removed by rotary evaporation and the residue was diluted with ethyl acetate and washed with water. The aqueous phase was extracted once with ethyl acetate, the organic phases were combined, dried over anhydrous sodium sulfate, filtered and evaporated to give compound **2-c** (3.9 g, 99% yield).

### Synthesis of compound 2-b

Lithium chloride (0.72g, 17.01mmol) in a reaction flask was heated by an electric heat gun at vacuum for 5 minutes. After degassed and purged with nitrogen, the mixture was added anhydrous DMF (40mL) and cuprous chloride (1.68g, 17.01mmol) was added. After degassed and purged with nitrogen twice, the mixture was stirred at room temperature for 1 hour, was added potassium acetate (1.67 g, 17.01 mmol) and bis-pinacolborate (4.32 g, 17.01 mmol) under protection of nitrogen. **2-c** (3.9 g, 17.01 mmol) was added after degassed and purged with nitrogen twice, the reaction mixture was stirred at room temperature overnight. The reaction mixture was filtered through celite and a solvent mixture of ice-colded water and ethyl acetate petroleum ether (about 3/1) was added to the filtrate. The filtrate was filtered through celite again, the organic phase was separated from the filtrate and the aqueous phase was extracted once with a solvent mixture of ethyl acetate and petroleum ether (about 3/1). The organic phases were combined, washed with water, washed with saturated ammonium chloride, washed with brine, dried over anhydrous sodium sulfate, filtered, evaporated to dryness, and purified by a column (mobile phase: petroleum ether/ethyl acetate, 100/0 to 80/20) to give compound **2-b** (2.8 g, 46%). ¹H NMR (CDCl₃, 400 MHz) *δ* 5.84 (1H, d, *J*=3.2 Hz), 5.67 (1H, d, *J*=3.2 Hz), 5.02 - 4.93 (2H, m), 3.85 (2H, s), 3.31 - 3.20 (3H, m), 2.44 - 2.34 (2H, m), 1.77 (3H, s), 1.35 (6H, d, *J*=6.8 Hz), 1.26 (12H, s).

### Synthesis of compound 2-a

**2-b** (2.8 g, 7.84 mmol) in a reaction flask was blown with argon for 1 min, anhydrous toluene (160 mL) was added and was blown with argon for 5 min. Grubbs 2nd catalyst (0.33 g, 0.39 mmol) was added and degassed and purged with argon for 3 times. The reaction mixture was heated and stirred at 100°C for 24 hours. Supplemented with Grubbs 2nd catalyst (0.055g, 0.065mmol) and degassed and purged with argon gas for 3 times, and the reaction mixture was heated and stirred at 100°C for 10 hours. The reaction mixture was cooled to room temperature, the solvent was removed by rotary evaporation, the residue was diluted with ethyl acetate, washed with water, washed with brine, dried over anhydrous sodium sulfate, filtered, evaporated, and purified by a column (the silica gel was mixed with 0.5 mL of triethylamine added into the solution, and the silica gel column was wetted with 200 mL of petroleum ether with 10% ethyl acetate containing 0.5% triethylamine, and then the column was washed with 200 mL of petroleum ether) (mobile phase: petroleum ether/ethyl acetate, 100/0 to 85/15), the resulting sample was then pulped with ethyl acetate petroleum ether to give compound **2-a** (1.0 g, 39% yield). LC-MS (ESI): m/z 330.2 (M+H)⁺.

### Synthesis of compound 2

To a reaction flask was added **1-a** (50 mg, 0.095 mmol), **2-a** (47 mg, 0.14 mmol), [1,1'-bis(di-tert-butylphosphino) ferrocene] palladium dichloride (12 mg, 0.019 mmol), cesium fluoride (43 mg, 0.28 mmol), 1,4-dioxane (9 mL) and H₂O (1 mL). After degassed and purged with nitrogen for 3 times, the reaction mixture was heated and stirred at 100°C for 5 hours. Upon completion, the reaction mixture was cooled to room temperature, concentrated under reduced pressure to remove the organic solvent, diluted by adding ethyl acetate to the residue, the organic phase was washed with water, brine, dried over anhydrous sodium sulfate, filtered and concentrated under reduced pressure to obtain the crude product. The crude product was purified by Prep-HPLC to give compound **2** (30 mg, 49%). LC-MS (ESI): m/z 648.1 [M+H]⁺; ¹H NMR (DMSO-*d₆,* 400 MHz): *δ* 9.54 (1H, s), 8.88 (1H, d, *J* = 6.4 Hz), 4.16 (3H, s), 3.91-4.07 (2H, m), 3.53-3.68 (2H, m), 3.41-3.53 (1H, m), 2.37-2.51 (2H, m), 1.50 (3H, s), 1.32-1.48 (4H, m), 1.29 (6H, d, *J* = 6.4 Hz).

### Example 3 Synthetic route of compound 3

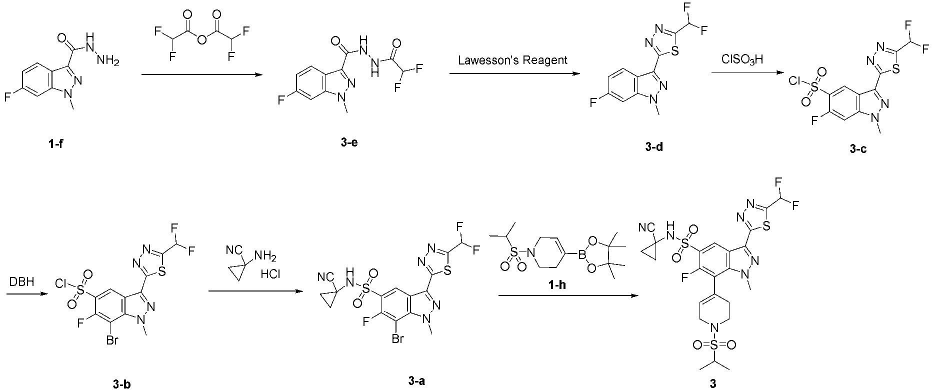

### Synthesis of compound 3-e

Compound **1-f** (900 mg, 4.32 mmol) was suspended in 20 mL of anhydrous DCM at room temperature, was added triethylamine (1.80 mL, 12.97 mmol) under an ice-water bath, followed by dropwise addition of difluoroacetic (806 uL, 6.48 mmol). The reaction mixture was stirred under a nitrogen ice-water bath for 2 hours. The reaction mixture was added 100 mL of water and extracted with DCM (100 mL). The organic phase was concentrated under reduced pressure and the residue was dissolved in ethyl acetate (100 mL), washed sequentially with 10% sodium bisulfate (100 mL), saturated sodium bicarbonate (100 mL), brine (100 mL), dried over anhydrous sodium sulfate, filtered, the filtrate was concentrated under reduced pressure, and the residue was dried in vacuum for 1 h to give compound **3-e** (1.2 g, 97%). LC-MS (ESI): m/z = 287.0 [M+H]⁺.

### Synthesis of compound 3-d

Compound **3-e** (1.20 g, 4.19 mmol) was dissolved in 50 mL of 1,4-dioxane at room temperature and Lawson's reagent (2.54 g, 6.29 mmol) was added. The reaction mixture was stirred under nitrogen at 80°C for 5 hours. The reaction mixture was cooled to room temperature, filtered, the filtrate was concentrated under reduced pressure. The residue was dissolved in ethyl acetate (100 mL), washed sequentially with saturated sodium bicarbonate solution (100 mL*2), brine (100 mL), dried over anhydrous sodium sulfate, filtered, the filtrate was concentrated under reduced pressure, and the residue was purified by a flash column chromatography (PE/EA=3:1) to give compound **3-d** (820 mg, 69%). LC-MS (ESI): m/z = 285.0 [M+H]⁺.

### Synthesis of compound 3-c

Compound **3-d** (820 mg, 2.88 mmol) was dissolved in 15 mL of chlorosulfonic acid at room temperature. The reaction mixture was stirred under nitrogen at 65°C for 18 hours. The reaction mixture was cooled to room temperature and slowly added dropwise to an ice-water mixture (200 mL). After the dropwise addition, the mixture was extracted with ethyl acetate (100 mL*2). The organic phase was dried over anhydrous sodium sulfate, filtered and the filtrate was concentrated under reduced pressure to give compound **3-c** (1.1 g, 100%). LC-MS (ESI): m/z = 383.0[M+H]⁺.

### Synthesis of compound 3-b

Dibromohydantoin (986 mg, 3.45 mmol) was added to a solution of compound **3-c** (1.1 g, 2.87 mmol) in 20 mL of DCM at room temperature, followed by dropwise addition of trifluoromethanesulfonic acid (0.56 mL) under an ice-colded water bath. After the dropwise addition, the reaction mixture was stirred under an ice-water bath for 10 minutes, then warmed to room temperature and stirred for 3 hours. The reaction mixture was slowly poured into ice-colded water (100 mL) and extracted with DCM (150 mL), the organic phase was washed sequentially with water (100 mL), brine (100 mL), dried over anhydrous sodium sulfate, filtered, and the filtrate was concentrated under reduced pressure, and the residue was suspended in a mixed solvent of petroleum ether and ethyl acetate (3:1, 20 mL), and dispersed by ultrasonication for 3 min, and the mixture was filtered. The filter cake was washed with mixed solvent (PE/EA=3:1, 10 mL), the solid was collected and dried under vacuum for 30 min to give compound **3-b** (1.3 g, 98%). LC-MS (ESI): m/z = 460.8[M+H]⁺.

### Synthesis of compound 3-a

Compound 1-amino-cyclopropanecarbonitrile hydrochloride (334 mg, 2.82 mmol) was dissolved in 10 mL of DCM at room temperature, followed by addition of pyridine (2.05 mL, 25.34 mmol) under an ice-colded water bath, stirred for 30 min under an ice-colded water bath. Compound **3-b** (650 mg, 1.41 mmol) dissolved in DCM (10 mL) and added dropwise to the above mixture under an ice-colded water bath, the reaction mixture was brought to room temperature and stirred overnight. The reaction mixture was concentrated under reduced pressure, and the residue was dissolved in 150 mL of ethyl acetate, washed sequentially with 10% sodium bisulfate solution (100 mL*2), brine (100 mL), dried over anhydrous sodium sulfate, filtered, and the filtrate was concentrated under reduced pressure, and the residue was purified by a flash column chromatography (EA/PE=0~100%) to give compound **3-a** (250 mg, 35%). LC-MS (ESI): m/z = 507.0[M+H]⁺.

### Synthesis of compound 3

Compound **3-a** (50 mg, 0.099 mmol) was dissolved in 18 mL of 1,4-dioxane at room temperature and the mixture was added compound **1-h** (47 mg, 0.15 mmol), 1,1'-di-tert-butylphosphinoferrocene palladium dichloride (13 mg, 0.020 mmol), cesium fluoride (45 mg, 0.30 mmol) and water (2 mL). The reaction mixture was stirred at 100°C under nitrogen for 7 hours. The reaction mixture was cooled to room temperature, concentrated under reduced pressure, and 50 mL of water was added to the residue and extracted with DCM (50 mL*2). The organic phase was dried over anhydrous sodium sulfate, filtered, and the filtrate was concentrated under reduced pressure to obtain the crude product. The crude product was purified by prep-HPLC (basic method, NH₄HCO₃) to give compound **3** (26.3 mg, 43%). LC-MS (ESI): m/z = 616.1 [M+H]⁺

### Example 4 Synthetic route of Compound 4

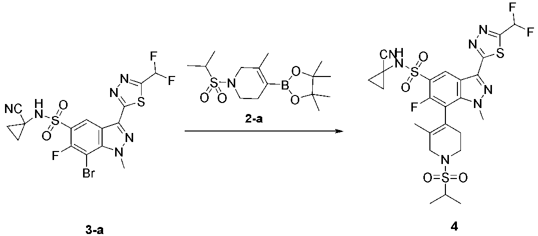

At room temperature, Compound 3-a (50 mg, 0.099 mmol) was dissolved in 18 mL of 1,4-dioxane and the mixture was added compound **2-a** (49 mg, 0.15 mmol), 1,1'-di-tert-butylphosphinoferrocene palladium dichloride (13 mg, 0.020 mmol), cesium fluoride (45 mg, 0.30 mmol) and water (2 mL). The reaction mixture was stirred at 100°C under nitrogen for 7 hours, then was cooled to room temperature, concentrated under reduced pressure. The residue was added 50 mL of water and extracted with DCM (50 mL*2). The organic phase was dried over anhydrous sodium sulfate, filtered, and the filtrate was concentrated under reduced pressure to obtain the crude product. The crude product was purified by prep-HPLC (basic method, NH₄HCO₃) to give compound **4** (7 mg, 11%). LC-MS (ESI): m/z = 630.1 [M+H]⁺; ¹H NMR (CDCl₃, 400MHz) *δ* 9.19(1H, d, *J*=6.4Hz), 7.11(1H, t, *J*=53.6Hz), 5.76(1H, s), 4.15(3H, s), 4.13-4.05(1H, m), 3.96-3.86(1H, m), 3.85-3.76(1H, m), 3.58-3.47(1H, m), 3.35-3.23(1H, m), 2.59-2.49(2H, m), 1.77-1.65(2H, m), 1.58(3H, s), 1.51-1.37(8H, m).

### Example 5 Synthetic route of compound 5

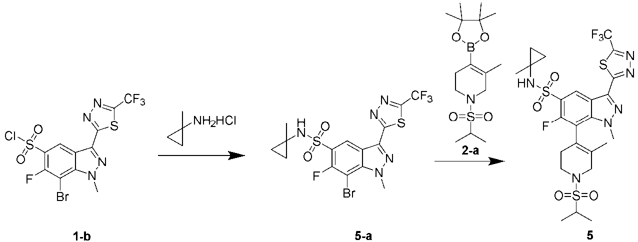

### Synthesis of compound 5-a

1-methylcyclopropanamine hydrochloride (135 mg, 1.25 mmol) and DIPEA (1290 mg, 10.00 mmol) were added to a reaction flask charged with **1-b** (600 mg, 1.25 mmol) and dichloromethane (100 mL) with stirring under an ice-colded water bath. After addition, the reaction mixture was slowly brought to room temperature. After stirred for 3 hours at room temperature, the reaction mixture was added saturated aqueous sodium bisulfate solution (100 mL), the organic phases were separated and the aqueous phase was extracted with dichloromethane (30 mL*2). The organic phases were combined, washed with brine, dried over anhydrous sodium sulfate, filtered and concentrated to give compound **5-a** (420 mg, 65%).

### Synthesis of compound 5

To a reaction vial were added **2-a** (50 mg, 0.15 mmol), **5-a** (52 mg, 0.10 mmol), [1,1'-bis(di-tert-butylphosphino)ferrocene] palladium(II) dichloride ( II ) (23 mg, 0.04 mmol), cesium fluoride (200 mg, 1.32 mmol), 1,4-dioxane (4 mL) and water (0.8 mL). After degassed and purged with nitrogen for three times, the reaction mixture was heated to 100°C and stirred for 12 hours. The reaction mixture was concentrated under reduced pressure, the residue was washed with ethyl acetate (10 mL*3), the organic phase was concentrated under reduced pressure and purified by prep-HPLC to give compound **5** (11.2 mg, 17%). LC-MS (ESI): m/z 637.1(M+H) +.

### Example 6 Synthetic route of compound 6

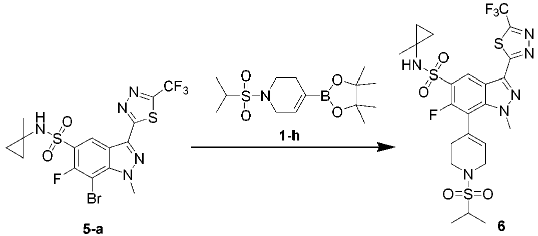

### Synthesis of compound 6

To a reaction flask was added **1-h** (32 mg, 0.10 mmol), **5-a** (52 mg, 0.10 mmol), [1,1'-bis(di-tert-butylphosphino)ferrocene] palladium(II) dichloride ( II ) (23 mg, 0.04 mmol), cesium fluoride (200 mg, 1.32 mmol), 1,4-dioxane (4 mL) and water (0.8 mL). After degassed and purged with nitrogen for three times, the reaction mixture was heated to 100°C and stirred for 12 hours. The reaction mixture was concentrated under reduced pressure, the residue was washed with ethyl acetate (10 mL*3), the organic phase was concentrated under reduced pressure and purified by prep-HPLC to give compound **6** (18.2 mg, 29%). LC-MS (ESI): m/z 623.1(M+H)⁺.

### Example 7 Synthetic route of compound 7

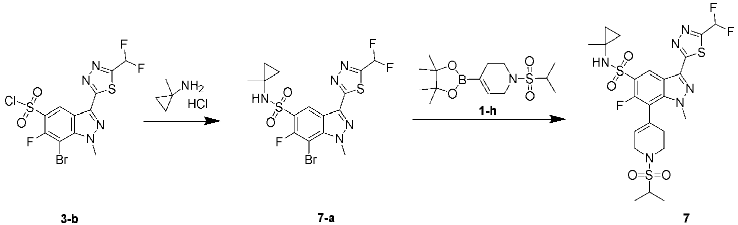

### Synthesis of compound 7-a

Compound 1-methylcyclopropylamine hydrochloride (303 mg, 2.82 mmol) was dissolved in 10 mL of DCM at room temperature, followed by addition of triethylamine (1.96 mL, 14.08 mmol) under an ice-water bath, and stirred under an ice-water bath for 30 min. A solution of compound **3-b** (650 mg, 1.41 mmol) dissolved in DCM (10 mL) was added dropwise to the above mixture under an ice-colded water bath. After addition, the reaction mixture was brought to room temperature and stirred for 2 hours. The reaction mixture was concentrated under reduced pressure, and the residue was dissolved in 100 mL of ethyl acetate, washed with 10% sodium bisulfate solution (100 mL*2), brine (100 mL) sequentially, dried over anhydrous sodium sulfate, filtered, and the filtrate was concentrated under reduced pressure, and the residue was purified by a flash column chromatography (EA/ PE=0~100%) to give compound **7-a** (550 mg, 79%)). LC-MS (ESI): m/z = 496.0 [M+H]⁺.

### Synthesis of compound 7

To a reaction flask were added **7-a** (50 mg, 0.10 mmol), **1-h** (48 mg, 0.15 mmol), [1,1'-bis(di-tert-butylphosphino)ferrocene]palladium dichloride (13 mg, 0.020 mmol), cesium fluoride (46 mg, 0.30 mmol), 1,4-dioxane (9 mL) and H₂O (1 mL). After degassed and purged with nitrogen for 3 times, the reaction mixture was heated and stirred at 100°C for 5 hours. Upon completion, the reaction mixture was cooled to room temperature, concentrated under reduced pressure to remove the organic solvent, the residue was diluted with ethyl acetate, the organic phase was washed with water, brine, dried over anhydrous sodium sulfate, filtered and concentrated under reduced pressure, purified by flash column chromatography (mobile phase: ethyl acetate/petroleum ether: 0% to 100%) to obtain the crude product. The crude product was purified by Prep-HPLC to give compound 7 (30 mg, 49%). LC-MS (ESI): m/z = 605.2 [M+H]⁺.

### Example 8 Synthetic route of compound 8

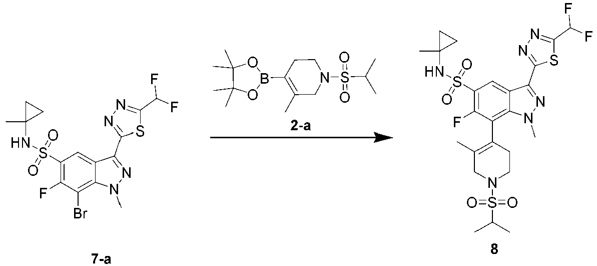

### Synthesis of compound 8

To a reaction flask was added **7-a** (50 mg, 0.10 mmol), **2-a** (50 mg, 0.15 mmol), [1,1'-bis(di-tert-butylphosphino)ferrocene] palladium dichloride (13 mg, 0.020 mmol), cesium fluoride (46 mg, 0.30 mmol), 1,4-dioxane (9 mL) and H₂O (1 mL). After degassed and purged with nitrogen for 3 times, the reaction mixture was heated and stirred at 100°C for 5 hours. Upon completion, the reaction mixture was cooled to room temperature, concentrated under reduced pressure to remove the organic solvent, the residue was diluted with ethyl acetate, the organic phase was washed with water, brine, dried over anhydrous sodium sulfate, filtered and concentrated under reduced pressure, purified by flash column chromatography (mobile phase: ethyl acetate/petroleum ether: 0% to 100%) to obtain the crude product. The crude product was purified by Prep-HPLC to give compound **8** (15 mg, 24%). LC-MS (ESI): m/z = 619.2 [M+H]⁺.

### The synthetic route for compound 1'

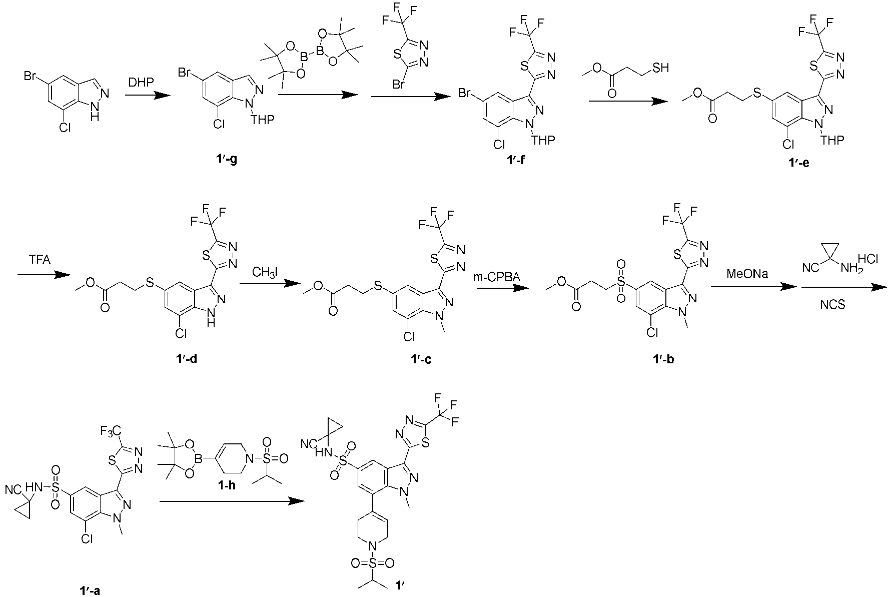

### Synthesis of compound 1'-g

To a solution of compound 5-bromo-7-chloro-1H-indazole (2.0 g, 8.64 mmol) in 3,4-dihydro-2H-pyran (25 mL) was carefully added trifluoroacetic acid (0.066 mL, 0.86 mmol). After addition, the reaction mixture was heated to 90°C and stirred for 3 hours. Upon completion, the reaction mixture was cooled to room temperature, diluted with ethyl acetate, washed with saturated sodium bicarbonate, washed with brine, dried over anhydrous sodium sulfate, filtered, and concentrated to obtain the crude product. The crude product was purified by flash column chromatography (mobile phase: ethyl acetate/petroleum ether 0% to 50%) to give compound **1'-g** (2.5 g, 92%). LC-MS (ESI): m/z 314.9 (M+H)⁺.

### Synthesis of compound 1'-f

To a microwave tube was added methoxy(cyclooctadiene)iridium conJugate dimer (104 mg, 0.16 mmol), 4,4'-di-tert-butyl-2,2'-dipyridine (85 mg, 0.32 mmol), pinacol ester of bis(boronic acid) (603 mg, 2.38 mmol) and methyl tertiary-butyl ether (6 mL), and the resulting mixture was stirred at room temperature for 5 min after degassed and purged with nitrogen twice. A solution of **1'-g** (0.5 g, 1.58 mmol) dissolved in methyl tert-butyl ether (6 mL) was added, sealed after degassed and purged with nitrogen for 3 times, and stirred at 85°C for 3.5 hours. The reaction mixture was evaporated to give borate intermediate.

The borate intermediate was dissolved in toluene (20 mL), followed by addition of 2-bromo-5-(trifluoromethyl)-1,3,4-thiadiazole (556 mg, 2.38 mmol), palladium acetate (36 mg, 0.16 mmol), Xantphos (183 mg, 0.32 mmol), NMM (0.52 mL, 4.75 mmol) and water (10 mL ). After degassed and purged with nitrogen for 3 times, the reaction mixture was heated to 40°C and stirred for 18 hours. Upon completion, the reaction mixture was diluted with ethyl acetate, washed with water, washed with brine, dried over anhydrous sodium sulfate, filtered, evaporated, and purified by silica gel column chromatography (mobile phases: ethyl acetate/petroleum ether 0% to 50%) to give compound **1'-f** (400 mg, 54%). LC-MS (ESI): m/z 466.9 (M+H)⁺.

### Synthesis of compound 1'-e

A reaction flask charged with **1'-f** (350 mg, 0.75 mmol), 1,4-dioxane (20 mL), Xantphos (43 mg, 0.075 mmol), DIPEA (0.39 mL, 2.25 mmol) and methyl 3-mercaptopropionate (0.099 mL, 0.90 mmol) was degassed and purged with nitrogen twice, followed by addition of Pd₂( dba)₃ (34 mg, 0.037 mmol), then was degassed and purged with nitrogen for 3 times. The reaction mixture was heated and stirred at 85°C for 2 hours. Upon completion, the reaction mixture was cooled to room temperature, evaporated to dryness, and purified by silica gel column chromatography (mobile phase: ethyl acetate/petroleum ether 0% to 50%) to give compound **1'-e** (350 mg, 92%). LC-MS (ESI): m/z 507.0 (M+H)⁺.

### Synthesis of compound 1'-d

To a solution of **1'-e** (350 mg, 0.69 mmol) dissolved in dichloromethane (20 mL) was added TFA (5 mL) at room temperature. After addition, the reaction mixture was stirred at room temperature overnight. Upon completion, the solvent was removed by rotary evaporation at room temperature, diluted by addition of ethyl acetate, washed with saturated sodium bicarbonate, washed with brine, dried over anhydrous sodium sulfate, filtered, and evaporated to give compound **1'-d** (250 mg, 86%). LC-MS (ESI): m/z 423.0(M+H)⁺.

### Synthesis of compound 1'-c

To a solution of **1'-d** (250 mg, 0.59 mmol) dissolved in DMF (5 mL) was added cesium carbonate (385 mg, 1.18 mmol) and iodomethane (0.096 mL, 1.18 mmol) at room temperature. After addition, the reaction mixture was stirred at room temperature overnight. Upon completion, the reaction mixture was diluted by adding ethyl acetate, washed with water, washed with brine, dried over anhydrous sodium sulfate, filtered and evaporated to dryness. Purified by flash column chromatography (mobile phase: ethyl acetate/petroleum ether 0% to 50%) to give compound **1'-c** (200 mg, 77%). LC-MS (ESI): m/z 437.4 (M+H)⁺.

### Synthesis of compound 1'-b

To a solution of **1'-c** (200 mg, 0.46 mmol) disssolved in dichloromethane (10 mL) was added m-CPBA (237 mg, 1.37 mmol) under an ice-colded water bath. After addition, the reaction mixture was kept warm at 0°C and stirred for 1 hour. Upon completion, the solvent was removed by rotary evaporation at room temperature, diluted by adding ethyl acetate, washed with saturated sodium bicarbonate, washed with brine, dried over anhydrous sodium sulfate, filtered and evaporated to dryness. The crude product was purified by flash column chromatography (mobile phase: ethyl acetate/petroleum ether, 0% to 100%) to give compound **1'-b** (200 mg, 93%). LC-MS (ESI): m/z 469.0 (M+H)⁺.

### Synthesis of compound 1'-a

The reaction flask was charged with **1'-b** (200 mg, 0.43 mmol), methanol (10 mL) and dichloromethane (10 mL). Sodium methanol (69 mg, 1.28 mmol) was added under an ice-water bath. The reaction mixture was stirred under an ice-colded water bath for 1 hour. To the ice-colded water bath was added 1-amino-1-cyclopropanecarbonitrile hydrochloride (202 mg, 1.70 mmol). The organic solvent was removed and dried by oil pump for 30 minutes at room temperature. 1-Amino-1-cyclopropanecarbonitrile hydrochloride (51 mg, 0.43 mmol) and DMF (10 mL) were added. Triethylamine (0.73 mL, 0.85 mmol) and NCS (171 mg, 1.28 mmol) were added under an ice-colded water bath. The reaction mixture was stirred for 1 hour under an ice-colded water bath. The reaction mixture was added to ice-colded water and extracted twice with ethyl acetate. The organic phase was washed with water, brine, dried over anhydrous sodium sulfate, filtered and evaporated, and purified by column chromatography (PE/EA=3/1, 1/1) to give compound **1'-a** (103 mg, 52%). LC-MS (ESI): m/z 462.9 (M+H)⁺.

### Synthesis of compound 1'

**1'-a** (46 mg, 0.10 mmol), **1-h** (16 mg, 0.05 mmol), dichlorodi-tert-butyl-(4-dimethylaminophenyl)phosphopalladium(II) (18 mg, 0.03 mmol), potassium phosphate (20 mg, 0.09 mmol) were added to a microwave tube, then 1,4-dioxane (5 mL) and water (1 mL) were added. The reaction mixture was under atmosphere of nitrogen, sealed and stirred at 110°C for 11 hours. The reaction mixture was concentrated under reduced pressure and purified by prep-HPLC to give compound **1'** (15.9 mg, 76%). LC-MS (ESI): m/z 616.4 (M+H)⁺; ¹H NMR (400MHz, DMSO-*d*₆): *δ* 8.86 (1H, d, *J*=2.0Hz), 7.70 (1H, d, *J*=2.0Hz), 6.00 (1H, s), 4.24 (3H, s), 4.07 (2H, d, *J*=2.4Hz), 3.63 (2H, t, *J*=5.2Hz), 3.42-3.54 (1H, m), 2.57 (2H, s), 1.40-1.47 (2H, m), 1.30-1.35 (2H, m), 1.30 (6H, d, *J*=6.8Hz).

### The synthetic route for compound 2'

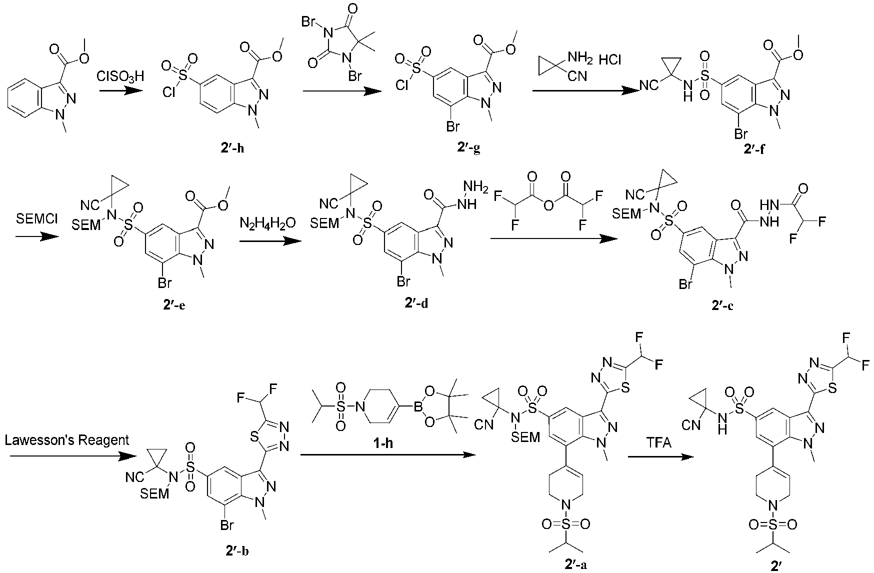

### Synthesis of compound 2'-h

Chlorosulfonic acid (30 mL) was added to a reaction flask and 1-methyl-3-methoxycarbonylindazole (10 g, 52.58 mmol) was added in batches over 10 min under an ice bath. The reaction mixture was carried out for 5 min at room temperature. The reaction mixture was stirred at 65°C for 18 h (with tail gas drying and absorption unit). The reaction mixture was cooled to room temperature and then added dropwise into 300 g of ice, extracted with ethyl acetate, washed with water, washed with brine, dried over anhydrous sodium sulfate, filtered, and evaporated to give compound **2'-h** (13.5 g, 89%).

### Synthesis of compound 2'-g

The reaction flask was charged with **2'-h** (13.5 g, 46.76 mmol) and dichloromethane (135 mL). Dibromohydantoin (13.37 mg, 46.76 mmol) was added under ice bath. Trifluoromethanesulfonic acid (5 mL) was added dropwise into the suspension. The reaction mixture was stirred under an ice bath for 10 minutes and then stirred at room temperature for 1.5 hours. Trifluoromethanesulfonic acid (5 mL) was added dropwise into the suspension. Dichloromethane (60mL) was added. The reaction mixture was stirred at room temperature for 1.5 hours. Ice-colded water was added into the reaction mixture, extracted twice with ethyl acetate, washed twice with water, washed with brine, dried over anhydrous sodium sulfate, filtered, evaporated to dryness and purified over a column (mobile phase: petroleum ether/ethyl acetate 100:0~60:40) to give the product compound **2'-g** (10.1 g, 59%).

### Synthesis of compound 2'-f

A reaction flask was charged with 1-amino-1-cyclopropanecarbonitrile hydrochloride (7.05 g, 59.46 mmol), pyridine (35 mL) and a small amount of 3A molecular sieve, and then stirred under ice bath for 5 minutes. A dry solution of **2'-g** (10.1 g, 27.48 mmol) dissolved in dichloromethane (70 mL) containing molecular sieves was added dropwise within 5 min under ice bath. The reaction mixture was carried out for 10 min under the ice bath and then for 2 h at room temperature. The orgainc solvent was removed by rotary evaporaiton. Ice-colded water and sodium bisulfate solid were added to adjust pH=6~7, petroleum ether was added, filtered, and the filter cake was washed with sodium bisulfate aqueous soluiton, water, and petroleum ether. The aqueous phase was extracted with ethyl acetate, the extract was washed with water, evaporated to dryness, slurried with ethyl acetate petroleum ether, the solids were combined and dried under vacuum to give compound **2'-f** (11.3 g, 100%). LC-MS (ESI): m/z 412.9 (M+H) +.

### Synthesis of compound 2'-e

The reaction flask was charged with **2'-f** (11.3 g, 27.34 mmol) and dichloromethane (150 mL). Triethylamine (12.5 mL, 89.93 mmol) was added and stirred for 10 min under ice bath. SEMCl (8.0 mL, 45.20 mmol) was added dropwise under ice bath and stirred for 10 min. The reaction mixture was stirred for 1.5 hours under a room temperature water bath. The solvent was removed by rotary evaporation at room temperature, water and ethyl acetate were added and throughly stirred, filtered, the filtrate was partitioned, the aqueous phase was extracted once with ethyl acetate, the organic phases were combined, washed with water, brine and then evaporated to dryness to give compound **2'-e** (14.8 g, 100%). LC-MS(ESI): m/z 560.1(M+NH₄) ⁺.

### Synthesis of compound 2'-d

The reaction flask was charged with **2'-e** (14.8 g, 27.23 mmol), anhydrous ethanol (150 mL) and hydrazine hydrate (10 mL, 206.15 mmol). The reaction mixture was heated and stirred at 52°C for 7 hours. The reaction mixture was cooled to room temperature. The solvent was removed by rotary evaporation at room temperature, ice-colded water was added, extracted twice with ethyl acetate, washed with water, washed with brine, dried over anhydrous sodium sulfate, filtered and then evaporated to dryness to give compound **2'-d** (14.5 g, 98%). LC-MS (ESI): m/z 1084.8 (2M+H)⁺.

### Synthesis of compound 2'-c

The reaction flask was charged with **2'-d** (14.5 g, 26.68 mmol) and dichloromethane (150 mL). Triethylamine (5.19 mL, 37.35 mmol) was added dropwise under an ice bath. The reaction mixture was stirred under ice bath for 5 minutes. Difluoroacetic anhydride (3.25 mL, 28.01 mmol) was added dropwise into the reaction mixture under ice bath. The reaction mixture was stirred under ice bath for 1 hour. Methanol (2.5mL) was added and the the reaction mixture was stirred for 20 minutes under the temparature range from ice bath to room temperature. The solvent was removed by rotary evaporation at room temperature. Water and ethyl acetate were added to the reaction flask, partitioned, aqueous phase extraction was performed once, the organic phases were combined, washed with water, washed with brine, dried over anhydrous sodium sulfate, filtered, and evaporated to dryness, purified by a column (mobile phases: petroleum ether/ethyl acetate, 100/0 to 60/40) to give compound **2'-c** (13.2 g, 80%). LC-MS (ESI): m/z 638.0 (M+NH₄)⁺.

### Synthesis of compound 2'-b

The reaction flask was charged with **2'-c** (11.3 g, 18.18 mmol), Lawson's reagent (17.75 g, 43.89 mmol) and THF (200 mL). The reaction mixture was directly heated and stirred under argon protection at 80°C for 10 hours. The reaction mixture was cooled to room temperature and the solvent was removed by rotary evaporation. The crude product was diluted with ethyl acetate/petroleum ether mixture, washed three times with aqueous sodium bicarbonate, washed with brine, dried over anhydrous sodium sulfate, filtered and evaporated to obtain the crude product, ammonia-methanol/methanol/ dichloromethane (1:1:20) solution (200mL) was added and stirred for 2 hours at room temperature. The reaction mixture was evaporated to dryness and purified by a column (mobile phase: petroleum ether/ethyl acetate, 100/0 to 70/30) to give compound **2'-b** (5.8 g, 51%). LC-MS (ESI): m/z 619.0 (M+H) ⁺.

### Synthesis of compound 2'-a

Compound **2'-b** (50 mg, 0.081 mmol), **1-h** (51 mg, 0.16 mmol), [1,1'-bis(diphenylphosphino)ferrocene]palladium dichloride (12 mg, 0.016 mmol), potassium carbonate (33 mg, 0.24 mmol) were added to 1,4-dioxane (10 mL) and water (1 mL), and the reaction mixture was replaced with nitrogen three times and then heated and stirred at 100°C for 6 h. The reaction mixture was filtered through celite. Water (100 mL) was added into the filtrate, extracted with ethyl acetate (100 mL), the organic phase was washed with brine (100 mL), dried over sodium sulfate, filtered off the desiccant, and the filtrate was concentrated under reduced pressure to obtain the crude product. The crude product was purified by column chromatography (mobile phase, PE/EA 1/1) to give compound **2'-a** (50 mg, 85%). LC-MS (ESI): m/z 728.2 (M+H) ⁺.

### Synthesis of compound 2'

Compound **2'-a** (50 mg, 0.069 mmol) was dissolved dichloromethane (4.5 mL), to which trifluoroacetic acid (1.5 mL) was added under an ice-water bath, and the reaction mixture was stirred for 2 h at room temperature. The dichloromethane was removed by concentration under reduced pressure and the residue was adjusted to pH 7~8 with saturated sodium bicarbonate solution and extracted with ethyl acetate (50 mL). The organic phase was washed with brine (50 mL), dried over sodium sulfate, filtered off the desiccant, and the filtrate was concentrated under reduced pressure to obtain the crude product. The crude product was purified by prep-HPLC (alkaline condition) to give Compound 2' (33 mg, 80%). LC-MS (ESI): m/z 598.4 (M+H) ⁺.

### The route of synthesis for compound 3'

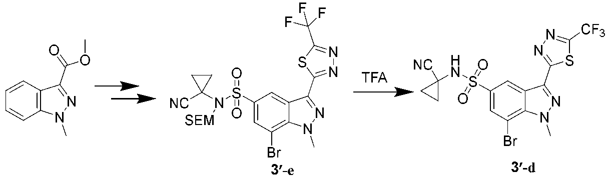

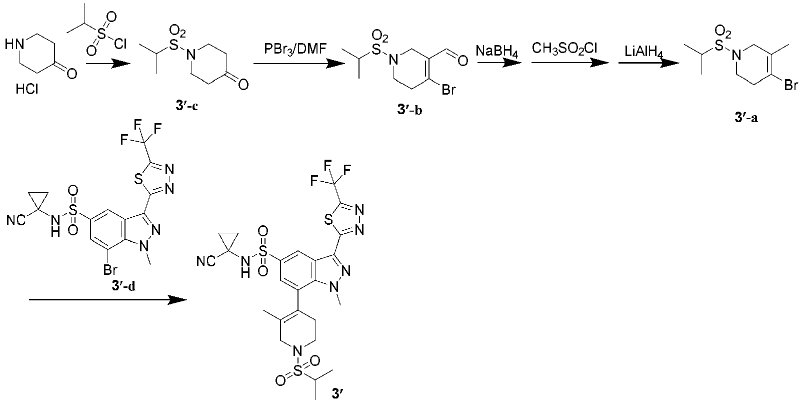

### Synthesis of compound 3'-e

With reference to the synthetic route of compound **2'-b,** compound **3'-e** was synthesized by using **2'-d** as the starting material and trifluoroacetic anhydride instead of difluoroacetic anhydride.

### Synthesis of compound 3'-d

The reaction flask was charged with **3'-e** (88 mg, 0.14 mmol), dichloromethane (5 mL). Trifluoroacetic acid (1 mL) and water (0.1 mL) were added dropwise into the reaction flask at room temperature. After addition, the reaction mixture was carried out at room temperature for 3 hours. The reaction mixture was concentrated at room temperature to remove the solvent, diluted with dichloromethane, washed with saturated sodium bicarbonate, washed with brine, dried over anhydrous sodium sulfate, filtered, and evaporated to dryness to obtain the crude product. The crude product was purified by flash column chromatography (mobile phase: petroleum ether/ethyl acetate, 0% to 100%) to give compound **3'-d** (56 mg, 80%). LC-MS (ESI): m/z 506.9 [M+H]⁺.

### Synthesis of compound 3'-c

To a reaction flask was added 4-oxopiperidone hydrochloride (2.72 g, 20.06 mmol), acetone (30 mL) and water (15 mL). Anhydrous potassium carbonate (7.21 g, 52.16 mmol) was added by dropwise under an ice bath, followed by addition of 2-propylsulfonyl chloride (3.38 mL, 30.09 mmol) by dropwise. The reaction mixture was stirred for 18 hours under the temperature range form ice bath to room temperature. The reaction mixture was quenched by the addition of saturated sodium bicarbonate solution, extracted three times with dichloromethane, the organic phases were combined, washed once with brine, dried by adding anhydrous sodium sulfate and anhydrous magnesium sulfate, filtered, evaporated to dryness, and the crude product was washed twice with petroleum ether to give compound **3'-c** (2.7 g, 66%).

### Synthesis of compound 3'-b

N,N-dimethylformamide (3.18 mL, 39.46 mmol) and dichloromethane (60 mL) were added to a reaction flask. A solution of phosphorus tribromide (2.97 mL, 31.57 mmol) dissolved in dichloromethane (10 mL) was added slowly and dropwise under an ice bath. After addition, the reaction mixture was stirred under an ice bath for 1 hour. A solution of **3'-c** (2.7 g, 13.15 mmol) dissolved in dichloromethane (20 mL) was added dropwise under an ice bath. After addition, the reaction mixture was stirred at room temperature overnight. The reaction mixture was cooled and stirred under an ice bath for 5 min, and saturated sodium bicarbonate solution was added slowly and dropwise until no bubbles were formed. The solution was partitioned, the aqueous phase was extracted twice with dichloromethane, the organic phases were combined, dried over anhydrous sodium sulfate, filtered, evaporated to dryness, and purified by a column (mobile phase: petroleum ether/ethyl acetate, 100/0 to 70/30) to give compound **3'-b** (1.6 g, 41%). LC-MS(ESI): m/z 313.1(M+NH₄)⁺.

### Synthesis of compound 3'-a

**3'-b** (330 mg, 1.11 mmol) and anhydrous methanol (10 mL) were added into a reaction flask and then stirred for 5 min under an ice bath, followed by addition of sodium borohydride (22 mg, 0.58 mmol). The reaction mixture was stirred under an ice bath for 1 hour. Saturated aqueous ammonium chloride solution was added dropwise to the reaction mixture, methanol was removed by rotary evaporation and then extracted twice with ethyl acetate. The organic phase was washed with brine, dried over anhydrous sodium sulfate, filtered and evaporated to dryness to obtain the crude product. Anhydrous dichloromethane (10mL), triethylamine (0.55mL, 3.96mmol) and methanesulfonyl chloride (0.2mL, 2.58mmol) were added dropwise into the crude product under ice bath. After addition, the reaction mixture was stirred under ice bath for 1 hour. The solvent was removed by rotary evaporation, dried with an oil pump, and anhydrous tetrahydrofuran (10 mL) was added into the residue. Aluminum lithium hydroxide tetrahydrofuran solution (1 M, 2.5 mL, 2.50 mmol) was added slowly and dropwise within 20 min under an ice-salt bath. After addition, the reaction mixture was stirred for 1 hour under an ice-salt bath. The reaction mixture was quenched by slow addition of sodium sulfate decahydrate solid into the reaction mixture. The reaction mixture continued to be stirred for 15 minutes and then diluted by adding ethyl acetate and stirred for 15 minutes at room temperature. Filtered, evaporated to dryness and then purified by a column (mobile phase: petroleum ether/ethyl acetate, 100/0 to 80/20) to give compound **3'-a** (95 mg, 30%). LC-MS (ESI): m/z 281.9 (M+H)⁺.

### Synthesis of compound 3'

A microwave tube was charged with **3'-a** (95 mg, 0.34 mmol), bis-pinacol borate (128 mg, 0.50 mmol), Pd(dppf)Cl₂·CH₂Cl₂ (15 mg, 0.018 mmol) and potassium acetate (100 mg, 1.02 mmol). The microwave tube was degassed and purged with nitrogen, and then 1,4-dioxane (4 mL) was added. The microwave tube was degassed and purged with nitrogen for 3 times and then sealed. The reaction mixture was heated and stirred at 80°C for 20 hours. The reaction mixture was cooled to room temperature and **3'-d** (100 mg, 0.20 mmol), Pd(dtbpf)Cl₂ (10 mg, 0.015 mmol), cesium fluoride (200 mg, 1.32 mmol) and water (1 mL) were added. Nitrogen was replaced 3 times and sealed. The reaction mixture was heated and stirred at 100°C for 2 hours. Supplemented with 3'-d (100 mg, 0.20 mmol), Pd(dtbpf)Cl₂ (20 mg, 0.031 mmol) and cesium fluoride (120 mg, 0.79 mmol) and the microwave tube was degassed and purged with nitrogen for 3 times and then sealed. The reaction mixture was heated and stirred at 100°C for 5 h. The reaction mixture was cooled to room temperature and then diluted with ethyl acetate, washed with water, and the aqueous phase was extracted with ethyl acetate once. The organic phases were combined, washed with brine, dried over anhydrous sodium sulfate, filtered, evaporated to dryness, and purified by a column (mobile phase: dichloromethane/methanol, 100/0 to 97/3) to obtain the crude product. The crude product was purified by prep-HPLC (ammonium bicarbonate) to give compound **3'** (32 mg, 15%). LC-MS(ESI): m/z 630.1(M+H)⁺; ¹H NMR (DMSO-*d*₆, 400 MHz): *δ* 9.26 (1H, bs), 8.87 (1H, d, *J*=2.0 Hz), 7.64 (1H, d, *J*=1.6 Hz), 4.19 (3H, s), 4.01 - 3.88 (2H, m), 3.68 - 3.51 (2H, m), 3.49 (1H, q, *J*=6.8 Hz), 1.47 (3H, s), 2.50 - 2.42 (2H, m), 1.45 - 1.39 (2H, m), 1.38 - 1.32 (2H, m), 1.30 (6H, d, *J*=6.7 Hz).

### Example 9 Synthetic route of compound 9

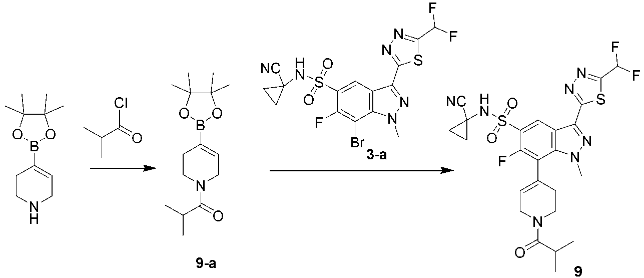

### Synthesis of compound 9-a

Compound 1,2,3,6-tetrahydropyridine-4-boronic acid pinacol ester (100 mg, 0.48 mmol) was dissolved in dichloromethane (5 mL), to which diisopropylethylamine (185 mg, 1.43 mmol) was added, the system was cooled down to 0°C, then isobutyryl chloride (61 mg, 0.57 mmol) was added dropwise into the system. After addition, the reaction mixture was stirred for 2 hours at room temperature. Saturated sodium bicarbonate solution (10 mL) was added into the reaction mixture, extracted twice with dichloromethane (50 mL), dried over sodium sulfate, filtered the desiccant, and rotary evaporated under reduced pressure to obtain the crude product. The crude product was purified by column chromatography (mobile phase, PE/EA = 1/1 to 1/5) to give compound **9-a** (75 mg, 56%). LC-MS (ESI): m/z 280.2 (M +H)⁺.

### Synthesis of compound 9

Compound **3-a** (50 mg, 0.099 mmol) was dissolved in 18 mL of 1,4-dioxane at room temperature and compound **9-a** (41 mg, 0.15 mmol), 1,1'-di-tert-butylphosphinoferrocene palladium dichloride (13 mg, 0.020 mmol), cesium fluoride (45 mg, 0.30 mmol), water (2 mL) were added. The reaction mixture was stirred at 100°C under nitrogen protection for 16 hours. The reaction mixture was cooled to room temperature, concentrated under reduced pressure, and the residue was purified by flash column chromatography (EA/PE= 0~100%) to obtain the crude product. The crude product was then separated and purified by Prep-HPLC (alkaline method, NH₄HCO₃) to give compound **9** (23.5 mg, 41%). LC-MS (ESI): m/z = 580.2 [M+H]⁺.

### Example 10 Synthetic route of compound 10

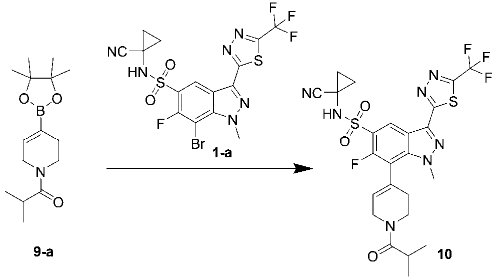

### Synthesis of compound 10

To a reaction flask was added **1-a** (50 mg, 0.095 mmol), **9-a** (40 mg, 0.14 mmol), [1,1'-bis(di-tert-butylphosphino)ferrocene]palladium dichloride (12 mg, 0.019 mmol), cesium fluoride (43 mg, 0.28 mmol), 1,4-dioxane (9 mL) and H₂O (1 mL). After degassed and purged with nitrogen for 3 times, the reaction mixture was heated and stirred at 100°C for 5 hours. Upon completion, the reaction mixture was cooled to room temperature, concentrated under reduced pressure to remove the organic solvent, and the residue was diluted with ethyl acetate. The organic phase was washed with water, brine, dried over anhydrous sodium sulfate, filtered and concentrated under reduced pressure to obtain the crude product. The crude product was purified by Prep-HPLC to give compound **10** (25 mg, 44%). LC-MS (ESI): m/z 598.1 [M+H]⁺.

### Example 11 Synthetic route of compound 11

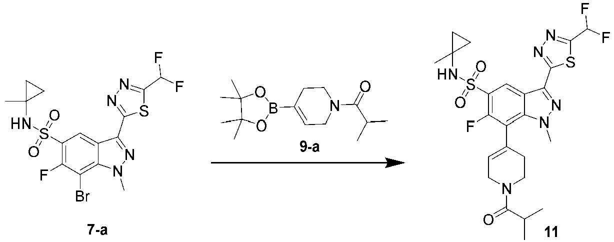

### Synthesis of compound 11

To a reaction flask was added **7-a** (50 mg, 0.10 mmol), **9-a** (42 mg, 0.15 mmol), [1,1'-bis(di-tert-butylphosphino)ferrocene]palladium dichloride (13 mg, 0.020 mmol), cesium fluoride (46 mg, 0.30 mmol), 1,4-dioxane (9 mL) and H₂O (1 mL). After degassed and purged with nitrogen for 3 times, the reaction mixture was heated and stirred at 100°C for 5 hours. Upon completion, the reaction mixture was cooled to room temperature, concentrated under reduced pressure to remove the organic solvent, diluted by adding ethyl acetate to the residue, the organic phase was washed with water, brine, dried over anhydrous sodium sulfate, filtered and concentrated under reduced pressure to obtain the crude product. The crude product was purified by Prep-HPLC to give compound **11** (25 mg, 44%). LC-MS (ESI): m/z 569.1 [M+H]⁺.

### The synthetic route for compound 4'

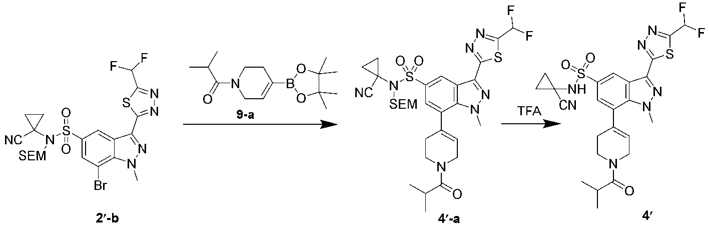

### Synthesis of compound 4'-a

Compounds **2'-b** (55 mg, 0.09 mmol), **9-a** (37 mg, 0.13 mmol), [1,1'-bis(diphenylphosphino)ferrocene]palladium dichloride (13 mg, 0.02 mmol), and potassium carbonate (37 mg, 0.27 mmol) were added into 1,4-dioxane (5 mL) and water (0.5 mL), and the reaction mixture was degassed and purged with nitrogens for 10 times and then sealed. The reaction mixture was heated and stirred at 100°C for 6 h. The reaction mixture was cooled to room temperature and evaporated under reduced pressure to obtain the crude product. The crude product was purified by column chromatography (mobile phase, PE/EA= 3/1 to 1/1) to give compound **4'-a** (60 mg, 98%). LC-MS (ESI): m/z 692.1(M+H)⁺.

### Synthesis of compound 4'

Compound **4'-a** (70 mg, 0.10 mmol) was dissolved in dichloromethane (4.5 mL), to which trifluoroacetic acid (1.5 mL) was added at 0°C. The reaction mixture was stirred at room temperature for 2 hours. The reaction mixture was evaporated to remove the dichloromethane and the pH was adjusted to 7~8 with saturated sodium bicarbonate solution, extracted with ethyl acetate (100 mL), washed with brine (100 mL), and rotary evaporated under reduced pressure to obtain the crude product. The crude product was purified by HPLC (alkaline condition) to give compound **4'** (30 mg, 53%). LC-MS (ESI): m/z 562.2 (M+H) ⁺.

### Example 12 Synthetic route of compound 12

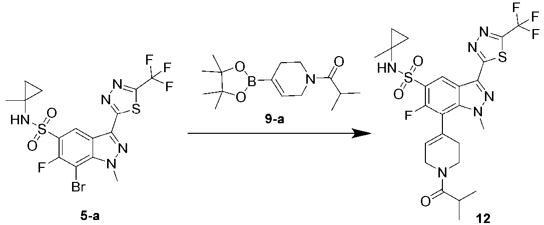

### Synthesis of compound 12

To a reaction flask was added **5-a** (50 mg, 0.097 mmol), **9-a** (41 mg, 0.15 mmol), [1,1'-bis(di-tert-butylphosphino)ferrocene]palladium dichloride (13 mg, 0.019 mmol), cesium fluoride (44 mg, 0.29 mmol), 1,4-dioxane (9 mL) and H₂O (1 mL). After degassed and purged with nitrogen for 3 times, the reaction mixture was heated and stirred at 100°C for 5 hours. Upon completion, the reaction mixture was cooled to room temperature, concentrated under reduced pressure to remove the organic solvent, diluted with ethyl acetate to the residue, the organic phase was washed with water, brine, dried over anhydrous sodium sulfate, filtered, concentrated under reduced pressure to obtain the crude product. The crude product was purified by Prep-HPLC to give compound **12** (5 mg, 9%). LC-MS (ESI): m/z 587.2 [M+H]⁺.

### Example 13 Synthetic route of compound 13

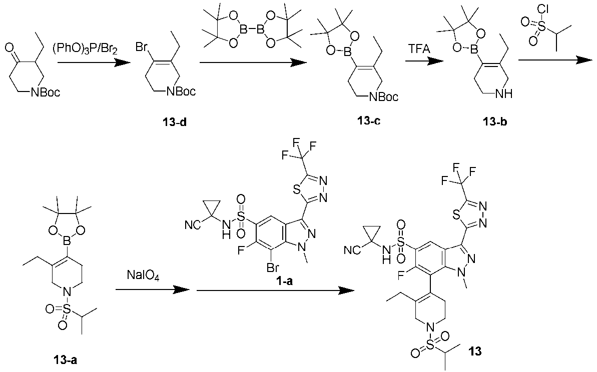

### Synthesis of compound 13-d

Triphenyl phosphite (2.3 g, 7.41 mmol) and anhydrous dichloromethane (20 mL) were added into a reaction flask. Liquid bromine (1.25 g, 7.82 mmol) was added slowly and dropwise into the flask under a dry ice ethyl acetate bath. The reaction mixture was kept at this temperature with stirring for 5 min and then triethylamine (1.15 mL, 8.27 mmol) and tert-butyl 3-ethyl-4-oxopiperidine-1-carboxylate (1.48 g, 6.51 mmol) were added dropwise. After addition, the reaction mixture was stirred at this temperature for 1 hour, stirred at room temperature for 18 hours and then heated to reflux for 2 hours. The reaction solvent was removed by rotary evaporation, ethyl acetate and petroleum ether (1:2) were added to the residue, washed twice with water, washed with brine and dried over anhydrous sodium sulfate. The organic phase was filtered, evaporated to dryness and the crude was purified by a column (mobile phase: petroleum ether/ethyl acetate, 100/0 to 88/12) to give compound **13-d** (800 mg, 42%).

### Synthesis of compound 13-c

To a reaction vial was added **13-d** (800 mg, 2.76 mmol), pinacol ester of bisboronic acid (1400 mg, 5.51 mmol), Pd(dppf)Cl₂ (105 mg, 0.14 mmol), potassium acetate (810 mg, 8.25 mmol), and anhydrous 1,4-dioxane (12 mL). The reaction mixture was degassed and purged with nitrogen for 3 times, heated and stirred at 90°C for 6 hours. The reaction mixture was cooled to room temperature, the solvent was removed by rotary evaporation and diluted with ethyl acetate. The diluted solution was washed with water, brine, dried over anhydrous sodium sulfate, filtered, evaporated to dryness, and the crude was purified by a column (mobile phase: petroleum ether/ethyl acetate, 100/0 to 88/12) to give compound **13-c** (500 mg, 54% yield). LC-MS (ESI): m/z 338.2 (M+H)⁺.

### Synthesis of compound 13-b

**13-c** (500 mg, 1.48 mmol) was added to a reaction flask, followed by addition of dichloromethane (9 mL) and trifluoroacetic acid (3 mL). The mixture was stirred at room temperature for 3 hours under nitrogen prptection. The reaction mixture was concentrated under reduced pressure to give compound **13-b** (360 mg). LC-MS (ESI): m/z238.2 (M+H)⁺.

### Synthesis of compound 13-a

**13-b** (360 mg, 1.52 mmol) was added to a reaction flask, followed by addition of dichloromethane (50 mL), triethylamine (154 mg, 1.52 mmol) and isopropylsulfonyl chloride (360 mg, 2.53 mmol). The mixture was stirred at room temperature for 2 hours under nitrogen prptection. The reaction mixture was washed with saturated sodium bisulfate (100 mL) and concentrated to give compound **13-a** (226 mg, 43%). LC-MS (ESI): m/z 344.2 (M+H) ⁺.

### Synthesis of compound 13

To a reaction vial was added **13-a** (55 mg, 0.16 mmol), acetone (4 mL), water (2.0 mL), ammonium acetate (100 mg, 1.30 mmol) and sodium periodate (100 mg, 0.47 mmol). The reaction mixture was stirred at room temperature for 24 hours and then heated and stirred at 60°C for 18 hours. The reaction mixture was cooled to room temperature, ethyl acetate and a small amount of sodium bisulfate solid were added, the liquid was partitioned and the aqueous phase was extracted once with ethyl acetate. The organic phases were combined, dried over anhydrous sodium sulfate, filtered, and evaporated to give crude product A. To a microwave tube were added compound **1-a** (70 mg, 0.13 mmol), Pd(dtbpf)Cl₂ (10 mg, 0.015 mmol), a solution of crude A dissolved in 1,4-dioxane (2.5 mL) and an aqueous solution (0.5 mL) of cesium fluoride (82 mg, 0.54 mmol). mL) solution. The microwave tube was sealed, degassed and purged with nitrogen for 3 times. The reaction mixture was heated and stirred at 70°C for 18 hours. The reaction mixture was cooled to room temperature, diluted with ethyl acetate, the diluted solution was washed with water and the aqueous phase was extracted once with ethyl acetate. The organic phases were combined, evaporated to dryness and purified by a column (mobile phase: petroleum ether/ethyl acetate, 100/0 to 40/60) to give the crude product. The crude product was purified by prep-HPLC (ammonium bicarbonate) to give compound **13** (6.3 mg, 6.0% yield). LC-MS(ESI): m/z 662.3 (M+H)⁺. ¹H NMR (DMSO-*d*₆, 400 MHz) *δ* 9.57 (1H, bs), 8.88 (1H, d, *J*=6.4 Hz), 4.18 (3H, s), 4.09 - 3.95 (2H, m), 3.67 - 3.44 (3H, m), 2.48 - 2.40 (2H, m), 1.94 - 1.78 (2H, m), 1.52 - 1.27 (10H, m), 0.85 (3H, t, *J*=7.6 Hz).

### The synthetic route for compound 5'

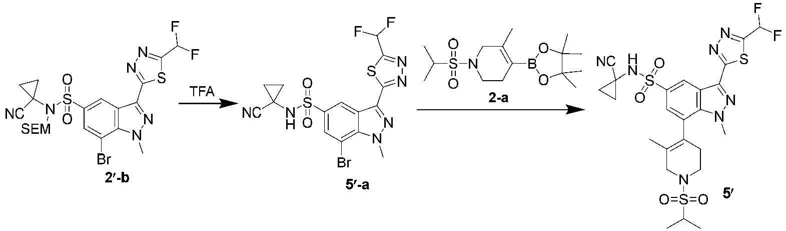

### Synthesis of compound 5'-a

A reaction flask was charged with **2'-b** (80 mg, 0.13 mmol) and dichloromethane (5 mL). Trifluoroacetic acid (1 mL) was added dropwise at room temperature. After addition, the reaction mixture was allowed to react for 4 hours at room temperature. The reaction mixture was concentrated at room temperature to remove the solvent, the residue was diluted with dichloromethane, washed with saturated sodium bicarbonate, washed with brine, dried over anhydrous sodium sulfate, filtered and evaporated to dryness to obtain the crude product. The crude product was purified by flash column chromatography (mobile phase: ethyl acetate/(petroleum ether/dichloromethane 4:1), 0% to 100%) to give compound **5'-a** (50 mg, 79%). LC-MS (ESI): m/z 488.9 [M+H]⁺.

### Synthesis of compound 5'

Compound **5'-a** (200 mg, 0.41 mmol) was dissolved in 36 mL of 1,4-dioxane at room temperature, followed by addition of compound **2-a** (202 mg, 0.61 mmol), 1,1'-di-tert-butylphosphinoferrocene palladium dichloride (53 mg, 0.082 mmol), cesium fluoride (186 mg, 1.23 mmol) and water ( 4mL). The reaction mixture was stirred at 100°C under nitrogen protection for 8 hours. The reaction mixture was cooled to room temperature, concentrated under reduced pressure, 50 mL of water was added to the residue and extracted with DCM (50 mL*2). The organic phase was dried over anhydrous sodium sulfate, filtered, and the filtrate was concentrated under reduced pressure to obtain the crude product. The crude product was purified by Prep-HPLC (basic method, NH₄HCO₃) to give compound **5'** (82 mg, 33%). LC-MS (ESI): m/z = 612.1 [M+H]⁺.

### The synthetic route for compound 6'

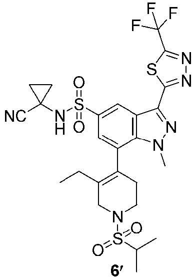

With reference to the synthetic route of compound **13,** compound **6'** was synthesized by using **3'-d** instead of **1-a.** LC-MS(ESI): m/z 644.4 (M+H)⁺. ¹H NMR (DMSO-*d*₆, 400 MHz) *δ* 9.30 (1H, bs), 8.88 (1H, d, *J*=1.6 Hz), 7.63 (1H, d, *J*=1.6 Hz), 4.22 (3H, s), 4.11 - 3.93 (2H, m), 3.72 - 3.60 (1H, m), 3.59 - 3.45 (2H, m), 2.50 - 2.44 (2H, m), 1.95 - 1.76 (2H, m), 1.50 - 1.26 (10H, m), 0.85 (3H, t, *J*=7.6 Hz).

### Synthetic method for similar compounds:

| Compound | Structure | Synthetic method |
|---|---|---|
| **14** | 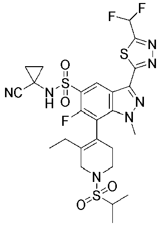 | Referring to the synthesis route of compound **13,** using **3-a** instead of **1-a.** |
| **15** | 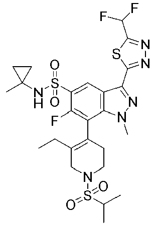 | Referring to the synthesis route of compound **13,using 7-a** instead of **1-a.** |
| **16** | 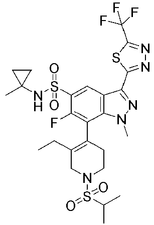 | Referring to the synthesis route of compound **13,** using **5-a** instead of **1-a.** |
| **17** | 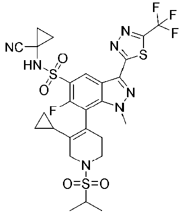 | Referring to the synthesis route of compound **13,** using 3-Cyclopropyl-4-oxopiperidine-1-carboxylic acid tert-butyl ester instead of 3-Ethyl-4-oxopiperidine-1-carboxylic acid tert-butyl ester. |
| **18** | 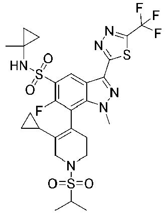 | Referring to the synthesis route of compound **13,** using 3-Cyclopropyl-4-oxopiperidine-1-carboxylic acid tert-butyl ester instead of 3-Ethyl-4-oxopiperidine-1-carboxylic acid tert-butyl ester, using **5-a** instead of **1-a.** |
| **19** | 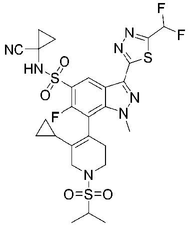 | Referring to the synthesis route of compound **13,** using 3-Cyclopropyl-4-oxopiperidine-1-carboxylic acid tert-butyl ester instead of 3-Ethyl-4-oxopiperidine-1-carboxylic acid tert-butyl ester, using **3-a** instead of **1-a.** |
| **20** | 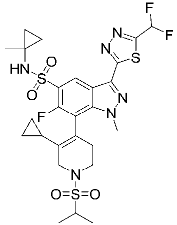 | Referring to the synthesis route of compound **13,** using tert-butyl 3-cyclopropyl-4-oxopiperidine-1-carboxylate instead of tert-butyl 3-ethyl-4-oxopiperidine-1-carboxylate, using **7-a** instead of **1-a.** |

### The synthetic route for compound 7'

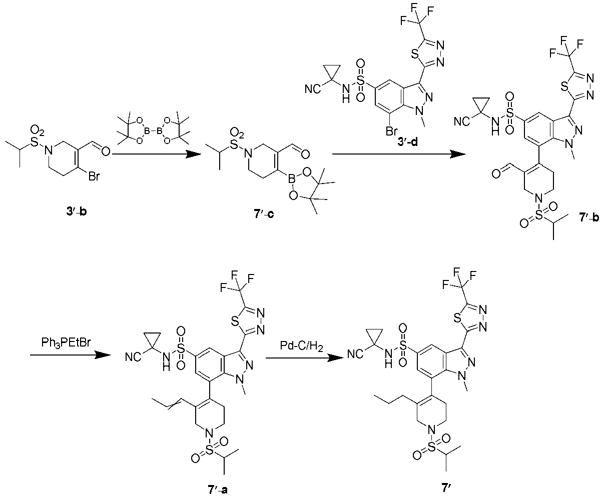

### Synthesis of compound 7'-c

**3'-b** (400 mg, 1.35 mmol), bis-pinacol borate (403 mg, 1.59 mmol), Pd(dppf)Cl₂·CH₂Cl₂ (80 mg, 0.098 mmol) and potassium acetate (396 mg, 4.04 mmol) were added into a microwave tube, degassed and purged with nitrogen and 1,4-dioxane (10 mL) was added. The microwave tube was degassed and purged with nitrogen for 3 times and sealed. The reaction mixture was heated and stirred at 80°C for 6 hours. The reaction mixture was cooled to room temperature, and the reaction mixture (2mL) was removed and proceeded directly to the next step of the reaction. The remaining reaction mixture was diluted with ethyl acetate, washed once with brine, the aqueous phase was extracted with ethyl acetate for 3 times, the organic phases were combined, evaporated and dried by oil pump to obtain the crude product of **7'-c** (463 mg, 100%). LC-MS (ESI): m/z 344.2 (M+H)⁺.

### Synthesis of compound 7'-b

A solution of **7'-c** (345 mg, 1.01 mmol), Pd(dtbpf)Cl₂ (41 mg, 0.063 mmol), **3'-d** (335 mg, 0.66 mmol) and cesium fluoride (340 mg, 2.24 mmol) dissolved in water (2 mL) was added to a reaction flask. The reaction mixture was degassed and purged with nitrogen for 3 times, heated and stirred at 100°C for 2 hours. The reaction mixture was cooled to room temperature and diluted with ethyl acetate. The diluent was washed with brine, evaporated to dryness, and the crude was purified by column purification (mobile phase: dichloromethane/ethyl acetate, 100/0 to 70/30) to give compound **7'-b** (290 mg, 68%). LC-MS (ESI): m/z 644.1 (M+H)⁺.

### Synthesis of compound 7'-a

**7'-b** (125 mg, 0.19 mmol), ethyltriphenylphosphonium bromide (87 mg, 0.23 mmol), anhydrous potassium carbonate (81 mg, 0.59 mmol) and 1,4-dioxane (6 mL) were added in a microwave tube. The reaction mixture was sealed, degassed and purged with nitrogen for 3 times, heated and stirred at 80°C for 7 hours. Monitoring result showed that the reaction was incomplete, thus, the reaction mixture was continued to heat and stirr at 80°C for 3 hours. The reaction mixture was cooled to room temperature, water was added and extracted twice with ethyl acetate. The organic phases were combined, evaporated to dryness, and purified by a column (mobile phase: petroleum ether/ethyl acetate, 100/0 to 40/60) to give compound **7'-a** (60 mg, 47% yield). LC-MS (ESI): m/z 656.3 (M+H)⁺.

### Synthesis of compound 7'

A reaction flask was charged with **7'-a** (60 mg, 0.092 mmol), ethyl acetate (5 mL) and palladium carbon (10% Pd, containing 40-60% water) (60 mg, 0.56 mmol). The reaction mixture was degassed and purged with hydrogen and stirred at room temperature for 7 hours. The reaction mixture was degassed and purged with nitrogen and supplemented with palladium carbon (10% Pd, containing 40-60% water) (30 mg, 0.28 mmol), degassed and purged with hydrogen and stirred at room temperature for 3 hours. The reaction mixture was filtered through celite, the filter cake was washed with dichloromethane/methanol (10:1), the organic phases were combined, rotary evaporated to dryness and then purified by prep-HPLC (ammonium bicarbonate) to give compound **7'** (21.3 mg, 35% yield). LC-MS(ESI): m/z 658.3 (M+H)⁺. ¹H NMR (DMSO-*d*₆, 400 MHz) *δ* 9.31 (1H, s), 8.88 (1H, d, *J*=2.0 Hz), 7.61 (1H, d, *J*=1.6 Hz), 4.21 (3H, s), 4.07 - 3.92 (2H, m), 3.67 - 3.45 (3H, m), 2.50 - 2.44 (2H, m), 1.95 - 1.82 (1H, m), 1.81 - 1.70 (1H, m), 1.48 - 1.21 (12H, m), 0.69 (3H, t, *J*=7.3 Hz).

### Synthetic method for similar compounds:

| Compounds | Structure | Synthetic methods |
|---|---|---|
| **21** | 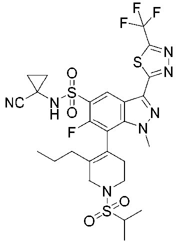 | Referring to the synthesis route of compound **7',** using **1-a** instead of**3'-d.** |
| **22** | 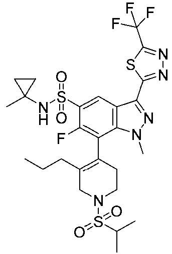 | Referring to the synthesis route of compound **7'**, using **5-a** instead of**3'-d.** |
| **23** | 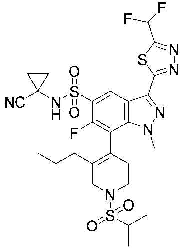 | Referring to the synthesis route of compound **7',** using **3-a** instead of**3'-d.** |
| **24** | 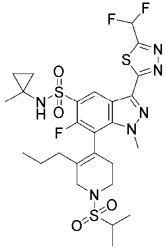 | Referring to the synthesis route of compound 7', using **7-a** instead of**3'-d.** |

### Biological activity assays

### PARG enzyme inhibition assay

### Experimental procedure:

The in vitro PARG assay experiment was performed in a standard 384-well plate with a total reaction volume of 15 µL. In a buffer solution (50 mM Tris-HCl pH 7.5, 30 mM KCl, 1 mM EDTA, 3 mM DTT, 0.01% Tween-20, 0.025% BSA), 5 µL of PARG (389-976) (manufactured by ChemPartner Co., Ltd.) was added into the 384-well plate containing test compounds (with various concentrations) to obtain a final concentration of 1.5 pM, followed by incubation at room temperature for 30 minutes. Subsequently, 5 µL of Bio PARylated His-TEV-PARP1 (2-1014) substrate (manufactured by ChemPartner Co., Ltd.) was added into the above reaction mixture to obtain a final concentration of 12 nM, and the reaction was carried out at room temperature for 30 minutes after the addition was completed. Then, 5 µL of detection reagent was added. The detection reagent was prepared by buffer solution (50 mM Tris-HCl pH 7.5, 30 mM KCl, 1 mM EDTA, 3 mM DTT, 0.01% Tween-20, 0.025% BSA) consisting of 3 µM compound PDD00017273, 9 nM Mab anti-6HIS XL665 (Cisbio: 61HISXLA) and 0.9 nM streptavidin cryptate (Cisbio: 610SATLA). All three components were used at 3X working concentration, corresponding to final concentrations of 1 µM, 3 nM and 0.3 nM, respectively. After incubation at room temperature for 120 minutes in the dark place, the TR-FRET signals were measured at an excitation wavelength (Ex) of 340 nm, emission wavelengths (Em) of 665 nm and 615 nm. The ratio for each well was calculated as Em 665 / Em 615, and the compound inhibition rate was determined based on the obtained data.

**Table 1 Biochemical activity against PARG of the representative compounds of the rresent invention**

| Compound No. | IC₅₀ (PARG enzyme) | Compound No. | IC₅₀ (PARG enzyme) | Compound No. | IC₅₀ (PARG enzyme) |
|---|---|---|---|---|---|
| **1** | 0.23 nM | **2** | 0.30 nM | **5** | 0.43 nM |
| **6** | 0.35 nM | **1'** | 0.22 nM | **2'** | 0.17 nM |
| **3'** | 0.28 nM | **3** | 0.19 nM | **4** | 0.48 nM |
| **7** | 0.21 nM | **8** | 0.17 nM | **9** | 0.56 nM |
| **10** | 1.24 nM | **11** | 0.70 nM | **4'** | 0.60 nM |

### Cellular assay: Experiment on Inhibitory effect of compounds on cell line proliferation detected by CTG assay

The relevant assays were performed in 3 84-well plates or 96-well plates. The detailed procedure is as follows:
The tested cell suspension was added into 384-well or 96-well plates (excluding peripheral wells) (384-well plate: 50 µL; 96-well plate: 100 µL), and the plates were incubated in a carbon dioxide incubator overnight; specifically, HCC1395 and HCC1428 cells were incubated in a 5% carbon dioxide incubator at 37°C overnight. The tested compounds (9-10 compound concentration gradients via 3-fold serial dilution) were added into the corresponding well by a HPD300 micro-dosing instrument. The cell plates were incubated in a carbon dioxide incubator for 7 days; specifically, for HCC1395 and HCC1428 cells, maintaining the incubation condition at 5% carbon dioxide and 37°C throughout this period. On the day of detection, firstly, the 384-well or 96-well plates were equilibrated at room temperature for 10-30 minutes, and then CellTiter-Glo reagent was added into each plate (384-well plate: 25 µL; 96-well plate: 100 µL), the plates were shaked for 10 minutes in the dark place, and incubated for another 10 minutes. The plates were placed into the Victor NIVO microplate reader for measurement. The efficacy inhibition rate curves were ploted using XLFit software and the IC₅₀ values were calculated.

**Table 2 Anti-proliferative activity assay results of the representative compounds of the present disclosure against cell lines**

| Compound No. | IC50 (µM) (HCC1395) | IC50 (µM) (HCC1428) | Compound No. | IC50 (µM) (HCC1395) | IC50 (µM) (HCC1428) |
|---|---|---|---|---|---|
| **1** | 0.0949 | | **2** | 0.0368 | |
| **5** | 0.0430 | | **6** | 0.0913 | |
| **1'** | 0.0955 | 0.1165 | **2'** | 0.0633 | |
| **3'** | 0.0352 | | **3** | 0.0992 | |
| **4** | 0.0445 | | **7** | 0.0740 | |
| **8** | 0.0322 | | **9** | 0.04463 | |
| **12** | 0.7699 | | **13** | 0.0313 | |

### Kinetic solubility assay in PBS (pH 7.4)

PBS (pH 7.4) buffer solution was repared and preheated. 8 µL of stock solution (10 mM) for the tested compound was added into a sample tube containing 792 µL of PBS (pH 7.4) (final concentration of DMSO in the sample tube: 1%). Sample tube was shaked (1000 rpm) at room temperature for 1 hour, and then centrifuged (12000 rpm for 10 minutes) to precipitate the undissolved particles. Subsequently, the supernatant was transferred to a new tube or plate and 10-fold or 100-fold diluted with PBS (pH 7.4) buffer solution. Standard curve samples were prepared by adding 6 µL of the tested compound (10 mM) into 194 µL of MeOH/ACN (4:1), and then performing serial dilutions using MeOH:ACN (4:1) as the diluent. All the samples were analyzed via LC-MS/MS, and the solubility was calculated based on the analytical results.

### Pharmacokinetic study of Mice via intravenous injection and oral administration

### Experimental procedure:

Plasma samples from male C57BL/6J mice or male CD-1 mice after intravenous injection and oral administration dosing with the tested compound, were collected from the orbital venous plexus at 8-9 time points (e.g., 5 min, 15 min, 30 min, 1 h, 2 h, 4 h, 7 h, 24 h or 5 min, 15 min, 30 min, 1 h, 2 h, 4 h, 6 h, 8 h, 24 h). The collected blood samples were transferred to microcentrifuge tubes containing EDTA-K₂ anticoagulant, centrifuged at 4000 g and 4°C for 5 min. The resulting supernatant was pipetted and stored in a -75°C ± 15°C refrigerator . The compound concentrations in plasma samples at different time points were determined via LC-MS/MS, and the relevant pharmacokinetic parameters were calculated using WinNonlin software.

**Table 3-1 Pharmacokinetic determination results of the representative compounds of the present disclosure**

| Administration route | | Tested compound | | | |
|---|---|---|---|---|---|
| | | Compound **1^{a}** | Compound **1**'^{a} | Compound **2**^{b} | Compound **3'^{a}** |
| Intravenous administration (5mg/kg) | Cl (mL/min/kg) | 1.89 | 5.92 | 1.68 | 3.08 |
| | AUC₀₋ₗₐₛₜ (h*ng/mL) | 43973 | 14086 | 49617 | 27050 |
| | AUC_{0-inf} (h*ng/mL) | 44122 | 14087 | 50063 | 27052 |
| Oral administration (10mg/kg) | Cmax (ng/mL) | 4324 | 1889 | 5373 | 3621 |
| | AUC₀₋ₗₐₛₜ (h*ng/mL) | 32753 | 15694 | 46669 | 8693 |
| | AUC_{0-inf} (h*ng/mL) | 32781 | 15700 | 46898 | 8799 |

| | | | | | |
|---|---|---|---|---|---|
| ^{a}: Male CD-1 mice, values collected by processing and analyzing the mixed blood samples; ^{b}: Male CD-1 mice, mean values collected by individual processing and analyzing each blood sample. Vehicle for intravenous administration: 5% DMSO + 10% Solutol HS-15 + 85% (20% HP-β-CD in water) or 5% DMSO + 15% Solutol HS-15 + 80% Saline; Vehicle for oral administration: 0.1% Tween 80 + 0.5% MC in water. | | | | | |

**Table 3-2 Pharmacokinetic determination results of the representative compounds of the present disclosure**

| Administration route | | Tested compound | | | |
|---|---|---|---|---|---|
| | | Compound **3^{a}** | Compound **2'^{a}** | Compound **9^{b}** | Compound **4'^{b}** |
| Intravenous administration (5mg/kg) | Cl (mL/min/kg) | 2.47 | 5.35 | 9.80 | 23.5 |
| | AUC₀₋ₗₐₛₜ (h*ng/mL) | 33705 | 15564 | 8553 | 3534 |
| | AUC_{0-inf} (h*ng/mL) | 33707 | 15566 | 8558 | 3538 |
| Oral administration (10mg/kg) | Cmax (ng/mL) | 3301 | 2311 | 6155 | 2670 |
| | AUC₀₋ₗₐₛₜ (h*ng/mL) | 11951 | 6396 | 11491 | 7640 |
| | AUC_{0-inf} (h*ng/mL) | 11960 | 6483 | 11520 | 7651 |

**Table 3-3 Pharmacokinetic determination results of the representative compounds of the present disclosure**

| Administration route | | Tested compound | | | |
|---|---|---|---|---|---|
| | | Compound **4**^{b} | Compound **5**'^{a} | Compound **13^{a}** | Compound **6'^{a}** |
| Intravenous administration (5mg/kg) | Cl (mL/min/kg) | 4.17 | 11.6 | 2.32 | 6.62 |
| | AUC₀₋ₗₐₛₜ (h*ng/mL) | 18411 | 7141 | 35887 | 11555 |
| | AUC_{0-inf} (h*ng/mL) | 20311 | 7174 | 35937 | 12587 |
| Oral administration (10mg/kg) | Cmax (ng/mL) | 2006 | 857 | 2502 | 2917 |
| | AUC₀₋ₗₐₛₜ (h*ng/mL) | 10971 | 2368 | 25357 | 10665 |
| | AUC_{0-inf} (h*ng/mL) | 11283 | 2587 | 25399 | 10805 |

| | | | | | |
|---|---|---|---|---|---|
| ^{a}: Male CD-1 mice, values collected by processing and analyzing the mixed blood samples; ^{b}: Male CD-1 mice, mean values collected by individual processing and analyzing each blood sample. Vehicle for intravenous administration: 5% DMSO + 10% Solutol HS-15 + 85% (20% HP-β-CD in water) or 5% DMSO + 15% Solutol HS-15 + 80% Saline; Vehicle for oral administration: 0.1% Tween 80 + 0.5% MC in water. Data from Tables 3-1, 3-2 and 3-3 demonstrated that, comparing with the reference compounds, compounds of the present disclosure exhibited distinct advantages in pharmacokinetic (PK) parameters and achieved a significant increase on the exposure level. | | | | | |

### In vivo pharmacodynamic study

Estrogen was administered to BALB/c nude mice via injection twice weekly until the end of the experiment, subcutaneous inoculation with 1×10⁷ HCC1428 cells (suspended in 100 µL of RPMI medium supplemented with Matrigel at a 1:1 volume ratio). Grouping and drug administration were initiated when the average tumor volume reached 150-200 mm³. During the experiment, the body weight of the mice and tumor size were measured twice weekly, and the clinical symptoms of the animals were observed and recorded daily. The length (a) and width (b) of the tumors were measured using a vernier caliper, and the tumor volume was calculated according to the formula: TV = a×b²/2. The tumor growth inhibition rate (TGI, %) was calculated based on the tumor volume using the formula: TGI (%) = [1 - (Tᵢ - T₀)/ (Vᵢ - V₀)] ×100%, wherein Tᵢ represents the mean tumor volume of the treatment group on the i-th day after grouping, T₀ represents the mean tumor volume of the treatment group at the time of grouping, Vᵢ represents the mean tumor volume of the vehicle control group on the i-th day after grouping, and V₀ represents the mean tumor volume of the vehicle control group at the time of grouping.

Pharmacodynamic study results of the representative compound in the mouse model bearing subcutaneously implanted HCC1428 cell-derived tumors

| Group | Number of animals | Tested substance | Dosing frequency | Dose mg/kg | Tumor Growth Inhibition Rate TGI (%) |
|---|---|---|---|---|---|
| Group 1 | 7 | Vehicle control | PO, BID*28 | --- | --- |
| Group 2 | 7 | Compound 1 | PO, BID*28 | 30 | 106.8% |

| | | | | | |
|---|---|---|---|---|---|
| Note: The vehicle consists of 0.5% MC + 0.5% Tween 80; grouping and drug administration were initiated when the average tumor volume reached 195 mm³; PO denotes oral administration, and BID denotes twice daily. | | | | | |

The compound of the present disclosure exhibited extremely significant pharmacodynamic efficacy in the mouse model bearing subcutaneously implanted HCC1428 cell-derived tumors.

Although the specific embodiments of the present disclosure have been described above, those skilled in the art should understand that these are merely illustrative examples. Various changes or modifications may be made to these embodiments without departing from the principles and essence of the present disclosure. Therefore, the scope of protection of the present disclosure is defined by the appended claims.

## Claims

1. A heteroaromatic sulfonamide structural compound represented by formula I, a pharmaceutically acceptable salt thereof, a stereoisomer thereof, a tautomer thereof or an isotopically labeled compound thereof: 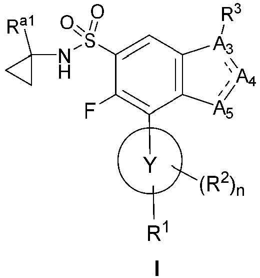
"-̅ -̅ -̅" represents a single bond or a double bond;
A₃ is C or N;
A₄ is N or CR⁴;
A₅ is NR⁵ or CR⁵; 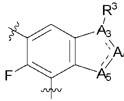
is 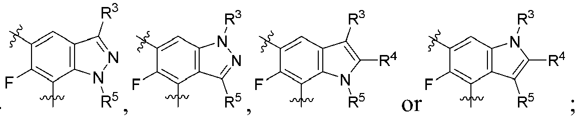
R^{a1} is cyano or C₁₋₆ alkyl;
ring Y is a "4- to 12-membered heterocycloalkyl containing 1 to 3 heteroatoms each independently selected from O, S and N", or a "4- to 8-membered heterocycloalkenyl containing 1 to 3 heteroatoms each independently selected from O, S and N";
R¹ is 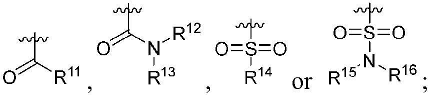
R¹¹, R¹², R¹³, R¹⁴, R¹⁵ and R¹⁶ are each independently selected from C₁₋₆ alkyl, C₁₋₆ alkyl substituted with one or more R¹⁻¹, C₃₋₁₀ cycloalkyl, C₃₋₁₀ cycloalkyl substituted with one or more R¹⁻², a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R¹⁻³, C₆₋₂₀ aryl, C₆₋₂₀ aryl substituted with one or more R¹⁻⁴, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R¹⁻⁵, C₄₋₈ cycloalkenyl, C₄₋₈ cycloalkenyl substituted with one or more R¹⁻⁶, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R¹⁻⁷;
R¹⁻¹, R¹⁻², R¹⁻³, R¹⁻⁴, R¹⁻⁵, R¹⁻⁶ and R¹⁻⁷ are each independently halogen, hydroxyl, cyano, C₁₋₆ alkyl, C₂₋₆ alkenyl, C₂₋₆ alkynyl, -OC₁₋₆ alkyl, -SC₁₋₆ alkyl, -C(=OR^{1a}, -NR^{1b1}R^{1b2}, -C(=O)OR^{1c}, -C(=O)NR^{1d1}R^{1d2}, -S(O)₂NR^{1c1}R^{1c2}, -S(O)₂R^{1f}, C₃₋₁₀ cycloalkyl, a "4- to 12-membered heterocycloalkyl containing 1 to 3 heteroatoms each independently selected from O, S and N", C₆₋₂₀ aryl, a "5- to 12-membered heteroaryl containing 1 to 4 heteroatoms each independently selected from O, S and N", C₄₋₈ cycloalkenyl, or a "4- to 8-membered heterocycloalkenyl containing 1 to 3 heteroatoms each independently selected from O, S and N"; when there are multiple substituents, they may be the same or different;
R^{1a}, R^{1b1}, R^{1b2}, R^{1c}, R^{1d1}, R^{1d2}, R^{1c1}, R^{1c2} and R^{1f} are each independently hydrogen, C₁₋₆ alkyl, or C₁₋₆ alkyl substituted with one or more halogen atoms;
n is 0, 1, 2, 3, 4, 5 or 6;
R² is halogen, hydroxyl, cyano, oxo, C₁₋₆ alkyl, C₁₋₆ alkyl substituted with one or more R²⁻¹, C₃₋₈ cycloalkyl, C₃₋₈ cycloalkyl substituted with one or more R²⁻², C₂₋₆ alkenyl, C₂₋₆ alkynyl, -OC₁₋₆ alkyl, -SC₁₋₆ alkyl, -C(=O)R^{2a}, -NR^{2b1}R^{2b2}, -C(=O)OR^{2c}, or -C(=O)NR^{2d1}R^{2d2}; alternatively, when n is 2, 3, 4, 5 or 6, any two R² are linked to each other, independently form, together with the atoms on the ring to which they are attached, a 3- to 8-membered carbocyclic ring, or a "3-to 8-membered heterocyclic ring containing 1 to 3 heteroatoms each independently selected from O, S and N";
R²⁻¹ and R²⁻² are each independently halogen, cyano, C₁₋₆ alkyl-O-, hydroxyl, -C(=O)R^{22a}, - NR^{22b1}R^{22b2}, -C(=O)OR^{22c}, or -C(=O)NR^{22d1}R^{22d2};
R^{2a}, R^{2b1}, R^{2b2}, R^{2c}, R^{2d1}, R^{2d2}, R^{22a}, R^{22b1}, R^{22b2}, R^{22c}, R^{22d1} and R^{22d2} are each independently hydrogen, C₁₋₆ alkyl, or C₁₋₆ alkyl substituted with one or more halogen atoms;
R³ is a "5- to 6-membered heteroaryl containing 1 to 4 heteroatoms each independently selected from O, S and N", or a "5- to 6-membered heteroaryl containing 1 to 4 heteroatoms each independently selected from O, S and N" and substituted with one or more R³⁻¹;
R³⁻¹ is halogen, hydroxyl, amino, cyano, C₁₋₆ alkyl, C₁₋₆ alkyl substituted with one or more halogen atoms, C₁₋₆ alkyl substituted with one or more -OC₁₋₆ alkyl, -OC₁₋₆ alkyl, -SC₁₋₆ alkyl, -OC₁₋₆ alkyl substituted with one or more halogen atoms, pentafluorosulfanyl, or -SC₁₋₆ alkyl substituted with one or more halogen atoms; when there are multiple substituents, they may be the same or different;
R⁴ is hydrogen, halogen, C₁₋₆ alkyl, or C₁₋₆ alkyl substituted with one or more halogen atoms; R⁵ is hydrogen, C₁₋₆ alkyl, or C₁₋₆ alkyl substituted with one or more R⁵⁻¹;
R⁵⁻¹ is deuterium, halogen, cyano, C₂₋₆ alkenyl, C₂₋₆ alkynyl, hydroxyl, -OC₁₋₆ alkyl, -C(=O)R^{5a}, -NR^{5b1}R^{5b2}, -C(=O)OR^{5c}, -C(=O)NR^{5d1}R^{5d2}, C₆₋₂₀ aryl, C₆₋₂₀ aryl substituted with one or more R⁵⁻¹⁻¹, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R⁵⁻¹⁻², C₃₋₁₂ cycloalkyl, C₃₋₁₂ cycloalkyl substituted with one or more R⁵⁻¹⁻³, a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R⁵⁻¹⁻⁴, C₄₋₈ cycloalkenyl, C₄₋₈ cycloalkenyl substituted with one or more R⁵⁻¹⁻⁵, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R⁵⁻¹⁻⁶; when there are multiple substituents, they may be the same or different;
R⁵⁻¹⁻¹, R⁵⁻¹⁻², R⁵⁻¹⁻³, R⁵⁻¹⁻⁴, R⁵⁻¹⁻⁵ and R⁵⁻¹⁻⁶ are each independently halogen, C₁₋₆ alkyl, or C₁₋₆ alkyl substituted with one or more halogen atoms;
R^{5a}, R^{5b1}, R^{5b2}, R^{5c}, R^{5d1} and R^{5d2} are each independently hydrogen, C₁₋₆ alkyl, or C₁₋₆ alkyl substituted with one or more halogen atoms;
the heteroaromatic sulfonamide structural compound represented by formula I is not any one of the following compounds:
N-(1-methylcyclopropyl)-3-(5-(difluoromethyl)-1,3,4-thiadiazol-2-yl)-1-ethyl-6-fluoro-7-(4-isobutyrylpiperazin-1-yl)-1H-indazole-5-sulfonamide;
N-(1-cyanocyclopropyl)-6-fluoro-7-(4-(isopropylsulfonyl)piperazin-1-yl)-1-methyl-3-(5-(trifluoromethyl)-1 ,3,4-thiadiazol-2-yl)-1H-indazole-5-sulfonamide.

2. The heteroaromatic sulfonamide structural compound represented by formula I, the pharmaceutically acceptable salt thereof, the stereoisomer thereof, the tautomer thereof or the isotopically labeled compound thereof according to claim 1, **characterized in that** the structure of the heteroaromatic sulfonamide structural compound represented by formula I is as shown in formula II: 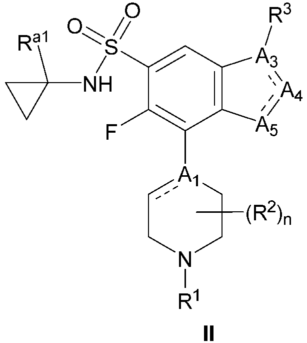
wherein, "-̅ -̅ -̅" represents a single bond or a double bond;
A₁ is C, CH or N;
A₃ is C or N;
A₄ is N or CR⁴;
A₅ is NR⁵ or CR⁵; 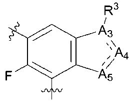
is 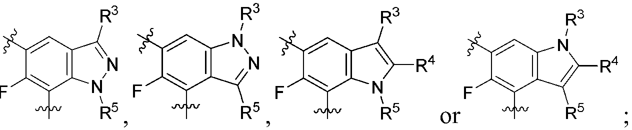
R^{a1} is cyano or C₁₋₆ alkyl;
R¹ is 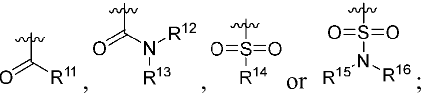
R¹¹, R¹², R¹³, R¹⁴, R¹⁵ and R¹⁶ are each independently C₁₋₆ alkyl, C₁₋₆ alkyl substituted with one or more R¹⁻¹, C₃₋₁₀ cycloalkyl, C₃₋₁₀ cycloalkyl substituted with one or more R¹⁻², a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R¹⁻³, C₆₋₂₀ aryl, C₆₋₂₀ aryl substituted with one or more R¹⁻⁴, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R¹⁻⁵, C₄₋₈ cycloalkenyl, C₄₋₈ cycloalkenyl substituted with one or more R¹⁻⁶, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R¹⁻⁷;
R¹⁻¹, R¹⁻², R¹⁻³, R¹⁻⁴, R¹⁻⁵, R¹⁻⁶ and R¹⁻⁷ are each independently halogen, hydroxyl, cyano, C₁₋₆ alkyl, C₂₋₆ alkenyl, C₂₋₆ alkynyl, -OC₁₋₆ alkyl, -SC₁₋₆ alkyl, -C(=O)R^{1a}, -NR^{1b1}R^{1b2}, -C(=O)OR^{1c}, -C(=O)NR^{1d1}R^{1d2}, -S(O)₂NR^{1c1}R^{1c2}, -S(O)₂R^{1f}, C₃₋₁₀ cycloalkyl, a "4- to 12-membered heterocycloalkyl containing 1 to 3 heteroatoms each independently selected from O, S and N", C₆₋₂₀ aryl, a "5- to 12-membered heteroaryl containing 1 to 4 heteroatoms each independently selected from O, S and N", C₄₋₈ cycloalkenyl, or a "4- to 8-membered heterocycloalkenyl containing 1 to 3 heteroatoms each independently selected from O, S and N"; when there are multiple substituents, they may be the same or different;
R^{1a}, R^{1b1}, R^{1b2}, R^{1c}, R^{1d1}, R^{1d2}, R^{1c1}, R^{1c2} and R^{1f} are each independently hydrogen, C₁₋₆ alkyl, or C₁₋₆ alkyl substituted with one or more halogen atoms;
n is 0, 1, 2, 3, 4, 5 or 6;
R² is halogen, hydroxyl, cyano, oxo, C₁₋₆ alkyl, C₁₋₆ alkyl substituted with one or more R²⁻¹, C₃₋₈ cycloalkyl, C₃₋₈ cycloalkyl substituted with one or more R²⁻², C₂₋₆ alkenyl, C₂₋₆ alkynyl, -OC₁₋₆ alkyl, -SC₁₋₆ alkyl, -C(=O)R^{2a}, -NR^{2b1}R^{2b2}, -C(=O)OR^{2c}, or -C(=O)NR^{2d1}R^{2d2}; alternatively, when n is 2, 3, 4, 5 or 6, any two R² are linked to each other, independently form, together with the atoms on the ring to which they are attached, a 3- to 8-membered carbocyclic ring, or a "3-to 8-membered heterocyclic ring containing 1 to 3 heteroatoms each independently selected from O, S and N";
R²⁻¹ and R²⁻² are each independently halogen, cyano, C₁₋₆ alkyl-O-, hydroxyl, -C(=O)R^{22a}, - NR^{22b1}R^{22b2}, -C(=O)OR^{22c}, or -C(=O)NR^{22d1}R^{22d2} ;
R^{2a}, R^{2b1}, R^{2b2}, R^{2c}, R^{2d1}, R^{2d2}, R^{22a}, R^{22b1}, R^{22b2}, R^{22c}, R^{22d1} and R^{22d2} are each independently hydrogen, C₁₋₆ alkyl, or C₁₋₆ alkyl substituted with one or more halogen atoms;
R³ is a "5- to 6-membered heteroaryl containing 1 to 4 heteroatoms each independently selected from O, S and N", or a "5- to 6-membered heteroaryl containing 1 to 4 heteroatoms each independently selected from O, S and N" and substituted with one or more R³⁻¹;
R³⁻¹ is halogen, hydroxyl, amino, cyano, C₁₋₆ alkyl, C₁₋₆ alkyl substituted with one or more halogen atoms, C₁₋₆ alkyl substituted with one or more -OC₁₋₆ alkyl, -OC₁₋₆ alkyl, -SC₁₋₆ alkyl, -OC₁₋₆ alkyl substituted with one or more halogen atoms, pentafluorosulfanyl, or -SC₁₋₆ alkyl substituted with one or more halogen atoms; when there are multiple substituents, they may be the same or different;
R⁴ is hydrogen, halogen, C₁₋₆ alkyl, or C₁₋₆ alkyl substituted with one or more halogen atoms; R⁵ is hydrogen, C₁₋₆ alkyl, or C₁₋₆ alkyl substituted with one or more R⁵⁻¹;
R⁵⁻¹ is deuterium, halogen, cyano, C₂₋₆ alkenyl, C₂₋₆ alkynyl, hydroxyl, -OC₁₋₆ alkyl, -C(=O)R^{5a}, -NR^{5b1}R^{5b2}, -C(=O)OR^{5c}, -C(=O)NR^{5d1}R^{5d2}, C₆₋₂₀ aryl, C₆₋₂₀ aryl substituted with one or more R⁵⁻¹⁻¹, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R⁵⁻¹⁻², C₃₋₁₂ cycloalkyl, C₃₋₁₂ cycloalkyl substituted with one or more R⁵⁻¹⁻³, a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R⁵⁻¹⁻⁴, C₄₋₈ cycloalkenyl, C₄₋₈ cycloalkenyl substituted with one or more R⁵⁻¹⁻⁵, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R⁵⁻¹⁻⁶; when there are multiple substituents, they may be the same or different;
R⁵⁻¹⁻¹, R⁵⁻¹⁻², R⁵⁻¹⁻³, R⁵⁻¹⁻⁴, R⁵⁻¹⁻⁵ and R⁵⁻¹⁻⁶ are each independently halogen, C₁₋₆ alkyl, or C₁₋₆ alkyl substituted with one or more halogen atoms;
R^{5a}, R^{5b1}, R^{5b2}, R^{5c}, R^{5d1} and R^{5d2} are each independently hydrogen, C₁₋₆ alkyl, or C₁₋₆ alkyl substituted with one or more halogen atoms;
the heteroaromatic sulfonamide structural compound represented by formula I is not any one of the following compounds:
N-(1-methylcyclopropyl)-3-(5-(difluoromethyl)-1,3,4-thiadiazol-2-yl)-1-ethyl-6-fluoro-7-(4-isobutyrylpiperazin-1-yl)-1H-indazole-5-sulfonamide;
N-(1-cyanocyclopropyl)-6-fluoro-7-(4-(isopropylsulfonyl)piperazin-1-yl)-1-methyl-3-(5-(trifluoromethyl)-1,3,4-thiadiazol-2-yl)-1H-indazole-5-sulfonamide.

3. The heteroaromatic sulfonamide structural compound represented by formula I, the pharmaceutically acceptable salt thereof, the stereoisomer thereof, the tautomer thereof or the isotopically labeled compound thereof according to claim 1, **characterized in that** in the structure represented by formula I:
A₃ is C;
A₄ is N;
A₅ is NR⁵; 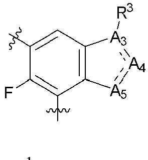
is 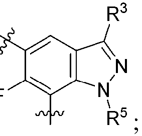
R^{a1} is cyano or C₁₋₆ alkyl;
Ring Y is a "4- to 12-membered heterocycloalkyl containing 1 to 3 heteroatoms each independently selected from O, S and N", or a "4- to 8-membered heterocycloalkenyl containing 1 to 3 heteroatoms each independently selected from O, S and N";
R¹ is 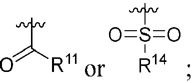
R¹¹ and R¹⁴ are each independently C₁₋₆ alkyl;
n is 0, 1 or 2;
R² is cyano, C₃₋₈ cycloalkyl or C₁₋₆ alkyl;
R³ is a "5- to 6-membered heteroaryl containing 1 to 4 heteroatoms each independently selected from O, S and N" and substituted with one or more R³⁻¹;
R³⁻¹ is C₁₋₆ alkyl substituted with one or more halogen atoms;
R⁵ is C₁₋₆ alkyl;
the heteroaromatic sulfonamide structural compound represented by formula I is not any one of the following compounds:
N-(1-methylcyclopropyl)-3-(5-(difluoromethyl)-1,3,4-thiadiazol-2-yl)-1-ethyl-6-fluoro-7-(4-isobutyrylpiperazin-1-yl)-1H-indazole-5-sulfonamide;
N-(1-cyanocyclopropyl)-6-fluoro-7-(4-(isopropylsulfonyl)piperazin-1-yl)-1-methyl-3-(5-(trifluoromethyl)-1,3,4-thiadiazol-2-yl)-1H-indazole-5-sulfonamide.

4. The heteroaromatic sulfonamide structural compound represented by formula I, the pharmaceutically acceptable salt thereof, the stereoisomer thereof, the tautomer thereof or the isotopically labeled compound thereof according to claim 1, **characterized in that** the heteroaromatic sulfonamide structural compound represented by formula I, the pharmaceutically acceptable salt thereof, the stereoisomer thereof, the tautomer thereof or the isotopically labeled compound thereof is as defined in Embodiment 1, Embodiment 2 or Embodiment 3:
Embodiment 1: in the heteroaromatic sulfonamide structural compound represented by formula I,
"-̅ -̅ -̅" represents a single bond or a double bond;
A₃ is C or N;
A₄ is N or CR⁴;
A₅ is NR⁵ or CR⁵; 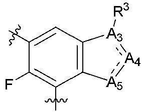
is 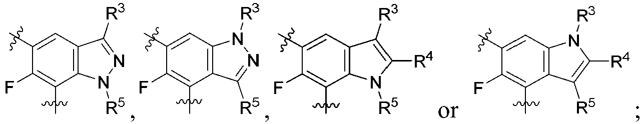
R^{a1} is cyano or C₁₋₆ alkyl;
ring Y is a "4- to 12-membered heterocycloalkyl containing 1 to 3 heteroatoms each independently selected from O, S and N", or a "4- to 8-membered heterocycloalkenyl containing 1 to 3 heteroatoms each independently selected from O, S and N";
R¹ is 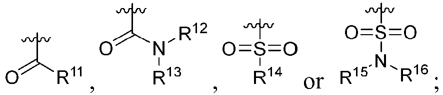
R¹¹, R¹², R¹³, R¹⁴, R¹⁵ and R¹⁶ are each independently C₁₋₆ alkyl, C₁₋₆ alkyl substituted with one or more R¹⁻¹, C₃₋₁₀ cycloalkyl, C₃₋₁₀ cycloalkyl substituted with one or more R¹⁻², a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R¹⁻³, C₆₋₂₀ aryl, C₆₋₂₀ aryl substituted with one or more R¹⁻⁴, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R¹⁻⁵, C₄₋₈ cycloalkenyl, C₄₋₈ cycloalkenyl substituted with one or more R¹⁻⁶, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R¹⁻⁷;
R¹⁻¹, R¹⁻², R¹⁻³, R¹⁻⁴, R¹⁻⁵, R¹⁻⁶ and R¹⁻⁷ are each halogen, hydroxyl, cyano, C₁₋₆ alkyl, C₂₋₆ alkenyl, C₂₋₆ alkynyl, -OC₁₋₆ alkyl, -SC₁₋₆ alkyl, -C(=O)R^{1a}, -NR^{1b1}R^{1b2}, -C(=O)OR^{1c}, - C(=O)NR^{1a1}R^{1a2}, -S(O)₂NR^{1c1}R^{1c2}, -S(O)₂R^{1f}, C₃₋₁₀ cycloalkyl, a "4- to 12-membered heterocycloalkyl containing 1 to 3 heteroatoms each independently selected from O, S and N", C₆₋₂₀ aryl, a "5- to 12-membered heteroaryl containing 1 to 4 heteroatoms each independently selected from O, S and N", C₄₋₈ cycloalkenyl, or a "4- to 8-membered heterocycloalkenyl containing 1 to 3 heteroatoms each independently selected from O, S and N"; when there are multiple substituents, they may be the same or different;
R^{1a}, R^{1b1}, R^{1b2}, R^{1c}, R^{1d1}, R^{1d2}, R^{1c1}, R^{1c2} and R^{1f} are each independently hydrogen, C₁₋₆ alkyl, or C₁₋₆ alkyl substituted with one or more halogen atoms;
n is 0, 1, 2, 3, 4, 5 or 6;
R² is halogen, hydroxyl, cyano, oxo, C₁₋₆ alkyl, C₁₋₆ alkyl substituted with one or more R²⁻¹, C₂₋₆ alkenyl, C₂₋₆ alkynyl, -OC₁₋₆ alkyl, -SC₁₋₆ alkyl, -C(=O)R^{2a}, -NR^{2b1}R^{2b2}, -C(=O)OR^{2c} or - C(=O)NR^{2d1}R^{2d2}; alternatively, when n is 2, 3, 4, 5 or 6, any two R² are linked to each other, independently form, together with the atoms on the ring to which they are attached, a 3- to 8-membered carbocyclic ring, or a "3- to 8-membered heterocyclic ring containing 1 to 3 heteroatoms each independently selected from O, S and N";
R²⁻¹ is halogen, cyano, C₁₋₆ alkyl-O-, hydroxyl, -C(=O)R^{22a}, -NR^{22b1}R^{22b2}, -C(=O)OR^{22c} or - C(=O)NR^{22d1}R^{22d2};
R^{2a}, R^{2b1}, R^{2b2}, R^{2c}, R^{2d1}, R^{2d2}, R^{22a}, R^{22b1}, R^{22b2}, R^{22c}, R^{22d1} and R^{22d2} are each independently hydrogen, C₁₋₆ alkyl, or C₁₋₆ alkyl substituted with one or more halogen atoms;
R³ is a "5- to 6-membered heteroaryl containing 1 to 4 heteroatoms each independently selected from O, S and N", or a "5- to 6-membered heteroaryl containing 1 to 4 heteroatoms each independently selected from O, S and N" and substituted with one or more R³⁻¹;
R³⁻¹ is halogen, hydroxyl, amino, cyano, C₁₋₆ alkyl, C₁₋₆ alkyl substituted with one or more halogen atoms, C₁₋₆ alkyl substituted with one or more -OC₁₋₆ alkyl, -OC₁₋₆ alkyl, -SC₁₋₆ alkyl, -OC₁₋₆ alkyl substituted with one or more halogen atoms, pentafluorosulfanyl, or -SC₁₋₆ alkyl substituted with one or more halogen atoms; when there are multiple substituents, they may be the same or different;
R⁴ is hydrogen, halogen, C₁₋₆ alkyl, or C₁₋₆ alkyl substituted with one or more halogen atoms;
R⁵ is hydrogen, C₁₋₆ alkyl, or C₁₋₆ alkyl substituted with one or more R⁵⁻¹;
R⁵⁻¹ is deuterium, halogen, cyano, C₂₋₆ alkenyl, C₂₋₆ alkynyl, hydroxyl, -OC₁₋₆ alkyl, -C(=O)R^{5a}, -NR^{5b1}R^{5b2}, -C(=O)OR^{5c}, -C(=O)NR^{5d1}R^{5d2}, C₆₋₂₀ aryl, C₆₋₂₀ aryl substituted with one or more R⁵⁻¹⁻¹, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R⁵⁻¹⁻², C₃₋₁₂ cycloalkyl, C₃₋₁₂ cycloalkyl substituted with one or more R⁵⁻¹⁻³, a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R⁵⁻¹⁻⁴, C₄₋₈ cycloalkenyl, C₄₋₈ cycloalkenyl substituted with one or more R⁵⁻¹⁻⁵, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R⁵⁻¹⁻⁶; when there are multiple substituents, they may be the same or different;
R⁵⁻¹⁻¹, R⁵⁻¹⁻², R⁵⁻¹⁻³, R⁵⁻¹⁻⁴, R⁵⁻¹⁻⁵ and R⁵⁻¹⁻⁶ are each independently halogen, C₁₋₆ alkyl, or C₁₋₆ alkyl substituted with one or more halogen atoms;
R^{5a}, R^{5b1}, R^{5b2}, R^{5c}, R^{5d1} and R^{5d2} are each independently hydrogen, C₁₋₆ alkyl, or C₁₋₆ alkyl substituted with one or more halogen atoms;
the heteroaromatic sulfonamide structural compound represented by formula I is not any one of the following compounds:
N-(1-methylcyclopropyl)-3-(5-(difluoromethyl)-1,3,4-thiadiazol-2-yl)-1-ethyl-6-fluoro-7-(4-isobutyrylpiperazin-1-yl)-1H-indazole-5-sulfonamide;
N-(1-cyanocyclopropyl)-6-fluoro-7-(4-(isopropylsulfonyl)piperazin-1-yl)-1-methyl-3-(5-(trifluoromethyl)-1,3,4-thiadiazol-2-yl)-1H-indazole-5-sulfonamide;
Embodiment 2: the structure of the said heteroaromatic sulfonamide structural compound represented by formula I is as shown in formula II: 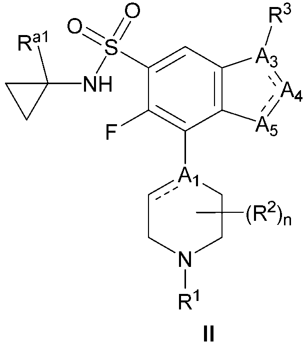
wherein, "-̅ -̅ -̅" represents a single bond or a double bond;
A₁ is C, CH or N;
A₃ is C or N;
A₄ is N or CR⁴;
A₅ is NR⁵ or CR⁵; 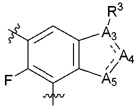
is 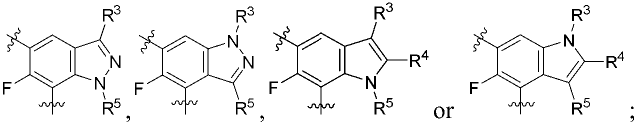
R^{a1} is cyano or C₁₋₆ alkyl;
R¹ is 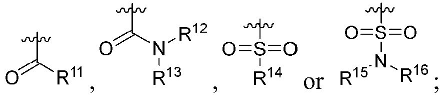
R¹¹, R¹² R¹³, R¹⁴, R¹⁵ and R¹⁶ are each independently C₁₋₆ alkyl, C₁₋₆ alkyl substituted with one or more R¹⁻¹, C₃₋₁₀ cycloalkyl, C₃₋₁₀ cycloalkyl substituted with one or more R¹², a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R¹⁻³, C₆₋₂₀ aryl, C₆₋₂₀ aryl substituted with one or more R¹⁻⁴, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R¹⁻⁵, C₄₋₈ cycloalkenyl, C₄₋₈ cycloalkenyl substituted with one or more R¹⁻⁶, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R¹⁻⁷;
R¹⁻¹, R¹⁻², R¹⁻³, R¹⁻⁴ R¹⁻⁵, R¹⁻⁶ and R¹⁻⁷ are each halogen, hydroxyl, cyano, C₁₋₆ alkyl, C₂₋₆ alkenyl, C₂₋₆ alkynyl, -OC₁₋₆ alkyl, -SC₁₋₆ alkyl, -C(=O)R^{1a}, -NR^{1b1}R^{1b2}, -C(=O)OR^{1c}, -C(=O)NR^{1d1}R^{1d2}, -S(O)₂NR^{1c1}R^{1c2}, -S(O)₂R¹¹, C₃₋₁₀ cycloalkyl, a "4- to 12-membered heterocycloalkyl containing 1 to 3 heteroatoms each independently selected from O, S and N", C₆₋₂₀ aryl, a "5-to 12-membered heteroaryl containing 1 to 4 heteroatoms each independently selected from O, S and N", C₄₋₈ cycloalkenyl, or a "4- to 8-membered heterocycloalkenyl containing 1 to 3 heteroatoms each independently selected from O, S and N"; when there are multiple substituents, they may be the same or different;
R^{1a}, R^{1b1}, R^{1b2}, R^{1c}, R^{1d1}, R^{1d2}, R^{1c1}, R^{1c2} and R^{1f} are independently hydrogen, C₁₋₆ alkyl,
or C₁₋₆ alkyl substituted with one or more halogen atoms;
n is 0, 1, 2, 3, 4, 5 or 6;
R² is halogen, hydroxyl, cyano, oxo, C₁₋₆ alkyl, C₁₋₆ alkyl substituted with one or more R²⁻¹, C₂₋₆ alkenyl, C₂₋₆ alkynyl, -OC₁₋₆ alkyl, -SC₁₋₆
alkyl, -C(=O)R^{2a}, -NR^{2b1}R^{2b2}, -C(=O)OR^{2c} or -C(=O)NR^{2d1}R^{2d2}; alternatively, when n is 2, 3, 4, 5 or 6, any two R² are linked to each other, independently form, together with the atoms on the ring to which they are attached, a 3- to 8-membered carbocyclic ring, or a "3- to 8-membered heterocyclic ring containing 1 to 3 heteroatoms each independently selected from O,
S and N";
R²⁻¹ is halogen, cyano, C₁₋₆ alkyl-O-,
hydroxyl, -C(=O)R^{22a}, -NR^{22b1}R^{22b2}, -C(=O)OR^{22c} or -C(=O)NR^{22d1}R^{22d2};
R^{2a}, R^{2b1}, R^{2b2}, R^{2c}, R^{2d1}, R^{2d2}, R^{22a}, R^{22b1}, R^{22b2}, R^{22c}, R^{22d1} and R^{22d2} are independently hydrogen, C₁₋₆ alkyl, or C₁₋₆ alkyl substituted with one or more halogen atoms;
R³ is a "5- to 6-membered heteroaryl containing 1 to 4 heteroatoms each independently selected from O, S and N", or a "5- to 6-membered heteroaryl containing 1 to 4 heteroatoms each independently selected from O, S and N" and substituted with one or more R³⁻¹;
R³⁻¹ is halogen, hydroxyl, amino, cyano, C₁₋₆ alkyl, C₁₋₆ alkyl substituted with one or more halogen atoms, C₁₋₆ alkyl substituted with one or more -OC₁₋₆ alkyl, -OC₁₋₆ alkyl, -SC₁₋₆ alkyl, -OC₁₋₆ alkyl substituted with one or more halogen atoms, pentafluorosulfanyl, or -SC₁₋₆ alkyl substituted with one or more halogen atoms; when there are multiple substituents, they may be the same or different;
R⁴ is hydrogen, halogen, C₁₋₆ alkyl, or C₁₋₆ alkyl substituted with one or more halogen atoms;
R⁵ is hydrogen, C₁₋₆ alkyl, or C₁₋₆ alkyl substituted with one or more R⁵⁻¹;
R⁵⁻¹ is deuterium, halogen, cyano, C₂₋₆ alkenyl, C₂₋₆ alkynyl, hydroxyl, -OC₁₋₆ alkyl, -C(=O)R^{5a}, -NR^{5b1}R^{5b2}, -C(=O)OR^{5c}, -C(=O)NR^{5d1}R^{5d2}, C₆₋₂₀ aryl, C₆₋₂₀ aryl
substituted with one or more R⁵⁻¹⁻¹, a "5- to 12-membered heteroaryl containing 1 to 4 heteroatoms each independently selected from O, S and N", a "5- to 12-membered heteroaryl containing 1 to 4 heteroatoms each independently selected from O, S and N" and substituted with one or more R⁵⁻¹⁻², C₃₋₁₂ cycloalkyl, C₃₋₁₂ cycloalkyl substituted with one or more R⁵⁻¹⁻³, a "4- to 12-membered heterocycloalkyl containing 1 to 3 heteroatoms each independently selected from O, S and N", a "4- to 12-membered heterocycloalkyl containing 1 to 3 heteroatoms each independently selected from O, S and N" and substituted with one or more R⁵⁻¹⁻⁴, C₄₋₈ cycloalkenyl, C₄₋₈ cycloalkenyl substituted with one or more R⁵⁻¹⁻⁵, a "4- to 8-membered heterocycloalkenyl containing 1 to 3 heteroatoms each independently selected from O, S and N", or a "4- to 8-membered heterocycloalkenyl containing 1 to 3 heteroatoms each independently selected from O, S and N" and substituted with one or more R⁵⁻¹⁻⁶; when there are multiple substituents, they may be the same or different;
R⁵⁻¹⁻¹, R⁵⁻¹⁻², R⁵⁻¹⁻³, R⁵⁻¹⁻⁴, R⁵⁻¹⁻⁵ and R⁵⁻¹⁻⁶ are independently halogen, C₁₋₆ alkyl, or C₁₋₆ alkyl substituted with one or more halogen atoms;
R^{5a}, R^{5b1}, R^{5b2}, R^{5c}, R^{5d1} and R^{5d2} are independently hydrogen, C₁₋₆ alkyl, or C₁₋₆ alkyl substituted with one or more halogen atoms;
the heteroaromatic sulfonamide structural compound represented by formula I is not any one of the following compounds:
N-(1-methylcyclopropyl)-3-(5-(difluoromethyl)-1,3,4-thiadiazol-2-yl)-1-ethyl-6-fluoro-7-(4-isobutyrylpiperazin-1-yl)-1H-indazole-5-sulfonamide;
N-(1-cyanocyclopropyl)-6-fluoro-7-(4-(isopropylsulfonyl)piperazin-1-yl)-1-methyl-3-(5-(trifluoromethyl)-1,3,4-thiadiazol-2-yl)-1H-indazole-5-sulfonamide;
Embodiment 3: in the structure represented by formula I,
A₃ is C;
A₄ is N;
A₅ is NR⁵; 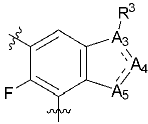
is 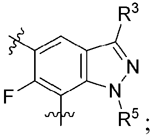
R^{a1} is cyano or C₁₋₆ alkyl;
ring Y is a "4- to 12-membered heterocycloalkyl containing 1 to 3 heteroatoms each independently selected from O, S and N", or a "4- to 8-membered heterocycloalkenyl containing 1 to 3 heteroatoms each independently selected from O, S and N";
R¹ is 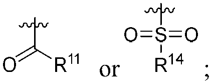
R¹¹ and R¹⁴ are independently C₁₋₆ alkyl;
n is 0, 1 or 2;
R² is cyano or C₁₋₆ alkyl;
R³ is a "5- to 6-membered heteroaryl containing 1 to 4 heteroatoms each independently selected from O, S and N" and substituted with one or more R³⁻¹;
R³⁻¹ is C₁₋₆ alkyl substituted with one or more halogen atoms;
R⁵ is C₁₋₆ alkyl;
the heteroaromatic sulfonamide structural compound represented by formula I is not any one of the following compounds:
N-(1-methylcyclopropyl)-3-(5-(difluoromethyl)-1,3,4-thiadiazol-2-yl)-1-ethyl-6-fluoro-7-(4-isobutyrylpiperazin-1-yl)-1H-indazole-5-sulfonamide;
N-(1-cyanocyclopropyl)-6-fluoro-7-(4-(isopropylsulfonyl)piperazin-1-yl)-1-methyl-3-(5-(trifluoromethyl)-1,3,4-thiadiazol-2-yl)-1H-indazole-5-sulfonamide.

5. The heteroaromatic sulfonamide structural compound represented by formula I, the pharmaceutically acceptable salt thereof, the stereoisomer thereof, the tautomer thereof or the isotopically labeled compound thereof according to claim 1 or 2, **characterized in that** the structure of the heteroaromatic sulfonamide structural compound represented by formula I or formula II is as shown in formula III: 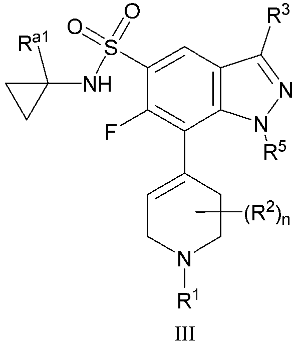
R^{a1} is cyano or C₁₋₆ alkyl;
R¹ is 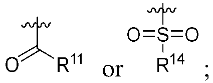
R¹¹ and R¹⁴ are independently C₁₋₆ alkyl;
n is 0 or 1;
R² is C₁₋₆ alkyl;
R³ is a "5- to 6-membered heteroaryl containing 1 to 4 heteroatoms each independently selected from O, S and N" and substituted with one or more R³⁻¹;
R³⁻¹ is C₁₋₆ alkyl substituted with one or more halogen atoms;
R⁵ is C₁₋₆ alkyl.

6. The heteroaromatic sulfonamide structural compound represented by formula I, the pharmaceutically acceptable salt thereof, the stereoisomer thereof, the tautomer thereof or the isotopically labeled compound thereof according to claim 1, **characterized in that**:
when R³ is a "5- to 6-membered heteroaryl containing 1 to 4 heteroatoms each independently selected from O, S and N", or a "5- to 6-membered heteroaryl containing 1 to 4 heteroatoms each independently selected from O, S and N" and substituted with one or more R³⁻¹, the said R³ is a "5- to 6-membered heteroaryl containing 1 to 3 heteroatoms each independently selected from S and N";
and/or, when R³⁻¹ is a C₁₋₆ alkyl substituted with one or more halogen atoms, the said R³⁻¹ is a C₁₋₂ alkyl substituted with two or three halogen atoms;
and/or, when ring Y is a "4- to 12-membered heterocycloalkyl containing 1 to 3 heteroatoms each independently selected from O, S and N", the said ring Y is a "4- to 8-membered heterocycloalkyl containing 1 to 2 heteroatoms each independently selected from O, S and N"; and/or, when ring Y is a "4- to 8-membered heterocycloalkenyl containing 1 to 3 heteroatoms each independently selected from O, S and N", the said ring Y is a "4- to 6-membered heterocycloalkenyl containing 1 to 3 heteroatoms each independently selected from O, S and N";
and/or, A₃ is C;
and/or, A₄ is N;
and/or, A₅ is NR⁵;
and/or, R^{a1} is cyano or C₁₋₆ alkyl;
and/or, n is 0, 1 or 2;
and/or, R² is cyano, C₃₋₈ cycloalkyl or C₁₋₆ alkyl;
and/or, when the definitions
of R^{a1}, R¹¹, R¹², R¹³, R¹⁴, R¹⁵, R¹⁶, R¹⁻¹, R¹⁻², R¹⁻³, R¹⁻⁴, R¹⁻⁵, R¹⁻⁶, R^{1 -7}, R^{1a}, R^{1b1}, R^{1b2}, R^{1c}, R^{1d1}, R^{1d2}, R^{1c1}, R^{1c2}, R^{1f}, R², R²⁻¹, R²⁻², R^{2a}, R^{2b1}, R^{2b2}, R^{2c}, R^{2d1}, R^{2d2}, R^{22a}, R^{22b1}, R^{22b2}, R^{22c}, R^{22d1}, R^{22d2}, R³⁻¹, R⁴, R⁵, R⁵⁻¹, R⁵⁻¹⁻¹, R⁵⁻¹⁻², R⁵⁻¹⁻³, R⁵⁻¹⁻⁴, R⁵⁻¹⁻⁵, R⁵⁻¹⁻⁶, R^{5a}, R^{5b1}, R^{5b2}, R^{5c}, R^{5d1} and R^{5d2} refer to C₁₋₆ alkyl, the said C₁₋₆ alkyl is C₁₋₄ alkyl.

7. The heteroaromatic sulfonamide structural compound represented by formula I, the pharmaceutically acceptable salt thereof, the stereoisomer thereof, the tautomer thereof or the isotopically labeled compound thereof according to claim 1, **characterized in that**: 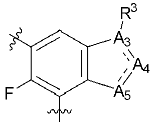
is 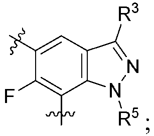
and/or, R^{a1} is cyano or methyl;
and/or, R¹ is 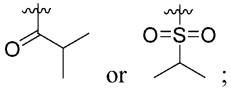
and/or, R² is cyano, cyclopropyl, ethyl, n-propyl or methyl;
and/or, R³ is 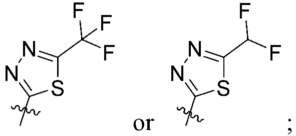
and/or, R⁵ is methyl;
and/or, ring Y is

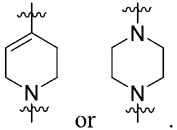

8. The heteroaromatic sulfonamide structural compound represented by formula I, the pharmaceutically acceptable salt thereof, the stereoisomer thereof, the tautomer thereof or the isotopically labeled compound thereof according to claim 1, **characterized in that**: 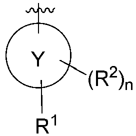 is

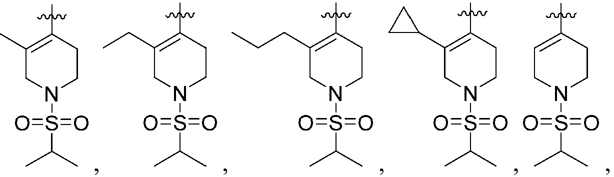

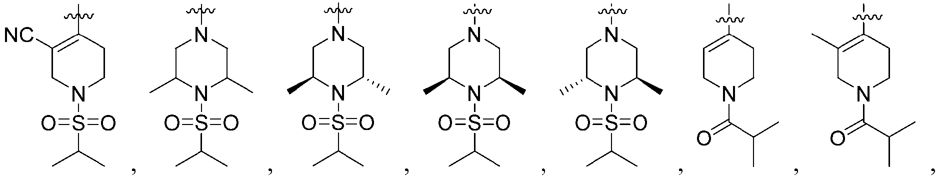

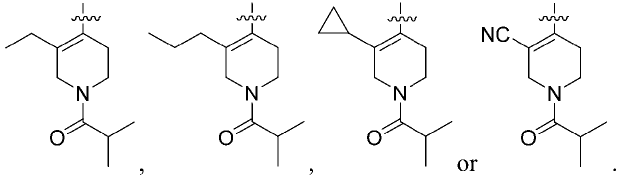

9. The heteroaromatic sulfonamide structural compound represented by formula I, the pharmaceutically acceptable salt thereof, the stereoisomer thereof, the tautomer thereof or the isotopically labeled compound thereof according to claim 1, **characterized in that** the compound represented by formula I is any one of the following structures:

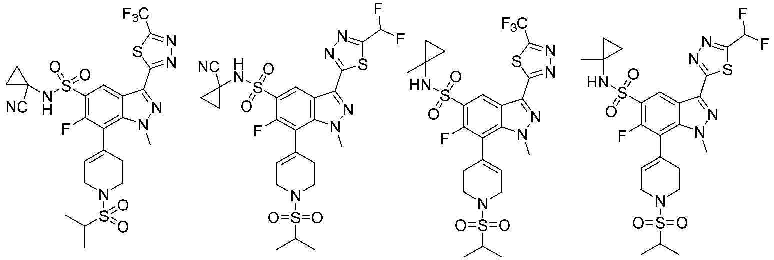

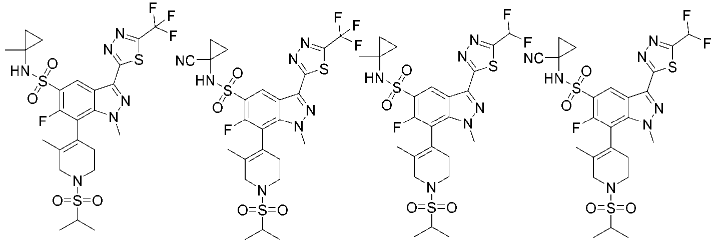

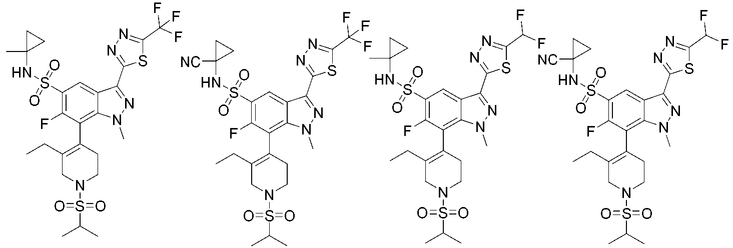

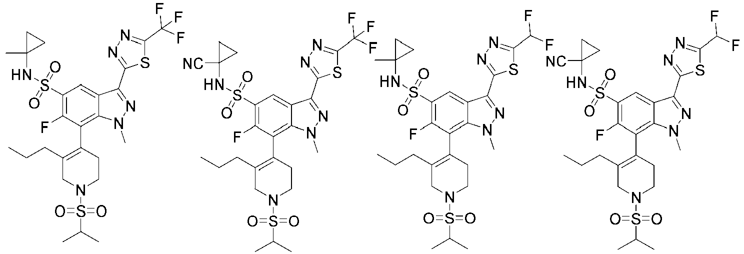

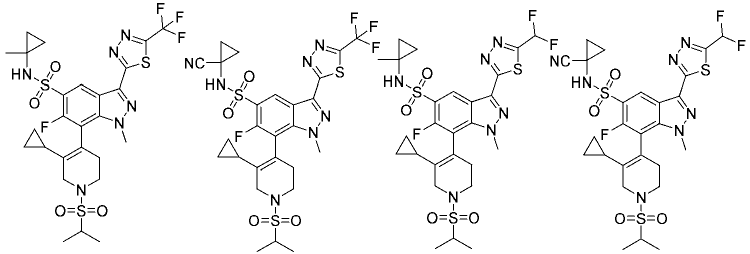

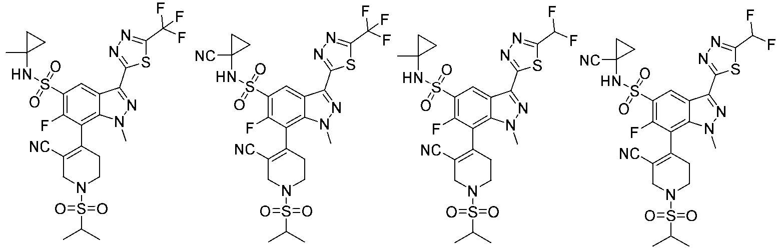

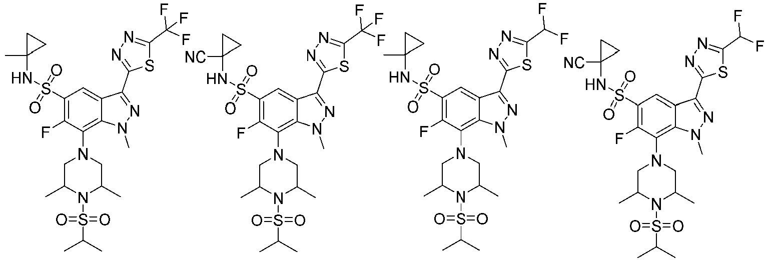

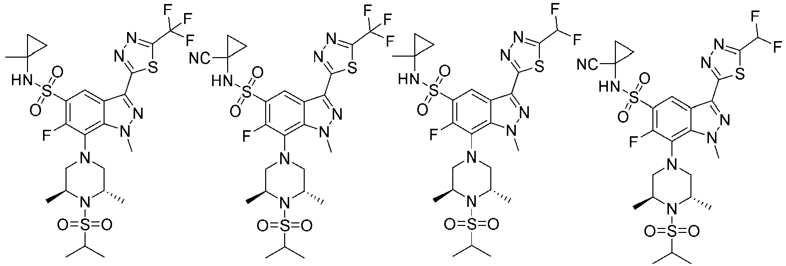

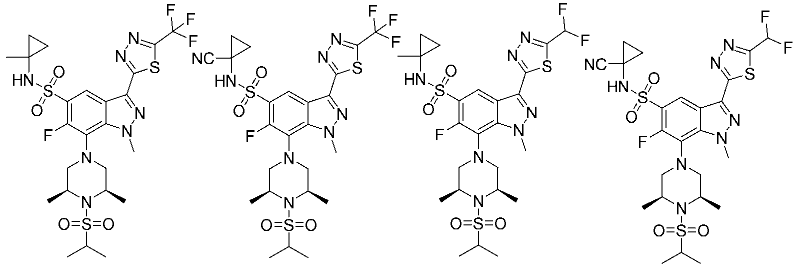

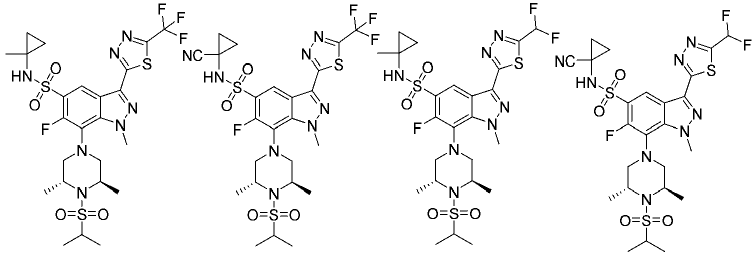

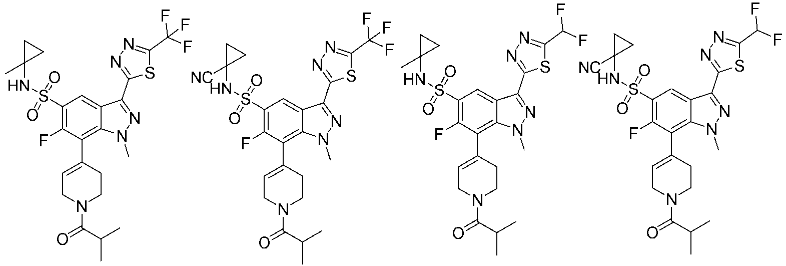

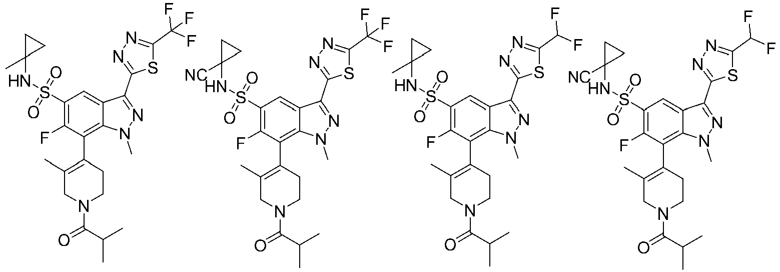

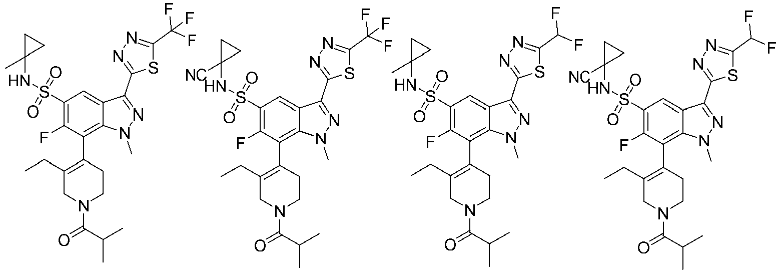

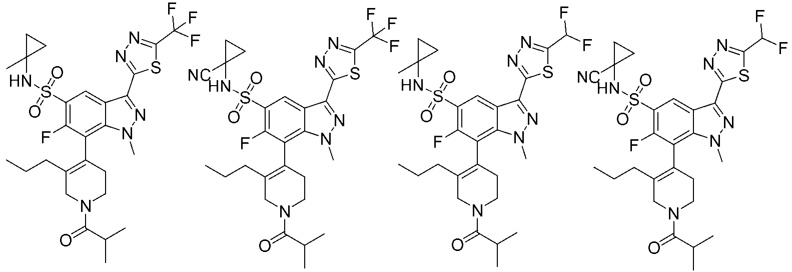

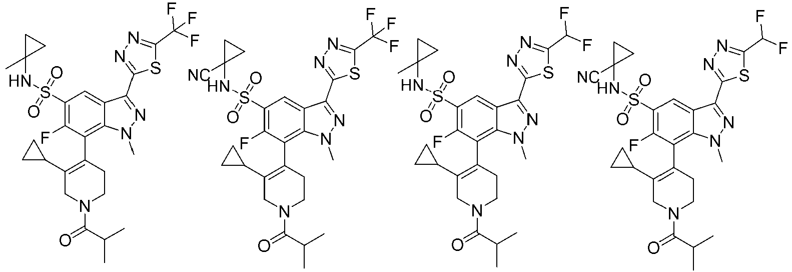

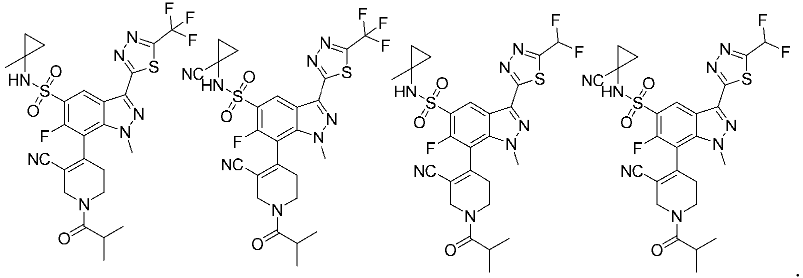

10. A pharmaceutical composition comprising substance A and pharmaceutically acceptable excipients; wherein the said substance A is a therapeutically effective amount of the heteroaromatic sulfonamide structural compound represented by formula I, the pharmaceutically acceptable salt thereof, the stereoisomer thereof, the tautomer thereof or the isotopically labeled compound thereof according to any one of claims 1 to 9.

11. Use of substance A in the preparation of a PARG inhibitor, wherein the said substance A is the heteroaromatic sulfonamide structural compound represented by formula I, the pharmaceutically acceptable salt thereof, the stereoisomer thereof, the tautomer thereof or the isotopically labeled compound thereof according to any one of claims 1 to 9.

12. Use of substance A in the preparation of a medicament, wherein the said medicament is for the treatment or prevention of PARG-mediated diseases; the said substance A is the heteroaromatic sulfonamide structural compound represented by formula I, the pharmaceutically acceptable salt thereof, the stereoisomer thereof, the tautomer thereof or the isotopically labeled compound thereof according to any one of claims 1 to 9.

13. Use of substance A in the preparation of a medicament, wherein the said medicament is for the treatment or prevention of a cancer; the said substance A is the heteroaromatic sulfonamide structural compound represented by formula I, the pharmaceutically acceptable salt thereof, the stereoisomer thereof, the tautomer thereof or the isotopically labeled compound thereof according to any one of claims 1 to 9; and the said cancer is one or more selected from colon cancer, appendiceal cancer, pancreatic cancer, MYH-associated polyposis, hematological cancer, breast cancer, endometrial cancer, gallbladder cancer, cholangiocarcinoma, prostate cancer, lung cancer, brain cancer, ovarian cancer, cervical cancer, testicular cancer, renal cancer, head and neck cancer, bone cancer, skin cancer, rectal cancer, liver cancer, esophageal cancer, gastric cancer, thyroid cancer, bladder cancer, lymphoma, leukemia and melanoma.
